# EUROPÄISCHE PATENTSCHRIFT

(11) **EP 2 181 195 B1**
(45) Veröffentlichungstag und Bekanntmachung des Hinweises auf die Patenterteilung: **09.04.2014**
(21) Anmeldenummer: 08843755.3
(22) Anmeldetag: 01.10.2008
(51) Int. Cl.: C12P 7/28, C12N 9/16

(54) **Fermentative Gewinnung von Aceton aus erneuerbaren Rohstoffen mittels neuen Stoffwechselweges**
Fermentative production of acetone from renewable resources by means of novel metabolic pathway
Production d'acétone par fermentation à partir de matières premières renouvelables, par nouvelle voie métabolique

(30) Priorität: 02.11.2007 DE 102007052463
(43) Veröffentlichungstag der Anmeldung: 05.05.2010
(73) Patentinhaber: Evonik Degussa GmbH, 45128 Essen (DE)
(72) Erfinder: VERSECK, Stefan, 40721 Hilden (DE); SCHAFFER, Steffen, 45699 Herten (DE); FREITAG, Werner, 46286 Dorsten (DE); SCHMIDT, Friedrich Georg, 45721 Haltern am See (DE); ORSCHEL, Matthias, 48149 Münster (DE); GRUND, Gerda, 48653 Coesfeld (DE); SCHMIDT, Wilfried, 45894 Gelsenkirchen-Buer (DE); BAHL, Hubert Johannes, 18059 Rostock (DE); FISCHER, Ralf-Jörg, 18198 Kritzmow (DE); MAY, Antje, 18057 Rostock (DE); DÜRRE, Peter, 89075 Ulm (DE); LEDERLE, Simone, 89077 Ulm (DE)
(86) Internationale Anmeldenummer: PCT/EP2008/063150
(87) Internationale Veröffentlichungsnummer: WO 2009/056423

(56) Entgegenhaltungen:
- WO-A-2008/131286
- BERMEJO L L ET AL: "Expression of Clostridium acetobutylicum ATCC 824 genes in Escherichia coli for acetone production and acetate detoxification" APPLIED AND ENVIRONMENTAL MICROBIOLOGY, AMERICAN SOCIETY FOR MICROBIOLOGY, US, Bd. 64, Nr. 3, 1. März 1998 (1998-03-01), Seiten 1079-1085, XP002334443 ISSN: 0099-2240 in der Anmeldung erwähnt
- ZHUANG Z ET AL: "The YbgC protein encoded by the ybgC gene of the tol-pal gene cluster of Haemophilus influenzae catalyzes acyl-coenzyme A thioester hydrolysis" FEBS LETTERS, ELSEVIER, AMSTERDAM, NL, Bd. 516, Nr. 1-3, 10. April 2002 (2002-04-10), Seiten 161-163, XP004349069 ISSN: 0014-5793 in der Anmeldung erwähnt
- ANEJA PUNITA ET AL: "Identification of an acetoacetyl coenzyme A synthetase-dependent pathway for utilization of L-(+)-3-hydroxybutyrate in Sinorhizobium meliloti" JOURNAL OF BACTERIOLOGY, Bd. 184, Nr. 6, März 2002 (2002-03), Seiten 1571-1577, XP002523134 ISSN: 0021-9193 in der Anmeldung erwähnt
- SCHWARZER DIRK ET AL: "Regeneration of misprimed nonribosomal peptide synthetases by type II thioesterases." PROCEEDINGS OF THE NATIONAL ACADEMY OF SCIENCES OF THE UNITED STATES OF AMERICA, Bd. 99, Nr. 22, 29. Oktober 2002 (2002-10-29), Seiten 14083-14088, XP002523135 ISSN: 0027-8424 in der Anmeldung erwähnt
- WOODS D R: "The genetic engineering of microbial solvent production" TRENDS IN BIOTECHNOLOGY, ELSEVIER PUBLICATIONS, CAMBRIDGE, GB, Bd. 13, Nr. 7, 1. Juli 1995 (1995-07-01), Seiten 259-264, XP004207180 ISSN: 0167-7799

## Beschreibung

### Gebiet der Erfindung

Gegenstand der Erfindung ist ein neuartiger, enzymatischer Biosyntheseweg zur Produktion von Aceton, der im Gegensatz zu herkömmlichen, fermentativen Verfahren von der Ethanol- und Butanol-Bildung entkoppelt ist, so wie die hierbei verwendeten Enzyme und Nukleinsäuren.

### Stand der Technik

### ABE-Prozess in Clostridium

Der klassische ABE Fermentationsprozess, also die mikrobielle Produktion von Aceton, Butanol und Ethanol, war für lange Zeit der weltweit zweitgrößte biotechnologische Prozess direkt nach der Ethanol-Fermentation mit Hefen. Die kommerzielle ABE Fermentation startete 1916 in England wo unter anderen Chaim Weizmann die Fähigkeit von *Clostridium acetobutylicum* zur Bildung der Lösungsmittel Aceton, Butanol und Ethanol entdeckte. Der Prozess wurde bis in die späten 50iger Jahre in der westlichen Welt angewendet, in Südafrika sogar noch bis 1981.

Zwei Hauptgründe sind dafür verantwortlich, dass dieser Prozess eingestellt wurde: Zum einen wurde die chemische Synthese von Aceton und Butanol immer günstiger, und zum anderen stieg der Preis für die Substrate der Fermentation stark an. Besonders der Preis für Melasse zog durch deren Verwendung als Futtermittelzusatz für Rinder stark an.

Die steigenden Kosten für petrochemische Vorprodukte, als auch neue technologische Möglichkeiten auf dem Gebiet des Pathway-Engineerings von Mikroorganismen eröffnen nun neue Optionen zur Entwicklung von Hochleistungsstämmen und kommerziellen Fermentationsprozessen zur Produktion von Lösungsmitteln wie Aceton.

Die klassische ABE-Fermentation basiert auf den Organismen *Clostridium acetobutylicum* und *Clostridium beijerinckii*. Beide sind Gram-positiv und vermehren sich unter strikt anaeroben Bedingungen. Diese Organismen können Mono-, Di- und Polysaccharide umsetzen, wobei die hauptsächlich in der Fermentation verwendeten Substrate Melasse und Stärke sind.

Der Fermentationsprozess mit C. *acetobutylicum* unterteilt sich in zwei Phasen. In der ersten Phase geht die Biomassebildung einher mit der Bildung von Acetat, Butyrat und Spuren von Ethanol ("Acidogene Phase"). In der zweiten Phase, der sogenannten "Solventogenen Phase", werden die Säuren dann genutzt, um die Fermentationsprodukte Aceton, Butanol und Ethanol zu bilden (ABE). Die Produkte Aceton, Butanol und Ethanol werden im Wildtyp C. *acetobutylicum* im Verhältnis von ungefähr 3 : 6 : 1 gebildet. Dieses Produktverhältnis kann je nach gewählten Kulturbedingungen (z.B. pH oder Nährstoffzufuhr)oder eingesetzten Substraten stark variieren.

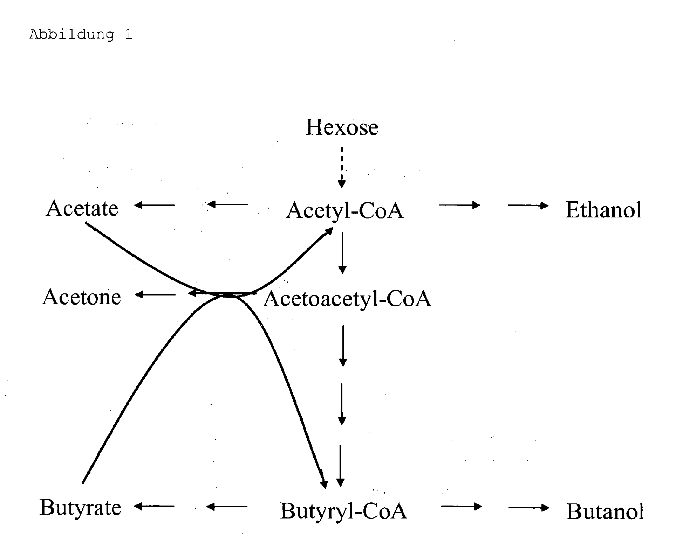

Die Enzyme der Lösungsmittel-Biosynthese von Aceton, Butanol und Ethanol sind weitgehend aufgereinigt und biochemisch charakterisiert (vgl. Abbildung 1; Duerre, P., and Bahl, H. 1996. Microbial production of acetone/butanol/isopropanol. In: Biotechnology, vol. 6, 2nd ed. M. Roehr, (ed.),VCH Verlagsgesellschaft mbH, Weinheim, Germany. p. 229-268.Duerre, P. 1998. New insights and novel developments in clostridial acetone/butanol/isopropanol fermentation. Appl. Microbiol. Biotechnol. 49: 639-648.). Auch die Genomsequenz von C. *acetobutylicum* liegt vor (Noelling, J., Breton, G., Omelchenko, M. V. & 16 other authors(2001). Genome sequence and comparative analysis of the solvent producing bacterium Clostridium acetobutylicum. J Bacteriol 183,4823-4838.).

In den letzten Jahren wurde eine Reihe von genetischen Werkzeugen entwickelt, die ein gezieltes *Pathway Engineering* ermöglichen. Bis jetzt stehen drei unterschiedliche, sich stabil erhaltene Replikons (pIM13, pCBU2 und pAMβ1; Lee et al. (1992). Vector construction, transformation, and gene amplification in Clostridium acetobutylicum ATCC 824. Ann. N. Y. Acad. Sci. 665: 39-51; Minton et al. (1993). Clostridial cloning vectors. In: The clostridia and biotechnology (D. R. Woods; Hrsg.). Butterworth-Heinemann. Stoneham, USA. S. 119-150) und zwei Antibiotika Resistenzmarker gegen Erythromycin und Thiamphenicol zur Verfügung (Green and Bennett (1998). Genetic manipulation of acid and solvent formation in Clostridium acetobutylicum ATCC 824. Biotechnol. Bioeng. 58:215-21.).

Dagegen sind Methoden wie Insertionsinaktivierung oder Gendeletionen noch nicht routinemäßig durchführbar, und auch die Antisense-basierte Inhibition der Genexpression in dieser Gruppe von Organismen variiert in der Effektivität und ist nie vollständig (Desai and Papoutsakis (1999). Antisense RNA strategies for metabolic engineering of Clostridium acetobutylicum. Appl. Environ. Microbiol. 65:936-45; Tummala et al. (2003). Antisense RNA downregulation of coenzyme A transferase combined with alcohol-aldehyde dehydrogenase overexpression leads to predominantly alcohologenic Clostridium acetobutylicum fermentations. J. Bacteriol. 185:3644-53.). Eine Reihe von Veröffentlichungen beschreibt das Metabolic-Engineering sowohl des "Solventogenen" als auch des "Acidogenen" Biosyntheseweges. Die Inaktivierung der Gene *buk* (Butyrat Kinase) oder pta (Phosphotrans-acetylase) führte zu drastischen Absenkungen der Konzentrationen von Butyrat bzw. Acetat (Green et al. (1996). Genetic manipulation of acid formation pathways by gene inactivation in Clostridium acetobutylicum ATCC 824. Microbiology. 142:2079-86.; Harris et al. (2000). Characterization of recombinant strains of the Clostridium acetobutylicum butyrate kinase inactivation mutant: need for new phenomenological models for solventogenesis and butanol inhibition? Biotechnol. Bioeng. 67:1-11.). Dennoch wurde von diesen Mutanten Butyrat und Acetat gebildet, da die Enzyme Acetat-Kinase und Phosphotransbutyrylase die inaktivierten Enzyme Butyrat-Kinase und Phosphotransacetylase substituierten. Beide Stämme bildeten hohe Konzentrationen von Milchsäure, welches vom Wildtyp C. *acetobutylicum* nicht angereichert wird, möglicherweise als Reaktion auf die Anreicherung von Pyruvat. Die Inaktivierung der Aldehyd-/Alkohol-Dehydrogenase adhE/aad führte zu einem drastischen Einbruch der Bildung von Butanol und zu einem gleichzeitigen Anstieg der Butyrat-Konzentration. Die im Wildtyp üblichen Konzentrationen von Butyrat und Butanol stellen sich aber wieder ein, wenn das *adhE*/*aad* Gen auf einem Plasmid in dieser Mutante exprimiert wird. Interessanterweise konnte weder in der AdhE/aad-Mutante noch in dem Stamm mit der adhE/aad-Komplementierung eine Bildung von Aceton nachgewiesen werden. Dieses Phänomen basiert auf polaren Effekten, die sich auf zwei "downstream" von *adhE*/*aad* lokalisierte Gene auswirken. Diese beiden Gene kodieren die beiden Untereinheiten der Coenzym A-Transferase, welche essentiell für die Biosynthese von Aceton ist (Green, E.M, and Bennett, G.N. 1996. Inactivation of an aldehyde/ alcohol dehydrogenase gene from Clostridium acetobutylicum ATCC 824. Appl. Biochem. Biotechnol. 57/58: 213-221). Dies ist das erste beschriebene Beispiel dass durch Pathway-Engineering in *C. acetobutylicum* die Aceton und Butyrat-Bildung von einander entkoppelt werden kann.

In weiteren Veröffentlichungen wurde diese Beobachtung bestätigt. In diesen Studien wurden mikrobielle Stämme untersucht, die die Fähigkeit zur Bildung von Aceton und Butanol verloren haben, da ihnen das 192-kb-Megaplasmid pSOL1 fehlt. Auf diesem Plasmid sind die meisten Gene für die Bildung von Aceton und Butanol lokalisiert, und auch einige für die Ethanol-Synthese (Cornillot, E., Nair, R., Papoutsakis, E.T., and Soucaille, P. 1997. The genes for butanol and acetone formation in Clostridium acetobutylicum ATCC 824 reside on a large plasmid whose loss leads to degeneration of the strain. J. Bacteriol. 179: 5442-5447). Diese degenerierten Stämme bilden nur halb soviel Ethanol wie die Wildtypstämme im Vergleich. Diese Stämme, ausgehend von *C. acetobutylicum* ATCC 824, wurden M5 (entstanden durch chemische Mutagenese) und DG1 (erhalten durch mehrfache Kultivierung des Wildtypes)genannt. Diese dienten als Rezipienten von Plasmiden, die die proteinkodierenden Sequenzen der Coenzym A-Transferase Untereinheiten A und B (*ctfA* und *ctfB*), sowie das Gen der Acetoacetat-Decarboxylase (adc), oder der Butyraldehyd-/Butanol-Dehydrogenase (*adhE*/*aad*)trugen. Die resultierenden Stämme produzierten entweder nur Aceton oder Butanol (Mermelstein et al. (1993). Metabolic engineering of Clostridium acetobutylicum ATCC824 for increased solvent production by enhancement of acetone formation enzyme activities using a synthetic operon. Biotech. Bioeng. 42:1053-1060.; Nair, R.V., and Papoutsakis, E.T. 1994. Expression of plasmid-encoded aad in Clostridium acetobutylicum M5 restores vigorous butanol production. J. Bacteriol. 176: 5843-5846; Cornillot, E., Nair, R., Papoutsakis, E.T., and Soucaille, P. 1997. The genes for butanol and acetone formation in Clostridium acetobutylicum ATCC 824 reside on a large plasmid whose loss leads to degeneration of the strain. J. Bacteriol. 179: 5442-5447). Die gemessenen Titer für das jeweilige Lösungsmittel lagen grundsätzlich unter den Konzentrationen von Aceton und Butanol im Wildtyp, wobei sich Acetat und Butyrat bis zu einer Gesamtkonzentration von 240 mM akkumulieren konnte. Die Ethanolbildung konnte mit Plasmiden, die das *adhE*/*aad*-Gen trugen, auf Wildtyp-Niveau wieder hergestellt werden.

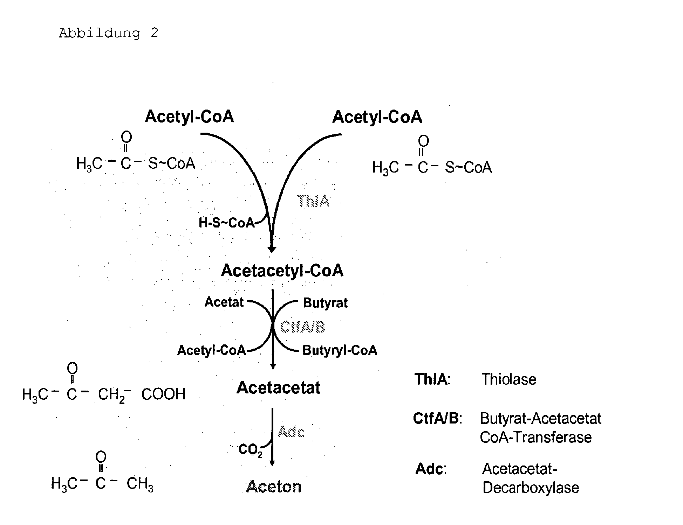

In Abbildung 2 ist der klassische, in *Clostridium* charakterisierte Stoffwechselweg für die Acetonsynthese dargestellt. Dieser Weg geht aus von Acetyl-CoA, einem zentralen Metaboliten, der in allen Mikroorganismen gebildet wird, unabhängig davon, welche C-Quelle verstoffwechselt wird oder welche Stoffwechselwege etabliert sind. Enzyme, die benötigt werden, sind: die β-Ketothiolase, die zwei Untereinheiten der Acetyl-CoA/Butyryl-CoA-Transferase, und die Acetoacetat-Decarboxylase.

Es konnte gezeigt werden, dass die heterologe Expression dieser Enzyme aus C. *acetobutylicum* in *Escherichia coli,* die die Acetonbildung ausgehend von Acetyl-CoA katalysieren (Acetoacetat-Decarboxylase, Acetyl-CoA/Butyryl-CoA-Transferase und Thiolase) zu einer Acetonbildung in diesem Organismus von ca. 150 mM führen, wobei aber auch nachteilhafter Weise hohe Mengen an Acetat (50 mM) anfielen (Bermejo L. L., N. E. Welker, E. T. Papoutsakis. 1998. Expression of Clostridium acetobutylicum ATCC 824 Genes in Escherichia coli for Acetone Production and Acetate Detoxification. Appl. Env. Microbiol. 64:1079-1085). Ein weiterer Nachteil ist hier, dass Aceton nur unter aeroben Bedingungen produziert wurde, da die Redoxequivalente, die während der Metabolisierung von Glukose zu Acetyl-CoA entstehen, unter anaeroben Bedingungen von *E. coli* nicht reoxidiert werden können. In diesem Verfahren wurde durch das die Reaktion katalysierende Enzym das vom Acetoacetyl abgespaltene CoA wieder auf ein Akzeptormolekül übertragen.

### Acyl-CoA spaltende Enzyme

Enzyme, die Acyl-CoA hydrolysieren können z.B. Acyl-CoA-Thioesterasen oder Acyl-CoA-Thiokinase sein. Der Hauptunterschied zwischen der Hydrolyse von Acyl-CoA durch eine Acyl-CoA-Thioesterase oder eine Acyl-CoA-Synthetase / Acyl-CoA-Thiokinase ist die Bildung von ATP bzw. GTP im Falle der Kinasen, da die Hydrolyse von AcetoaCetyl-CoA einen höheren ΔG₀' - Wert von -44 kJ besitzt verglichen mit der Hydrolyse von ATP (-31,8 kJ).

### Acetoacetyl-CoA-Hydrolase (EC 3.1.2.11)

Dieses Enzym katalysiert die Hydrolyse von Acetoacetyl-CoA zu Acetoacetat und Coenzym A, ohne dabei das CoA-Molekül gleichzeitig auf ein Akzeptormolekül wie z.B. eine Carbonsäure zu übertragen.

### Acyl-CoA-Thioesterasen (EC 3.1.2.18)

Das Protein YbgC aus *Haemophilus influenzae* ist eine für kurzkettige Moleküle spezifische Acyl-CoA-Thioesterase (EC 3.1.2.18), welches Butyryl-CoA und β-Hydroxybutyrat-CoA als Substrate akzeptiert. Die Km-Werte betragen 24 mM bzw. 20 mM für diese Substrate (Zhuang et al. (2002). The YbgC protein encoded by the ybgC gene of the tol-pal gene cluster of Haemophilus influenzae catalyzes acyl-coenzyme A thioester hydrolysis. FEBS Lett. 516:161-3.).

Auch in *Bacillus subtilis* ist eine Thioesterase II (TEII_{srf}) mit Aminosäuresequenz SEQ ID Nr. 1 und korrespondierender cDNA-Sequenz SEQ ID Nr. 2 beschrieben, welche mit den nicht-ribosomalen Peptidsynthetasen zur Bildung des Peptid-Antibiotikums Surfactin assoziiert vorliegt (Schwarzer D., H. D. Mootz, U. Linne, M. A. Marahiel. 2002. Regeneration of misprimed nonribosomal peptide synthetases by type II thioesterases. PNAS 99:14083-14088).

### Acyl-CoA-Synthetasen/Acyl-CoA-Thiokinasen (unter AMP-Bildung; EC 6.2.1.2, unter GDP-Bildung; EC 6.2.1.10))

Die physiologische Funktion dieser beiden genannten Enzyme liegt in der Acyl-CoA-Synthese, aber es sind auch Beispiele bekannt, in der diese Enzyme die umgekehrte, hydrolytische Reaktion unter Bildung von ATP katalysieren, wie z.B. die Succinyl-CoA-Thiokinase im Trikarbonsäurezyklus. Bisher wurde, weder *in vitro* noch *in vivo*, für die oben genannten Enzymklassen nachgewiesen, dass diese Acetoacetyl-CoA unter Bildung von Acetoacetat und freiem Coenzym A zu hydrolysieren vermögen.

Der Abbau von Polyhydroxyalkanen durch eine Acyl-CoA-Synthetase unter AMP-Bildung ist für AcsA2 aus *Sinorhizobium meliloti* beschrieben (Aneja et al. (2002). Identification of an acetoacetyl coenzyme A synthetase-dependent pathway for utilization of L-(+)-3-hydroxybutyrate in Sinorhizobium meliloti. J. Bacteriol. 184:1571-7.).

Bei einem weiteren aufgereinigten Enzym aus *Pseudomonas aeruginosa* konnte eine für kurzkettige Moleküle spezifische Acyl-CoA-Synthetase / Acyl-CoA-Thiokinase (unter AMP Bildung; EC 6.2.1.2) nachgewiesen werden. Dieses Enzym akzeptiert sowohl Butyryl-CoA und β-Hydroxybutyryl-CoA als auch 2-Ketobutyrat als Substrat, mit sehr niedrigen Km-Werten von jeweils 10, 25 und 25 µM (Shimizu et al. (1981). Butyryl-CoA synthetase of Pseudomonas aeruginosa-purification and characterization. Biochem. Biophys. Res. Commun. 103:1231-7.).

Aufgabe der vorliegenden Erfindung war es daher, einen neuen, vereinfachten Stoffwechselweg für die Aceton Synthese ausgehend vom Acetyl-CoA bereitzustellen.

### Beschreibung der Erfindung

Die hier beschriebene Erfindung behandelt die verbesserte Produktion von Aceton mit Hilfe rekombinanter Enzymtechniken: Ausgehend von Acetyl-CoA wird über Acetoacetyl-CoA Acetoacetat generiert, welches anschließend in Aceton und CO₂ umgewandelt wird. Gegenstand der vorliegenden Erfindung sind deshalb ein Verfahren zur Produktion von Aceton wie in Anspruch 1 beschrieben, so-wie Nukleinsäuresequenzen gemäß Anspruch 13.

Das erfindungsgemäße Verfahren hat den Vorteil, dass die Butyrat-Acetoacetat-CoA-Transferase, die in herkömmlichen Systemen Acetoacetyl-CoA zu Acetoacetat umwandelt und aus zwei Untereinheiten besteht, nun durch ein Enzym ersetzt wird, welches seine Aktivität als Monomer ausüben kann.

Hierdurch hat das erfindungsgemäße Verfahren den weiteren Vorteil, dass die Ausbeuten an Aceton in gegebenem System höher sind, als mit bekannter Butyrat-Acetoacetat-CoA-Transferase Ein weiterer Vorteil ist, dass das Verhältnis Acetat zu Aceton während des Produktionsprozesses zu Gunsten des Acetons verschoben wird.

Noch ein weiterer Vorteil des erfindungsgemäßen Verfahrens ist die Tatsache, dass die Aceton-Synthese von der von Butanol und Ethanol entkoppelt ist und somit bei Verwendung mikrobieller Produzenten sich diese durch die anfallenden, alkoholischen Nebenprodukte nicht vergiften. Dies führt zu höheren Produktkonzentrationen im Medium und somit zu verbesserten Raum-Zeit-Ausbeuten.

Ein weiterer Vorteil der von Butanol und Ethanol entkoppelten Herstellung von Aceton ist ein vereinfachtes Fermentationsprotokoll, sowie die einfachere Isolierung und Aufarbeitung des Produkets, da es nicht mehr von den alkoholischen Nebenprodukten abgetrennt werden muss. Dies führt dann auch zu einem geringerem Energiebedarf verglichen mit der Produktabtrennung bei der klassischen ABE-Fermentation. Des Weiteren ist an erfindungsgemäßem Verfahren vorteilhaft, dass es sich in verschiedenen Mikroorganismen durchführen lässt, die besonders gut charakterisiert sind, sich hervorragend aerob als auch anaerob kultivieren lassen und für die unterschiedlichste Möglichkeiten der genetischen Manipulation und somit weitere Verbesserungsmöglichkeiten bekannt sind. Insgesamt stellt die effektive, fermentative Herstellung von Aceton aus nachwachsenden Rohstoffen einen weiteren Beitrag zur umweltgerechten und ressourcenschonende Produktion einer Industriechemikalie dar.

Das erfindungsgemäße Verfahren zur Herstellung von Aceton und die Verwendung angegebener Polypeptide sowie Nukleinsäuren zur Durchführung des erfindungsgemäßen Verfahrens werden nachfolgend beispielhaft beschrieben, ohne dass die Erfindung auf diese beispielhaften Ausführungsformen beschränkt sein soll. Als Polypeptide sind solche beliebiger Kettenlänge gemeint. Somit werden hierunter auch jegliche Proteine, Enzyme und insbesondere Acetoacetat-CoA-Hydrolasen, Aryl-CoA-Thioesterasen, Acyl-CoA-Synthetasen oder Acyl-CoA-Thiokinasen verstanden. Unter einer "spontan erfolgenden Umsetzung" ist eine exergone chemische Reaktion zu verstehen. Unter Hybridisieren "unter stringenten Bedingungen" sind solche Versuchsbedingungen gemeint, wie man beispielsweise in der Northern-Blot-Technik die Verwendung einer 50 - 70 °C, vorzugsweise 60 - 65 °C warmen Waschlösung, beispielsweise 0,1x SSC-Puffer mit 0,1 % SDS (20x SSC: 3M NaCl, 0,3M Na-Citrat, pH 7,0) zur Elution unspezifisch hybridisierter cDNA-Sonden oder Oligonukleotide einsetzt. Dabei bleiben nur in hohem Maße komplementäre Nukleinsäuren aneinander gebunden. Die Einstellung stringenter Bedingungen ist dem Fachmann bekannt und ist z.B. in Ausubel et al., Current Protocols in Molecular Biology, John Wiley & Sons, N.Y. (1989), 6.3.1-6.3.6. beschrieben.

Die vorliegende Erfindung beinhaltet ein Verfahren zur Herstellung von Aceton ausgehend von Acetyl-CoenzymA umfassend die Schritte: A. enzymatische Umsetzung von Acetyl-CoA zu Acetoacetyl-CoA B. enzymatische Umsetzung von Acetoacetyl-CoA zu Acetoacetat und CoA und C. Decarboxylieren von Acetoacetat zu Aceton und CO₂, dadurch gekennzeichnet, dass in Verfahrensschritt B das CoenzymA nicht auf ein Akzeptor-Molekül übertragen wird.

In dem erfindungsgemäßen Verfahren wird in Verfahrensschritt A eine ß-Ketothiolase zur enzymatischen Umsetzung von Acetyl-CoA zu Acetoacetyl-CoA eingesetzt. Bevorzugt wird eine ß-Ketothiolase aus *Clostridium spec.*, also einem Mikroorganismus aus der Gattung Clostridium, besonders bevorzugt aus *Clostridium acetobutylicum* oder *Clostridium beijerinckii* eingesetzt. Vorzugsweise wird die enzymatische Umsetzung in Verfahrensschritt A durch ein Polypeptid mit einer Ketothiolase-Aktivität und mit einer Aminosäuresequenz, die zu mindestens 90%, vorzugsweise mindestens 95%, bevorzugt 96, 97, 98, 99 oder 100% identisch mit der Aminosäuresequenz SEQ ID Nr. 16 ist, katalysiert.

In dem erfindungsgemäßen Verfahren wird in Verfahrensschritt C eine Acetoacetatdecarboxylase zur Umsetzung von Acetoacetyl-CoA zu Aceton und CO₂ eingesetzt. Bevorzugt wird eine Acetoacetatdecarboxylase aus *Clostridium spec.*, also einem Mikroorganismus aus der Gattung Clostridium, besonders bevorzugt aus *Clostridium acetobutylicum* oder *Clostridium beijerinckii* eingesetzt. Vorzugsweise wird die enzymatische Umsetzung in Verfahrensschritt C durch ein Polypeptid mit einer Acetoacetatdecarboxylase-Aktivität und mit einer Aminosäuresequenz, die zu mindestens 90%, vorzugsweise mindestens 95%, bevorzugt 96%, 97%, 98%, 99% oder 100% identisch mit der Aminosäuresequenz SEQ ID Nr. 18 ist, katalysiert. Eine weitere Ausführungsform erfindungsgemäßen Verfahrens ist dadurch gekennzeichnet, dass die Umsetzung in Verfahrensschritt C spontan erfolgt.

Das erfindungsgemäße Verfahren zur Aceton-Produktion zeichnet sich dadurch aus, dass in Verfahrensschritt B das CoenzymA nicht auf ein Akzeptor-Molekül übertragen wird. Dies wird dadurch erreicht, dass Enzyme mit Acetoacetat-CoA-Hydrolase-Aktivität eingesetzt werden die eine Aminosäuresequenz haben, die zu mindestens 90% identisch ist mit der Aminosäuresequenz SEQ ID Nr. 1, 3 oder 5. Überraschenderweise können ebenfalls Enzyme, denen diese Aktivität bisher nicht zugeschrieben wurde, wie z.B. solche ausgewählt aus der Gruppe der Acyl-CoA-Thioesterasen, eine Acyl-CoA-Synthetasen oder Acyl-CoA-Thiokinasen ebenfalls diese Aufgabe lösen. Daher wird im erfindungsgemäßen Verfahren die enzymatische Umsetzung in Verfahrensschritt B durch eine Acetoacetat-CoA-Hydrolase, eine Acyl-CoA-Thioesterase, eine Acyl-CoA-Synthetase oder eine Acyl-CoA-Thiokinase katalysiert.

In einer bevorzugten Ausführungsform des erfindungsgemäßen Verfahrens kann die enzymatische Umsetzung in Verfahrensschritt B durch ein Polypeptid mit Acetoacetat-CoA-Hydrolase-Aktivität und mit einer Aminosäuresequenz, die zu mindestens 90%, vorzugsweise mindestens 95%, bevorzugt 96%, 97%, 98%, 99% oder 100% identisch mit der Aminosäuresequenz SEQ ID Nr. 1 ist, katalysiert werden. In einer weiteren bevorzugten Ausführungsform des erfindungsgemäßen Verfahrens kann die enzymatische Umsetzung in Verfahrensschritt B durch ein Polypeptid mit Acetoacetat-CoA-Hydrolase-Aktivität und mit einer Aminosäuresequenz, die zu mindestens 90%, vorzugsweise mindestens 95%, bevorzugt 96%, 97%, 98%, 99% oder 100% identisch mit der Aminosäuresequenz SEQ ID Nr. 3 ist, katalysiert werden. In einer besonders bevorzugten Ausführungsform des erfindungsgemäßen Verfahrens kann die enzymatische Umsetzung in Verfahrensschritt B durch ein Polypeptid mit Acetoacetat-CoA-Hydrolase-Aktivität und mit einer Aminosäuresequenz, die zu mindestens 90%, vorzugsweise mindestens 95%, bevorzugt 96%, 97%, 98%, 99% oder 100% identisch mit der Aminosäuresequenz SEQ ID Nr. 5 ist, katalysiert werden.

Vorzugsweise wird das Verfahren durch Mirkoorganismen durchgeführt. Die für das Verfahren verwendeten Mikroorganismen können dabei die Fähigkeit an sich besitzen, Aceton zu synthetisieren, oder können erst durch das genetische Pathway Engineering dazu befähigt werden, Aceton zu bilden. Dies kann durch Steigerung der zellulären Konzentration oder der Aktivität von Enzymen, die eine Acetat-unabhängige Aceton-Synthese ausgehend von Acetyl-CoA katalysieren erreicht werden.

Vorzugsweise wird das erfindungsgemäße Verfahren in gentechnisch veränderten Mikroorganismen durchgeführt. Solche können z.B. stammen aus eine Gattung ausgewält aus der Gruppe *Clostridium, Zymomonas, Escherichia, Salmonella, Rhodococcus, Pseudomonas, Bacillus, Lactobacillus, Enterococcus, Alcaligenes, Klebsiella, Paenibacillus, Arthrobacter, Corynebacterium, Brevibacterium, Pichia, Candida, Hansenula* und *Saccharomyces.* Beispielhafte Arten dieser Gattungen sind *Escherichia coli, Alcaligenes eutrophus, Bacillus licheniformis, Paenibacillus macerans, Rhodococcus erythropolis, Pseudomonas putida, Enterococcus faecium, Enterococcus gallinarium, Enterococcus faecalis, Bacillus subtilis* und *Saccharomyces cerevisiae.* In dem erfindungsgemäßen Verfahren wird bevorzugt ein Organismus; ausgewählt aus der Gruppe *Escherichia coli, Corynebacterium glutamicum, Clostridium spec., Clostridium aceticum, Acetobacterium woodii, Clostridium acetobutylicum, Clostridium beijerinckii, Yarrowia lipolytica, Saccharomyces spec., Saccharomyces cerevisiae* und *Pichia pastoris,* besonders bevorzugt *"E. coli* pUC19ayt", vgl. Beispiele 5 und 6, eingesetzt.

Rekombinante Mikroorganismen können durch die rekombinanten Gentechnikverfahren hergestellt werden, die dem Fachmann bekannt sind. Im Allgemeinen können die die Fremd-Gene tragende Vektoren in die Zellen durch konventionelle Transformations- oder Transfektionstechniken eingeschleust werden. Geeignete Methoden können gefunden werden bei Sambrook et al. (Molecular Cloning: A Laboratory Manual. 2nd ed., Cold Spring Harbor Laboratory, 1989). Bei den verwendeten Mikroorganismen kann es sich um Stämme handeln, die durch Mutagenese und Selektion, durch rekombinante DNA-Techniken oder durch eine Kombination beider Methoden hergestellt wurden. Für die Mutagenese können klassische *in vivo*-Mutageneseverfahren unter Verwendung mutagener Stoffe wie beispielsweise N-Methyl-N'-Nitro-N-Nitrosoguanidin oder ultraviolettes Licht verwendet werden. Weiterhin können für die Mutagenese *in vitro*-Methoden wie beispielsweise eine Behandlung mit Hydroxylamin (Miller, J. H.: A Short Course in Bacterial Genetics. A Laboratory Manual and Handbook for Escherichia coli and Related Bacteria, Cold Spring Harbor Laboratory Press, Cold Spring Harbor, 1992) oder mutagene Oligonukleotide (T. A. Brown: Gentechnologie für Einsteiger, Spektrum Akademischer Verlag, Heidelberg, 1993) oder die Polymerase-Kettenreaktion (PCR), wie sie im Handbuch von Newton und Graham (PCR, Spektrum Akademischer Verlag, Heidelberg, 1994) beschrieben ist, verwendet werden.

Weitere Anleitungen zur Erzeugung von Mutationen können dem Stand der Technik und bekannten Lehrbüchern der Genetik und Molekularbiologie wie z.B. dem Lehrbuch von Knippers ("Molekulare Genetik", 6. Auflage, Georg Thieme Verlag, Stuttgart, Deutschland, 1995), dem von Winnacker ("Gene und Klone", VCH Verlagsgesellschaft, Weinheim, Deutschland, 1990) oder dem von Hagemann ("Allgemeine Genetik", Gustav Fischer Verlag, Stuttgart, 1986) entnommen werden.

Bei Verwendung von *in*-*vitro*-Methoden wird das im Stand der Technik beschriebene Gen ausgehend von isolierter Gesamt-DNA eines Wildtypstammes mit Hilfe der Polymerase-Kettenreaktion amplifiziert, gegebenenfalls in geeignete Plasmidvektoren kloniert, und die DNA anschließend dem Mutageneseverfahren unterworfen. Anleitungen zur Amplifikation von DNA-Sequenzen mit Hilfe der Polymerase-Kettenreaktion (PCR) findet der Fachmann unter anderem im Handbuch von Gait: Oligonucleotide Synthesis: A Practical Approach (IRL Press, Oxford, UK, 1984) und bei Newton und Graham: PCR (Spektrum Akademischer Verlag, Heidelberg, Deutschland, 1994). In gleicher Weise können auch Methoden der *in*-*vitro*-Mutagenese verwendet werden wie sie beispielsweise in dem bekannten Handbuch von Sambrook et al. (Molecular Cloning, A Laboratory Manual, 2nd ed., Cold Spring Harbor Laboratory Press, Cold Spring Harbor, New York, USA, 1989) beschrieben sind. Entsprechende Methoden sind auch kommerziell in Form sogenannter "Kits" wie beispielsweise der von Papworth et al. (Strategies 9(3), 3-4 (1996)) beschriebene "QuikChange Site-Directed Mutagenesis Kit" der Firma Stratagene (La Jolla, USA) verfügbar.

Gegenstand der Erfindung sind ebenfalls Plasmide, die die erfindungsgemäß eingesetzten Polynukleotide enthalten und gegebenenfalls in den Bakterien replizieren. offenbart sind ebenfalls die rekombinanten Mikroorganismen, die mit den genannten Vektoren transformiert wurden. Dabei können die Polynukleotide unter der Wirkung eines einzigen oder mehrerer Promotoren stehen. Der Begriff "Verstärkung" beschreibt in diesem Zusammenhang die Erhöhung der intrazellulären Aktivität oder Konzentration eines oder mehrerer Enzyme oder Proteine in einem Mikroorganismus, die durch die entsprechende DNA kodiert werden, indem man beispielsweise die Kopienzahl des Gens bzw. der Gene, des ORFs bzw. der ORFs um mindestens eine (1) Kopie erhöht, einen starken Promotor funktionell mit dem Gen verknüpft oder ein Gen oder Allel oder ORF verwendet, das für ein entsprechendes Enzym bzw. Protein mit einer hohen Aktivität kodiert und gegebenenfalls diese Maßnahmen kombiniert. In *E. coli* werden exemplarisch *lac*, *tac* und *trp* als starke Promotoren genannt. Als offener Leserahmen (ORF, open reading frame) wird ein Abschnitt einer Nukleotidsequenz bezeichnet, der für ein Protein beziehungsweise Polypeptid oder Ribonukleinsäure kodiert oder kodieren kann, dem (der) nach dem Stand der Technik keine Funktion zugeordnet werden kann. Nach Zuordnung einer Funktion zu dem betreffenden Abschnitt der Nukleotidsequenz wird im Allgemeinen von einem Gen gesprochen. Unter Allelen versteht man im Allgemeinen alternative Formen eines gegebenen Gens. Die Formen zeichnen sich durch Unterschiede in der Nukleotidsequenz aus.

Als Genprodukt bezeichnet man im Allgemeinen das von einer Nukleotidsequenz, d.h. einem ORF, einem Gen oder einem Allel kodierte Protein oder die kodierte Ribonukleinsäure. Durch die Maßnahmen der Verstärkung, insbesondere Überexpression, wird die Aktivität oder Konzentration des entsprechenden Proteins im allgemeinen um mindestens 10%, 25%, 50%, 75%, 100%, 150%, 200%, 300%, 400% oder 500%, maximal bis 1000% oder 2000% bezogen auf die des Wildtyp-Proteins beziehungsweise auf die Aktivität oder Konzentration des Proteins im für das entsprechende Enzym bzw. Protein nicht rekombinanten Mikroorganismus oder Elternstamm erhöht. Als nicht rekombinanter Mikroorganismus oder Elternstamm (*parent strain*) wird der Mikroorganismus verstanden, an dem die Verstärkung oder Überexpression durchgeführt wird.
Die Gene oder Genkonstrukte können entweder in Plasmiden mit unterschiedlicher Kopienzahl vorliegen oder im Chromosom integriert und amplifiziert sein. Alternativ kann weiterhin eine Überexpression der betreffenden Gene durch Veränderung der Medienzusammensetzung und Kulturführung erreicht werden.

Gegenstand der Erfindung ist ein Verfahren, in dem genetisch veränderte Mikroorganismen Nukleinsäuren, die für Enzyme wie oben beschrieben proteinkodieren, die die Verfahrensschritte A bis C durchführen können, exprimieren.

Das erfindungsgemäße Verfahren ist dadurch gekennzeichnet, dass der Mikroorganismus mindestens enthält
eine Nukleinsäure a, proteinkodierend für mindestens ein Polypeptid
mit einer Ketothiolase-Aktivität und mit einer Aminosäuresequenz, die zu mindestens 90%, vorzugsweise mindestens 95%, bevorzugt 96%, 97%, 98%, 99% oder 100% identisch mit der Aminosäuresequenz SEQ ID Nr. 16 ist,
eine Nukleinsäure b, proteinkodierend für mindestens ein Polypeptid
welches eine Acetoacetat-CoA-Hydrolase, Acyl-CoA-Thioesterase, eine Acyl-CoA-Synthetase oder Acyl-CoA-Thiokinase ist,
oder mit Acetoacetat-CoA-Hydrolase-Aktivität und mit einer Aminosäuresequenz, die zu mindestens 90%, vorzugsweise mindestens 95%, bevorzugt 96%, 97%, 98%, 99% oder 100% identisch mit der Aminosäuresequenz SEQ ID Nr. 1 ist, oder mit Acetoacetat-CoA-Hydrolase-Aktivität und mit einer Aminosäuresequenz, die zu mindestens 90%, vorzugsweise mindestens 95%, bevorzugt 96%, 97%, 98%, 99% oder 100% identisch mit der Aminosäuresequenz SEQ ID Nr. 3 ist, oder mit Acetoacetat-CoA-Hydrolase-Aktivität und mit einer Aminosäuresequenz, die zu mindestens 90%, vorzugsweise mindestens 95%, bevorzugt 96%, 97%, 98%, 99% oder 100% identisch mit der Aminosäuresequenz SEQ ID Nr. 5 ist,
und eine Nukleinsäure c proteinkodierend für mindestens ein Polypeptid
mit einer Acetoacetatdecarboxylase-Aktivität und mit einer Aminosäuresequenz, die zu mindestens 90%, vorzugsweise mindestens 95%, bevorzugt 96%, 97%, 98%, 99% oder 100% identisch mit der Aminosäuresequenz SEQ ID Nr. 18 ist wobei die Nukleinsäuren a, b und c dafür verwendet werden, die Expression des Polypeptides im Mikroorganismus sicherzustellen.

Hierbei wird vorzugsweise eine Nukleinsäure a eingesetzt, die ausgewählt wird aus:
- aa: DNA-Sequenz beschrieben durch Sequenz 17 oder ihren Komplementärstrang,
- bb: DNA-Sequenzen, die unter stringenten Bedingungen zu den unter aa beschriebenen DNA-Sequenzen hybridisieren,
- cc: DNA-Sequenzen, die ohne die Entartung des genetischen Codes zu den in aa und bb definierten DNA-Sequenzen hybridisieren würden.

Als eine Nukleinsäure c wird vorzugsweise eine eingesetzt, die ausgewählt wird aus:
- dd: DNA-Sequenz beschrieben durch Sequenz 19 oder ihren Komplementärstrang,
- ee: DNA-Sequenzen, die unter stringenten Bedingungen zu den unter dd beschriebenen DNA-Sequenzen hybridisieren,
- ff: DNA-Sequenzen, die ohne die Entartung des genetischen Codes zu den unter dd und ee beschriebenen DNA-Sequenzen hybridisieren würden;

Als eine Nukleinsäure b wird vorzugsweise eine, die ausgewählt wird aus:
- gg: DNA-Sequenz beschrieben durch Sequenz 2 oder ihren Komplementärstrang,
- hh: DNA-Sequenzen, die unter stringenten Bedingungen zu den unter gg beschriebenen DNA-Sequenzen hybridisieren,
- ii: DNA-Sequenzen, die ohne die Entartung des genetischen Codes zu den unter gg und hh beschriebenen DNA-Sequenzen hybridisieren würden,
oder bevorzugt aus:
- jj: DNA-Sequenz beschrieben durch Sequenz 4 oder ihren Komplementärstrang,
- kk: DNA-Sequenzen, die unter stringenten Bedingungen zu den unter jj beschriebenen DNA-Sequenzen hybridisieren,
- ll: DNA-Sequenzen, die ohne die Entartung des genetischen Codes zu den unter jj und kk beschriebenen DNA-Sequenzen hybridisieren würden,
oder besonders bevorzugt aus:
- mm: DNA-Sequenz beschrieben durch Sequenz 6 oder ihren Komplementärstrang,
- nn: DNA-Sequenzen, die unter stringenten Bedingungen zu den unter mm beschriebenen DNA-Sequenzen hybridisieren,
- oo: DNA-Sequenzen, die ohne die Entartung des genetischen Codes zu den unter mm und nn beschriebenen DNA-Sequenzen hybridisieren würden,
eingesetzt.

In bevorzugten Ausführungsformen des erfindungsgemäßen Verfahrens liegen die Nukleinsäuren a, b und c auf einem Nukleotidstrang, der eine Expression aller drei Genprodukte ermöglicht.

Besonders bevorzugt liegen die Nukleinsäuren a, b und c auf einem Nukleotidstrang und dieser ist ausgewählt aus der Gruppe umfassend:
Plasmid pSKatt, mit der Nukleinsäuresequenz gemäß SEQ ID Nr. 7,
Plasmid pKSatt, mit der Nukleinsäuresequenz gemäß SEQ ID Nr. 8,
Plasmid pUC19att, mit der Nukleinsäuresequenz gemäß SEQ ID Nr. 9,
Plasmid pUC18att, mit der Nukleinsäuresequenz gemäß SEQ ID Nr. 10
   und
Plasmid pUC19ayt, mit der Nukleinsäuresequenz gemäß SEQ ID Nr. 11.

Gegenstand der Erfindung sind somit auch die Nukleinsäuren Plasmid pSKatt, mit der Nukleinsäuresequenz gemäß SEQ ID Nr. 7, Plasmid pKSatt, mit der Nukleinsäuresequenz gemäß SEQ ID Nr. 8, Plasmid pUC19att, mit der Nukleinsäuresequenz gemäß SEQ ID Nr. 9, Plasmid pUC18att, mit der Nukleinsäuresequenz gemäß SEQ ID Nr. 10 und Plasmid pUC19ayt, mit der Nukleinsäuresequenz gemäß SEQ ID Nr. 11.

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.
- Abbildung 1:: Biosynthese von Aceton, Butanol und Ethanol
- Abbildung 2:: Biosynthese von Aceton
- Abbildung 3:: Expression von TEII_{srf} in *E. coli* M15
- Abbildung 4:: Aufreinigung von TEII_{srf} aus E. coli
- Abbildung 5:: Lineweaver-Burk-Diagramme zur Ermittlung der Km-Werte von TEII_{srf} für die Substrate Acetyl-CoA und Acetoacetyl-CoA
- Abbildung 6:: Expression von AACS in *E. coli* BL21 (DE3)
- Abbildung 7:: Aufreinigung von AACS aus *E. coli*
- Abbildung 8:: Expression und Aufreinigung von YbgC
- Abbildung 9:: Schematische Darstellung der sukzessiven Klonierung der Gene *adc, teIIsrf* und *thlA* in das Plasmid pSK.
- Abbildung 10:: Erzeugung von pUC19-Konstrukten
- Abbildung 11:: Aceton- und Acetatbildung in 5 ml-Kulturen verschiedener Expressiosvektoren in E. *coli* HB101
- Abbildung 12:: Aceton- und Acetatbildung in 100-ml-Kulturen von *E. coli* HB101mit pUC19ayt (YbgC) und pUC19act (Kontrolle CtfA/B)

### Beispiele

### Beispiel 1: Thioesterase II (TEII_{srf}) aus Bacillus subtilis

Dieses Enzym ist mit den nicht-ribosomalen Peptid-Synthetasen zur Bildung von Surfactin (Peptid-Antibiotika) in *B. subtilis* ATCC 21332 assoziiert. Schwarzer *et al.* konnten das entsprechende Gen in das Plasmid pQE60, welches die Fusion des Zielproteins mit einem C-terminalen His-Tag vermittelt, klonieren (pTEIIsrf) und das Protein (28 kDa) aufreinigen (Schwarzer D., H. D. Mootz, U. Linne, M. A. Marahiel. 2002. Regeneration of misprimed nonribosomal peptide synthetases by type II thioesterases. PNAS 99:14083-14088). In anschließenden Untersuchungen wurde für dieses Protein eine hydrolytische Aktivität mit Acetyl-CoA und Propionyl-CoA als Substraten nachgewiesen.

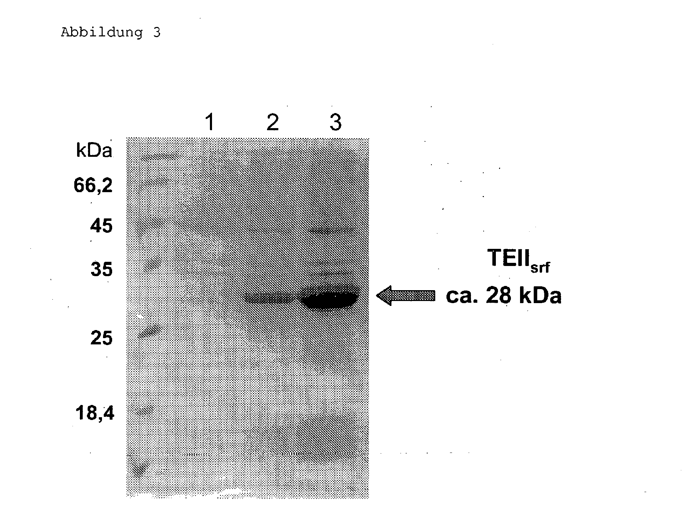

Das Plasmid pTEIIsrf mit SEQ ID Nr. 13 wurde hergestellt wie in Schwarzer D, Mootz HD, Linne U, Marahiel MA. Regeneration of misprimed nonribosomal peptide synthetases by type II thioesterases. Proc Natl Acad Sci U S A. 2002 Oct 29;99(22):14083-8 beschrieben.
Die Expression des Proteins erfolgte in *E. coli* M15 in LB_{Amp}-Medium bei 30 °C und 150 Upm und wurde bei einer optischen Dichte (OD₆₀₀ₙₘ) von 0, 6 - 0,8 mit 1 mM IPTG induziert. Nach weiteren 2 h Stunden wurde die Kultur geerntet. Während des Wachstums genommene Proben bestätigten die erfolgreiche Proteinexpression. Abb. 3 zeigt ein 12%iges SDS-Polyacrylamid-Gel nach Coomassiefärbung, auf das pro Spur 15 µl Rohextrakt aufgetragen wurde; 1: vor Induktion, 2: 1 h nach Induktion, 3: 2 h nach Induktion

Die vorgesehene Aktivitätsmessung mittels DTNB-Assay [5,5'-Dithiobis(2-nitrobenzoe-säure)], mit dem freie SH-Gruppen bestimmt werden, erforderte die Aufreinigung des Proteins. Dies erfolgte FPLC-gestützt über den fusionierten His-Tag von TEII_{srf} mittels Metall-Chelat-Affinitätschromatographie an Ni²⁺-NTA-Agarose und steigendem Imidazol-Gradienten.

Dazu wurden zunächst die Zellen geerntet (5000 x g, 15 min, 4 °C), in 3 ml/g Frischgewicht Zellaufschlußpuffer (50 mM HEPES, 300 mM NaCl, pH 7,8) suspendiert und ggf. bei -20 °C gelagert. Durch Ultraschallbehandlung (Sonicator Bandelin Sonopuls, Bandelin Berlin) wurden die Zellen aufgeschlossen und der Rohextrakt durch Zentrifugation (30000 x g, 30 min, 4 °C) gewonnen. Die Aufreinigung erfolgte an einer FPLC-Anlage (Pharmacia Biotech GmbH). Auf die nach Herstellerangaben entsprechend vorbereitete und äquilibrierte (50 mM HEPES, 300 mM NaCl, 30 mM Imidazol, pH 7) Ni²⁺-NTA-Agarose-Säule (Qiagen Superflow, Bettvolumen 5 ml) wurden 3,5 ml Rohextrakt aufgetragen. Anschließend wurde die Säule mit 50 ml Äquilibrierungspuffer gewaschen. Die Elution erfolgte mit einem linearen Imidazol-Gradienten über 50 ml (30 - 300 mM Imidazol in 50 mM HEPES, 300 mM NaCl, pH 7).

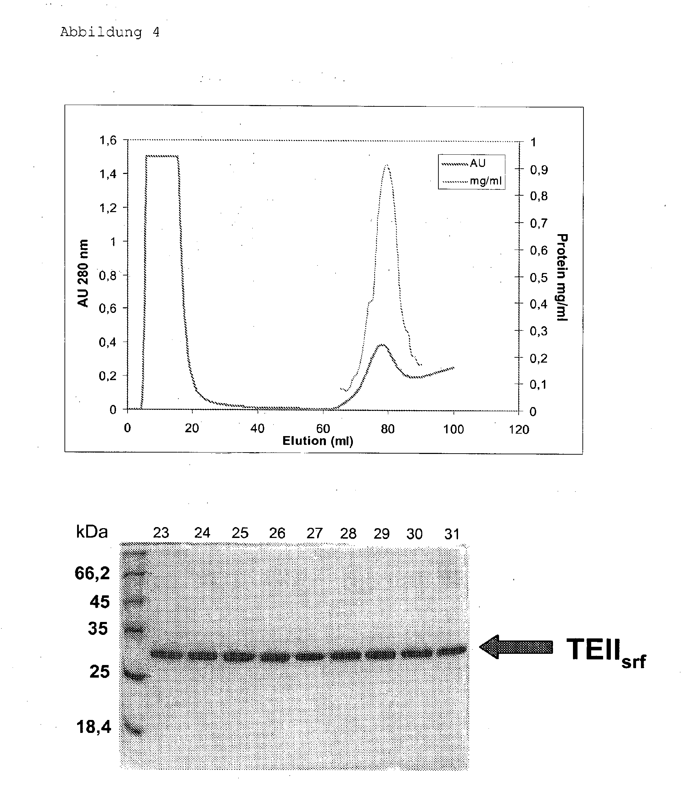

Abb. 4 zeigt im oberen Bereich das FPLC-Elutionsprofil an Ni²⁺-NTA-Agarose (Qiagen Superflow, Bettvolumen 5 ml) bei einem Programm mit: Waschen 0-50 ml, Elution 50-100 ml mit 30-300 mM Imidazol (linearer Gradient) und Fraktionsgrößen von je 1 ml; im unteren Bereich ist ein Coomassie gefärbtes 12 % SDS-PAGE-Gel abgebildet, auf das die Elutionsfraktionen 23-31, je µg Protein/Spur, aufgetragen sind.

Die erhaltenen Fraktionen wurden vereinigt und für die Aktivitätsbestimmung eingesetzt. Als Substrate dienten Acetoacetyl-CoA und zum Vergleich Acetyl-CoA. Im DTNB-Assay reagiert die durch die enzymatische Umsetzung zu Acetoacetat bzw. Acetat freigewordene endständige Sulfhydryl-Gruppe des CoA mit DTNB zu einem farbigen Produkt, das bei 412 nm photometrisch bestimmt werden kann.

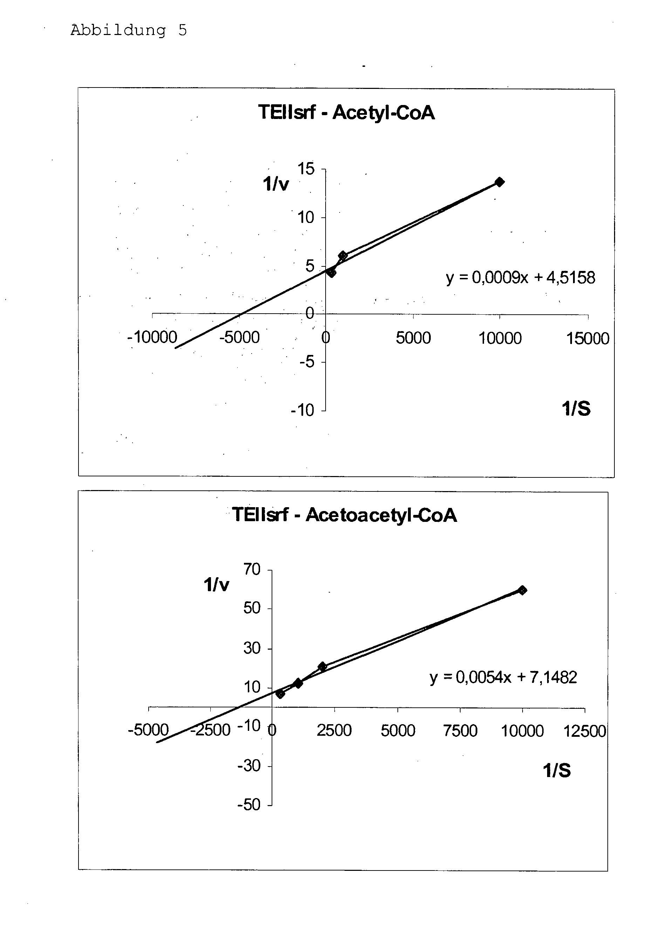

Folgende Km-Werte wurden bestimmt: Für Acetyl-CoA betrug dieser 2 · 10⁻⁴ mol · l⁻¹ (0,2 mM), für Acetoacetyl-CoA 7, 7 · 10⁻⁴ mol · l⁻¹ (0,77 mM). Damit weist TEII_{srf} eine höhere Substrataffinität zu Acetyl-CoA auf. Abb. 5 zeigt das Lineweaver-Burk-Diagramm zur Ermittlung der Km-Werte von TEII_{srf} für die Substrate Acetyl-CoA und Acetoacetyl-CoA.

### Beispiel 2: Acetoacetyl-CoA-Synthetase (AACS) aus Sinorhizobium meliloti

Die Acetoacetyl-CoA-Synthetase (AACS) mit Aminosäuresequenz SEQ ID Nr. 3 und korrespondierender cDNA-Sequenz SEQ ID Nr. 4 aus S. *meliloti* katalysiert die Umsetzung von Acetoacetat und Coenzym A unter Verbrauch von ATP zu Acetoacetyl-CoA und AMP. Von Interesse war im Rahmen der vroliegenden Erfindung allerdings die Rückreaktion.

Das entsprechende Gen *acsA2* wurde in den Expressionsvektor pET30 Xa/LIC kloniert wie in Aneja, P., R. Dziak, G.-Q. Cai, T. C. Charles. 2002. Identification.of an Acetoacetyl Coenzyme A Synthetase-Dependent Pathway for Utilization of L-(+)-3-Hydroxybutyrate in Sinorhizobium meliloti. J. Bacteriol. 184:1571-1577 offenbart. Das so erhaltene Plasmid "pRD112" vermittelt die N-terminale Fusion des Zielproteins mit einem His-Tag, der wiederum die affinitätschromatographische Aufreinigurig an Ni²⁺-NTA-Agarose ermöglicht.

Die Expression des Proteins erfolgte in *E. coli* BL21 (DE3)-Zellen bei 37 °C und 180 Upm. Bei einer optischen Dichte (OD₆₀₀ₙₘ) von 0,5-0,6 wurde die Kultur mit 1 mM IPTG induziert und anschließend für weitere 3 h inkubiert. Die erfolgreiche Expression des Proteins (ca. 72 kDa) wiesen Proben nach, die während des Wachstums genommen worden waren.

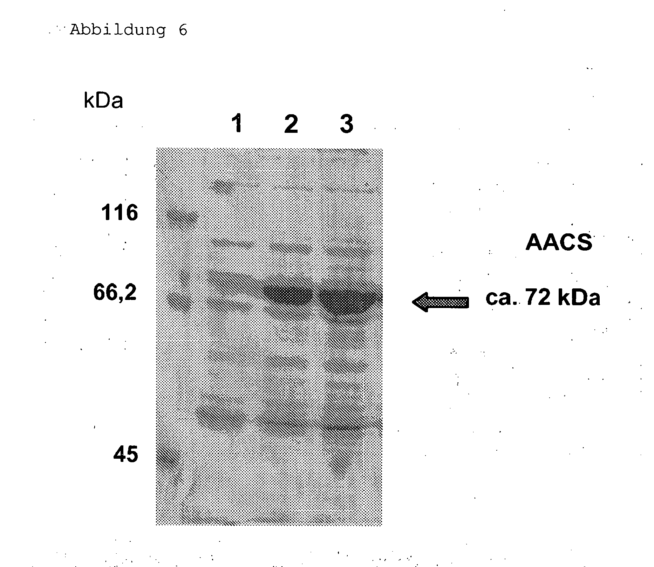

Abb. 6 stellt ein 7,5 %iges SDS-PAGE-Gel nach Coomassiefärbung dar; es wurden je 15 µl Rohextrakt/Spur (schneller Zellaufschluss) aufgetragen. 1: vor Induktion, 2: 1 h nach Induktion, 3: 2 h nach Induktion

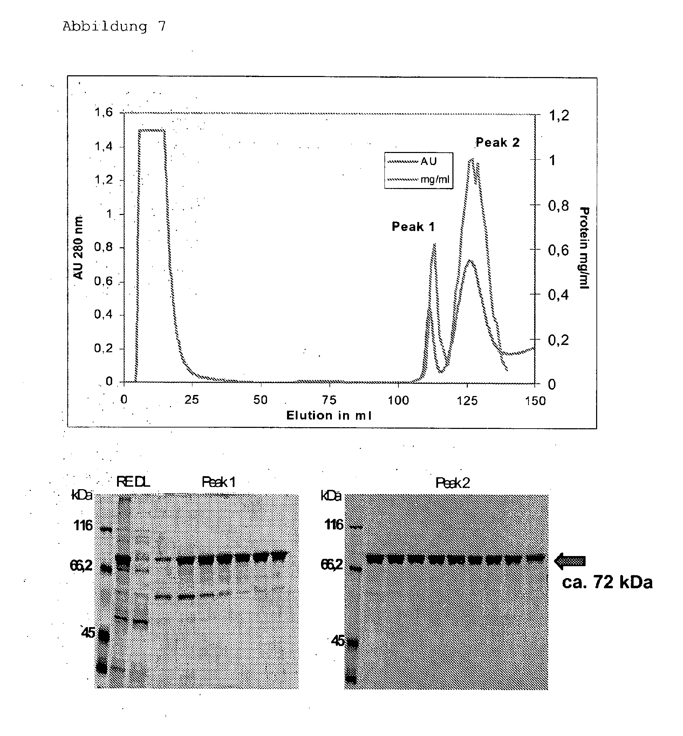

Die Aufreinigung erfolgte analog.derjenigen von TEII_{srf} mittels Metall-Chelat-Affinitätschromatographie an Ni²⁺-NTA-Agarose und einem Gradienten von 20-250 mM Imidazol (Abb. 7A). Dabei traten zwei Peaks auf (1 und 2), deren Fraktionen auf ihren Proteingehalt hin überprüft wurden. Eine anschließende Analyse in 7,5 %igen SDS-PA-Gelen zeigte, insbesondere beim ersten Peak, das Auftreten einer zusätzlichen Bande bei etwa 55 kDa. Da diese Bande im zweiten Peak deutlich schwächer war bzw. gar nicht auftrat, wurden die entsprechenden Fraktionen dieses Peaks vereinigt und zur Aktivitätsbestimmung eingesetzt. Als Substrate dienten wiederum Acetoacetyl-CoA und zum Vergleich Acetyl-CoA im DTNB-Assay. Die Bestimmung der Km-Werte ergab für Acetyl-CoA als Substrat einen Wert von 7 · 10⁻⁴ mol · l⁻¹ (0,7 mM). Für Acetoacetyl-CoA hingegen reichten die gemessenen Werte nicht zur Bestimmung eines Km-Wertes aus. Da jedoch die spezifische Aktivität des Enzyms mit Acetoacetyl-CoA lediglich 0,0038 µmol · min⁻¹ · mg⁻¹ betrug, wurde von einer weiteren Verwendung der AACS aus *S. meliloti* respektive einer Klonierung des entsprechenden Gens in Kombination mit den Clostridien-Genen abgesehen.

Abb. 7 zeigt die Aufreinigung von AAGS aus *E. coli*, im oberen Bereich ist das FPLC-Elutionsprofil an Ni²⁺-NTA-Agarose (Qiagen Superflow, Bettvolumen 5 ml) mit dem Programm Waschen 0-100 ml, Elution 100-150 ml mit 20-250 mM Imidazol (linearer Gradient), Fraktionsgröße je 1 ml dargesetllt; der untere Bereich zeigt ein 7,5 % SDS-PAGE-Gel nach Silberfärbung, ausgewählte Elutionsfraktionen von Peak 1 und Peak 2, je 1 µg Protein/Spur, RE: Rohextrakt, DL: Durchlauf.

### Beispiel 3: Acyl-CoA-Thioesterase YbgC aus Haemophilus influenzae

Das YbgC-Protein mit Aminosäuresequenz SEQ ID Nr. 5 und korrespondierender cDNA-Sequenz SEQ ID Nr. 6 aus *H. influenzae* weist Ähnlichkeiten zu einem entsprechenden Protein aus *E. coli* auf, wo dieses als cytoplasmatisches Protein zum so genannten Tol-Pal-System gehört. Dieses ist in Gram-negativen Bakterien weit verbreitet. Es ist wichtig für die Aufrechterhaltung der Zellwand-Integrität und hat möglicherweise eine Funktion beim Transport von Stoffen durch das Periplasma. Die Funktion von YbgC in *H. influenzae* hingegen und etwaige Beziehungen zu Funktionen des Tol-Pal-Systems sind bisher nicht publiziert. Die Veröffentlichung von Zhuang et al. (2002) beschreibt jedoch Untersuchungen der katalytischen Funktion dieses Proteins und die Analyse einer Thioesterase-Aktivität. Dabei konnte die Hydrolyse von kurzkettigen aliphatischen Acyl-CoA-Estern, wie z. B. Propionyl-CoA und Butyryl-CoA (Km-Werte 11-24 mM) durch YbgC gezeigt werden (Zhuang Z., F. Song, B. M. Martin, D. Dunaway-Mariano. 2002. The YbgC protein encoded by the ybgC gene of the tol-pal gene cluster of Haemophilus infuenzae catalyzes acyl-coenzyme A thioester hydrolysis. FEBS Lett. 516:161-163).

Ein *in*-*vitro*-Nachweis einer Aktivität mit Acetyl-CoA und Acetoacetyl-CoA im Rahmen dieses Vorhabens setzte erneut die Aufreinigung des Proteins voraus. Ausgehend von genomischer DNA wurde das Gens in ein anderes Expressionssystem, das Impact^{™} (Intein Mediated Purification with an Affinity Chitin-binding-Tag)-System der Firma NEB (Frankfurt a. M.) einkloniert. Sein Vorteil besteht darin, dass diese Methode eine einfache Aufreinigung eines rekombinanten Proteins in seiner nativen Form, d. h. ohne Tag ermöglicht.

Die Klonierung von *ybgc* in den Vektor pTYB1 (NEB Frankfurt a. M.) ermöglichte die Expression und anschließende Aufreinigung der Acyl-CoA-Thioesterase YbgC aus E. *coli..*

Das Gen *ybgc* wurde mittels PCR von genomischer DNA mit den Primern ybgcTYB1Ndefw (ATA TAC ATA TGT TGG ATA ATG GCT TTT C) und ybgcTYB1Xhorev (TCC GAA CTC GAG TTT TAA GTG ATG) amplifiziert. Dabei vermittelten die Primer den Einbau einer NdeI-Schnittstelle am 5'-Ende bzw. einer XhoI-Schnittstelle am 3'-Ende des amplifizierten Fragmentes. Es wurde der Taq-Mastermix (Qiagen, Hilden) nach den Angaben des Herstellers unter folgenden Bedingungen eingesetzt:

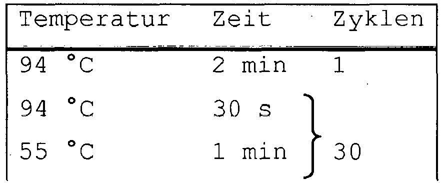

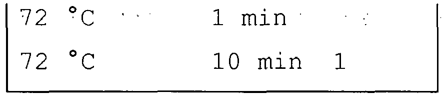

Es wurde ein Fragment der erwarteten Größe von etwa 430 Bp erhalten. Dieses wurde zunächst in den pJET-Vektor (Fermentas, St. Leon-Rot) gemäß den Herstellerangaben subkloniert (pJet_ybgcTYB, 410 Bp). Anschließend wurde das ybgc-Fragment aus diesem Plasmid mit den Restriktionsendonukleasen NdeI und *Xho*I wieder herausgeschnitten und geleluiert (Gel extraction Kit; peqlab Biotechnologie GmbH, Erlangen, nach Herstellerangaben). Der Vektor pTYB1 (7477 Bp) wurde ebenfalls mit NdeI und *Xho*I geschnitten (7439 Bp) und anschließend mit dem geschnittenen und geleluierten ybgc-Fragment unter Verwendung des Rapid Ligation Kits nach den Herstellerangaben (Fermentas GmbH, St. Leon-Rot) ligiert. Je 10 µl Ligationsansatz wurden zur Transformation von 100 µl CaCl₂-kompetenten *E. coli* XL1-B-Zellen eingesetzt. Erhaltene Klone wurden anschließend in LB-Ampicillin-Flüssigmedium angezogen und die Plasmid-DNA isoliert. Die isolierten Plasmide wurden mittels Restriktionsanalyse (*Nde*I/*Xho*I) überprüft und sequenziert (Agowa GmbH, Berlin). Das entstandene Plasmid wurde mit pTYBybgc (SEQ ID Nr. 15) bezeichnet und für die weitere Transformation in E. *coli* BL21 DE3 eingesetzt.

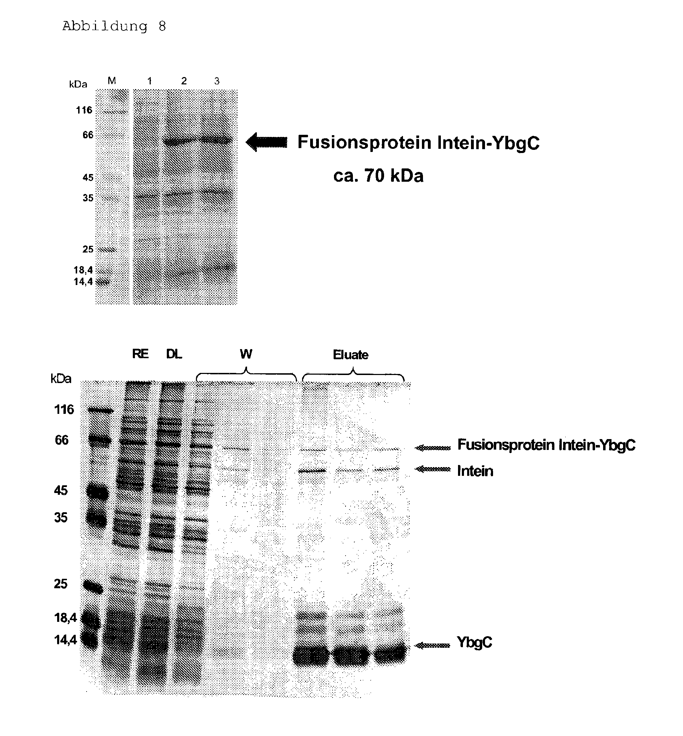

Die Expression erfolgte in *E. coli* BL21 (DE3) in LB_{Amp}-Medium bis zur Induktion bei einer OD₆₀₀₀ₙₘ von ~ 0,5 mit 0,5 mM IPTG bei 37 °C, danach wurde die Kultur 6 h bei 20 °C und 150 Upm weiter inkubiert. Das heterolog exprimierte Fusionsprotein (ca. 70 kDa) bindet an Chitin. Durch Zugabe von DTT wird eine Konformationsänderung bedingt und das Herausschneiden des Zielproteins (ca. 15 kDa) induziert, welches anschließend eluiert werden kann. Die Abbildung 8 zeigt SDS-PAGE-Gele. Im oberen Bereich ist eine SDS-PAGE zur Analyse der Expression von YbgC dargestellt. Es ist ein 10-20 %iges Gradienten-SDS-PA-Gel nach Coomassiefärbung. Es wurden je 15 µl Rohextrakt/Spur (schneller Zellaufschluss) aufgetragen. 1: vor Induktion, 2, 3: 6 h nach Induktion. Im unteren Teil ist der Verlauf der Aufreinigung von YbgC an Chitin-Säulen in einem 10-20 %iges Gradienten-SDS-PA-Gel nach Silberfärbung dargestellt. Es wurden je 2 µg Protein/Spur aufgetragen; -RE: Rohextrakt, DL: Durchlauf, W: Waschfraktionen, Eluate: proteinhaltige Elutionsfraktionen (Fraktionsgröße: je 1 ml).

Obwohl sich in den Elutionsfraktionen, neben dem gewünschten Protein von 15 kDa, noch Banden des Fusionsproteins und der abgespaltenen Intein-Domäne zeigten, wurden diese zur *in vitro-*Aktivitätsbestimmung im DTNB-Assay herangezogen. Die Messungen zur Ermittlung des Km-Wertes schwanken zwar (Daten nicht gezeigt), es ließen sich jedoch für Acetyl-CoA ein Km-Wert von 5,3 · 10⁻⁴ mol · l⁻¹ (0,53 mM) bzw. für Acetoacetyl-CoA als Substrat ein Km-Wert von 1,4 · 10⁻⁹ mol · l⁻¹ (0,14 mM) ermitteln. Damit konnte im direkten Vergleich eine sogar erhöhte Substrataffinität des Enzyms gegenüber Acetoacetyl-CoA bestimmt werden: Diese Ergebnisse sprachen dafür, YbgC in die weiteren Versuche zur Klonierung einzubeziehen.

### Beispiel 4: Klonierungsstrategie zur Konstruktion der Expressionsvektoren

Für die Acetonproduktion in *E*. *coli* wurde die Klonierung geeigneter Acyl-CoA-Thioesterase oder Acyl-CoA-Synthetase / Acyl-CoA-Thiokinase-Gene zusammen mit den Genen *thlA* (Genprodukt mit Aminosäuresequenz mit SEQ ID Nr. 16 und korrespondierender cDNA-Sequenz SEQ ID Nr. 17, Thiolase A) und *adc* (Genprodukt mit Aminosäuresequenz mit SEQ ID Nr. 18 und korrespondierender cDNA-Sequenz SEQ ID Nr. 19, Acetoacetat-Decarboxylase) aus *C*. *acetobutylicum* durchgeführt. Hierfür wurden zunächst die Plasmide pSK und pKS ausgewählt. Es handelt sich um Kloniervektoren, die sich im Wesentlichen hinsichtlich der Orientierung der Multi Cloning Site (MCS) unterscheiden. Die MCS liegt jeweils innerhalb einer vorhandenen *lacZ*'-Gensequenz, die für das N-terminale Fragment der β-Galaktosidase kodiert und ein Blau-Weiß-Screening rekombinanter Plasmide erlaubt. Für die Versuche im Rahmen des Projekts stand die Expression der klonierten Gene unter der Kontrolle des induzierbaren lac-Promotors im Vordergrund. Dafür war der Vektor pSK vorgesehen. Des Weiteren wurde eine Variante durch die Integration in das Plasmid pKS erzeugt, bei der die Gene unter Kontrolle des konstitutiven Thiolase-Promotors aus *C*. *acetobutylicum* exprimiert werden sollten.

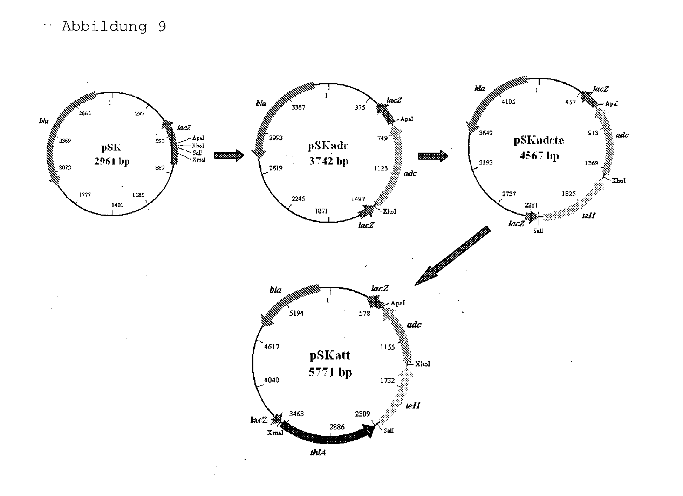

Die Klonierung der jeweiligen Gene in die Vektoren erfolgte sequentiell. Hierzu wurden zunächst Oligonukleotide für die Amplifikation der Gene unter Einführung entsprechender Schnittstellen entworfen und Polymerase-Kettenreaktionen durchgeführt. Alle Fragmente wurden amplifiziert und wie dargestellt in die Vektoren kloniert. Das strategische Vorgehen und die verwendeten Restriktionsschnittstellen im Fall von pSK sind in Abb. 9 schematisch verdeutlicht. Analog wurde mit dem Plasmid pKS verfahren.

### Im Folgenden werden die Klonierungsschritte im Detail offenbart:

Klonierung der Gene *adc*, *teII, thlA* in das Plasmid pKS: Ziel war es, die Gene *adc* (Acetacetat-Decarboxylase aus *Clostridium acetobutylicum*), *teII_{srf}* (Thioesterase II aus *Bacillus subtilis*) und *thlA* (Thiölase aus *C. acetobutylicum*) zusammen in einen Vektor zu klonieren und anschließend in *E. coli* zu exprimieren.

Das Acetacetat-Decarboxylase-Gen *adc* wurde mittels PCR vom Plasmid pDrive_adc (SEQ ID Nr. 20) mit den Primern AdcXhofwneu (CAT GCT CGA GAC GCG TTA CGT ATC) und AdcAparevneu (GAT GGG GCC CTG AAT TCT ATT ACT TAA G) amplifiziert. Dabei vermittelten die Primer den Einbau einer *Xho*I-Schnittstelle am 5'-Ende bzw. einer ApaI-Schnittstelle am 3'-Ende des amplifizierten Fragmentes. Es wurde die Polymerase GoTaq (Promega GmbH, Mannheim) nach den Angaben des Herstellers unter Zugabe von 4 mM MgCl₂ und folgenden Bedingungen eingesetzt:

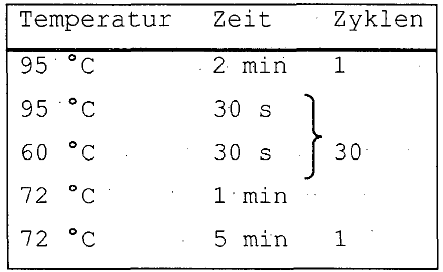

Es wurde ein Fragment der erwarteten Größe von etwa 800 Bp erhalten. Dieses wurde geleluiert (Gel extraction Kit; peqlab Biotechnologie GmbH, Erlangen, nach Hersteller-angaben) und anschließend, ebenso wie das Plasmid pKS, mit den Restriktionsendonukleasen *Xho*I und *Apa*I geschnitten (788 Bp) und der Vektor zusätzlich dephosphoryliert (Antarctic phosphatase, New England Biolabs GmbH, Frankfurt a. M., nach Herstellerangaben). Es schloss sich die Ligation des so behandelten Vektors und Fragments mit T4-Ligase (New England Biolabs GmbH, Frankfurt a. M.) nach den Herstellerangaben an. Je 10 µl Ligationsansatz wurden zur Transformation von 100 µl CaCl₂ kompetenten *E*. *coli* XL1-B-Zellen eingesetzt. Für jeden Ansatz wurden 100 µl CaCl₂-kompetente *E*. *coli* XL1-B-Zellen in vorgekühlte 1,5-ml-Reaktionsgefäße überführt und jeweils 10 µl Ligationsansatz dazugegeben, vorsichtig gemischt und die Ansätze für 30 min auf Eis inkubiert. Anschließend erfolgte ein Hitzeschock für 90 s bei 42 °C und die Zellen wurden danach erneut für 2 min auf Eis gestellt, je 0,5 ml vorgewärmtes LB-Medium dazugegeben und die Ansätze 60 min bei 37 °C unter leichtem Schütteln inkubiert. Von jedem Ansatz wurden 100 - 300 µl auf LB-Ampicillin-Medium ausplattiert und über Nacht bei 37 °C inkubiert. Die erhaltenen Klone wurden anschließend in LB-Ampicillin-Flüssigmedium angezogen und die Plasmid-DNA nach folgendem Protokoll isoliert: Zur schnellen Isolierung von Plasmid-DNA diente eine Modifikation der Schritte des ,Qiagen-Mini-Kits' ohne Säulenaufreinigung (Qiagen, Hilden), nach Birnboim und Doly (Birnboim, H. C., J. Doly. 1979. A rapid alkaline extraction procedure for screening recombinant plasmid DNA. Nucleic Acids Res. 7: 1513-1523). Die isolierten Plasmide wurden mittels Restriktionsanalyse (*Apa*I/*Xho*I) überprüft und positive Klone anschließend sequenziert (Agowa GmbH, Berlin). Dieses Plasmid wurde mit pKSadc bezeichnet und für die nachfolgenden Klonierungsschritte eingesetzt.

Das ThioesteraseII-Gen *teII_{srf}* wurde mittels PCR vom Plasmid pTEIIsrf mit den Primern TeIISalfw (CAA TTG TCG ACG GAT AAC AAT TTC ACA CAG A) und TeIIXhorev (CTA TCA ACT CGA GTC CAA GCT CAG CTA ATT AA) amplifiziert. Dabei vermittelten die Primer den Einbau einer *Sal*I-Schnittstelle am 5'-Ende bzw. einer *Xho*I-Schnittstelle am 3'-Ende des amplifizierten Fragmentes. Es wurde die Synergy-Polymerase (GeneCraft GmbH, Lüdinghausen) nach den Angaben des Herstellers unter folgenden Bedingungen eingesetzt:

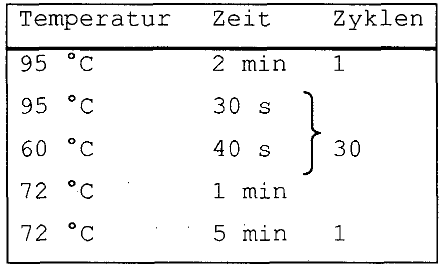

Es wurde ein Fragment der erwarteten Größe von etwa 850 Bp erhalten. Dieses wurde geleluiert (Gel extraction Kit; peqlab Biotechnologie GmbH, Erlangen, nach Hersteller-angaben) und anschließend, ebenso wie das Plasmid pKSadc, mit den Restriktionsendonukleasen *Sal*I und *Xho*I geschnitten (831 Bp) und der Vektor zusätzlich dephosphoryliert (Shrimp alkaline phosphatase SAP, Fermentas GmbH, St. Leon-Rot, nach Herstellerangaben). Es schloss sich die Ligation des so behandelten Vektors und Fragments unter Verwendung des Rapid Ligation Kits nach den Herstellerangaben (Fermentas GmbH, St. Leon-Rot) an. Je 10 µl Ligationsansatz wurden zur Transformation von 100 µl CaCl₂-kompetenten *E. coli* XL1-B-Zellen eingesetzt. Von jedem Ansatz wurden 100 - 300 µl auf LB-Ampicillin-Medium ausplattiert und über Nacht bei 37 °C inkubiert. Erhaltene Klone wurden anschließend in LB-Ampicillin-Flüssigmedium angezogen und die Plasmid-DNA isoliert. Die isolierten Plasmide wurden mittels Restriktionsanalyse (*Xho*I/*Sal*I*)* überprüft und positive Klone anschließend sequenziert (Agowa GmbH, Berlin). Dieses Plasmid wurde mit pKSadcte bezeichnet und für die nachfolgenden Klonierungsschritte eingesetzt.

Das Thiolase-Gen *thlA* wurde inklusive des Thiolase-Promotors mittels PCR von genomischer DNA aus *C. acetobutylicum* amplifiziert.

### Isolierung chromosomaler DNA von C. acetobutylicum:

Die Isolierung chromosomaler DNA aus C. *acetobutylicum* erfolgte prinzipiell nach Bertram (Bertram, J. 1989. Entwicklung eines Systems zum DNA-Transfer und zur Transposon-Mutagenese für *Clostridium acetobutylicum.* Dissertation, Universität Göttingen). Dazu wurden die Zellen anaerob in 2x YTG-Medium angezogen und bei einer OD₆₀₀ von ca. 1,2 geerntet. Die Zellen wurden durch Zentrifugation (5 min, 5.000 x g, 4 °C) sedimentiert. Das Zellsediment wurde dreimal mit 10 ml 1x TAE-Puffer (supplementiert mit 10 % [w/v] Saccharose) gewaschen und dann bei -20 °C gelagert. Die DNA aus 90 ml so behandelter Zellsuspension wurde wie folgt gewonnen:
- 1.: Suspendieren des Pellets in 3,8 m1 1x TAE mit Saccharose
- 2.: Zugabe von 1 ml Lysozym-RNase-Lsg.
- 3.: Inkubation: 30 min, 37 °C
- 4.: Zugabe von 500 µl 0,5 M EDTA (pH 8,0), 40 µl Tris-HCl (pH 8,0), 30 µl SDS (10 % [w/v])
- 5.: Inkubation: 10 min, 37 °C
- 6.: Zugabe von 200 µl Proteinase K-Lsg.
- 7.: Inkubation: 2 h, 37 °C
- 8.: Zugabe von 1,4 ml 5 M Na-Perchlorat,
- 9.: Zugabe von 7,3 ml Chloroform-Isoamylalkohol (24:1 [v/v]) auf Eis
- 10.: Zentrifugation: 10 min, 5.000 x g, 4°C
- 11.: Abnehmen der oberen, wässrigen Phase
- 12.: Zweimalige Wiederholung der Schritte 9. - 11.
- 13.: Fällung der DNA aus der wässrigen Phase durch Zugabe von 1 Vol. Isopropanol
- 14.: Inkubation: 5 min, RT
- 15.: Zentrifugation: 20 min, 16.000 x g, 4 °C
- 16.: Trocknung des Pellets bei RT für max. 2 h
- 17.: Suspendieren des Pellets in 2 ml TE-Puffer
- 18.: Zugabe von 200 µl Proteinase K-Lsg.
- 19.: Inkubation: über Nacht, 37 °C
- 20.: Erhöhung des Volumens auf 4 ml mit A. *dest.*
- 21.: Zugabe von 600 µl 3 M Na-Acetat (pH 5,2)
- 22.: Dreimalige Extraktion mit Chloroform-Isoamylalkohol (siehe Schritte 9. - 11.)
- 23.: Fällung der DNA durch Zugabe von 1 Vol. Isopropanol
- 24.: Inkubation: 5 min, RT
- 25.: Zentrifugation: 20 min, 16.000 x g, 4 °C
- 26.: Zweimaliges Waschen des Pellets mit 96 % (v/v) Ethanol (reinst, eiskalt)
- 27.: Trocknung des Pellets bei RT für maximal 2 h
- 28.: Suspendieren des Pellets in 200 µl TE

Die für die PCR eingesetzten Primer PthlAXmafw (CAT GAT TTC CCG GGG GTT AGC ATA TG) und PthlASalrev (CAG AGT TAT TTT TAA GTC GAC TTT CTA GCA C) vermittelten den Einbau einer XmaI-Schnittstelle am 5'-Ende bzw. einer SalI-Schnittstelle am 3'-Ende des amplifizierten Fragmentes. Es wurde der Taq-Mastermix (Qiagen, Hilden) nach den Angaben des Herstellers unter folgenden Bedingungen eingesetzt:

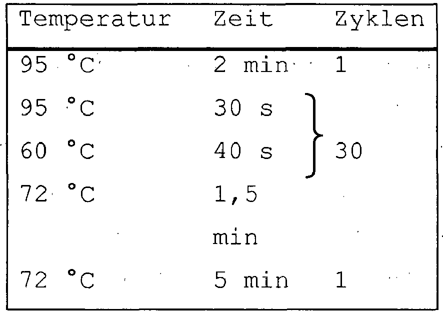

Es wurde ein Fragment der erwarteten Größe von etwa 1400 Bp erhalten. Dieses wurde zunächst in den pJET-Vektor (Fermentas, St. Leon-Rot) gemäß den Herstellerangaben subkloniert (pJet_PthlA). Anschließend wurde das *thlA*-Fragment aus diesem Plasmid mit den Restriktionsendonukleasen *Xho*I und *Cfr*9I wieder herausgeschnitten (1397 Bp) und geleluiert (Gel extraction Kit; peqlab Biotechnologie GmbH, Erlangen, nach Herstellerangaben). Das Plasmid pKSadcte wurde ebenfalls mit *Xho*I und *Cfr9I* behandelt und zusätzlich dephosphoryliert (Shrimp alkaline phosphatase SAP, Fermentas GmbH, St. Leon-Rot, nach Herstellerangaben). Es schloss sich die Ligation des so behandelten Vektors und Fragments unter Verwendung des Rapid Ligation Kits nach den Herstellerangaben (Fermentas GmbH, St. Leon-Rot) an. Je 10 µl Ligationsansatz wurden zur Transformation von 100 µl CaCl₂-kompetenten *E. coli* XL1-B-Zellen eingesetzt.

Erhaltene Klone wurden anschließend in LB-Ampicillin-Flüssigmedium angezogen und die Plasmid-DNA isoliert. Die isolierten Plasmide wurden mittels Restriktionsanalyse (*Sal*I/Cfrl9I) überprüft und positive Klone anschließend sequenziert (Agowa GmbH, Berlin).

Dieses Plasmid mit SEQ ID Nr. 8 wurde mit pKSatt bezeichnet und für die weitere Transformation in verschiedene *E. coli*-Stämme eingesetzt, welche nachfolgend auf die Bildung von Aceton untersucht wurden.

### Klonierung der Gene adc, teII_{srf}, thlA in das Plasmid pSK:

Für die Klonierung der drei Gene in den Vektor pSK wurde zunächst das bereits in pKS vorliegende *adc-teII_{srf}*-Fragment durch Restriktion mit *Apa*I und *S*alI herausgeschnitten (1625 Bp) und geleluiert (Gel extraction Kit; peqlab Biotechnologie GmbH, Erlangen, nach Herstellerangaben) und in den ebenso, geschnittenen und dephosphorylierten pSK-Vektor (SAP, Fermentas GmbH, St. Leon-Rot, nach Herstellerangaben) ligiert. Hierfür wurde der Quick Ligation Kit (New England Biolabs GmbH, Frankfurt a. M.) entsprechend den Herstellerangaben verwendetZur Überprüfung der erhaltenen Klone wurden diese anschließend in LB-Ampicillin-Flüssigmedium angezogen und die Plasmid-DNA isoliert. Die isolierten Plasmide wurden mittels Restriktionsanalyse (*Apa*I/*Sal*I) überprüft. Das so erhaltene Plasmid wurde mit pSKadcte bezeichnet und für den nachfolgenden Klonierungsschritt weiter verwendet.

Das Thiolase-Gen *thlA* wurde mittels PCR vom Plasmid pDrive_thl (SEQ ID Nr. 21) mit den Primern ThlAXmafw (GTC GAC CCG GGT CAA AAT TTA GGA G) und ThlASalrev (GCT TGT CGA ATT CAG ATC AGA G) amplifiziert. Dabei vermittelten die Primer den Einbau einer XmaI-Schnittstelle am 5'-Ende bzw. einer *Sal*I-Schnittstelle am 3'-Ende des amplifizierten Fragmentes. Es wurde der Taq-Mastermix (Qiagen), Hilden) nach den Angaben des Herstellers unter folgenden Bedingungen eingesetzt:

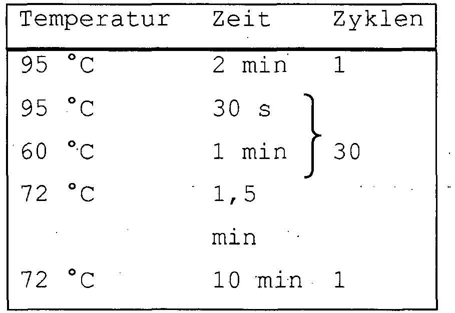

Es wurde ein Fragment der erwarteten Größe von etwa 1250 Bp erhalten. Dieses wurde zunächst in den pJET-Vektor (Fermentas, St. Leon-Rot) gemäß den Herstellerangaben subkloniert (pJet_thlA). Anschließend wurde das *thlA*-Fragment aus diesem Plasmid mit den Restriktionsendonukleasen *Xho*I und *Cfr*9I wieder herausgeschnitten (1243 Bp) und geleluiert (Gel extraction Kit; peqlab Biotechnologie GmbH, Erlangen, nach Herstellerangaben). Das Plasmid pSKadcte wurde ebenfalls mit *XhoI* und *Cfr*9I behandelt und zusätzlich dephosphoryliert (Shrimp alkaline phosphatase SAP, Fermentas GmbH, St. Leon-Rot, nach Herstellerangaben). Es schloss sich die Ligation des so behandelten Vektors und Fragments unter Verwendung des Quick Ligation Kit (New England Biolabs GmbH, Frankfurt a. M.) entsprechend den Herstellerangaben an. Je 10 µl Ligationsansatz wurden zur Transformation von 100 µl CaCl₂-kompetenten *E. coli* XL1-B-Zellen eingesetzt. Erhaltene Klone wurden anschließend in LB-Ampicillin-Flüssigmedium angezogen und die Plasmid-DNA isoliert. Die isolierten Plasmide wurden mittels Restriktionsanalyse (*Sal*I/Cfrl9I) überprüft und positive Klone anschließend sequenziert (Agowa GmbH, Berlin).

Dieses Plasmid mit SEQ ID Nr. 7 wurde mit pSKatt bezeichnet und für die weitere Transformation in verschiedene *E. coli*-Stämme eingesetzt.

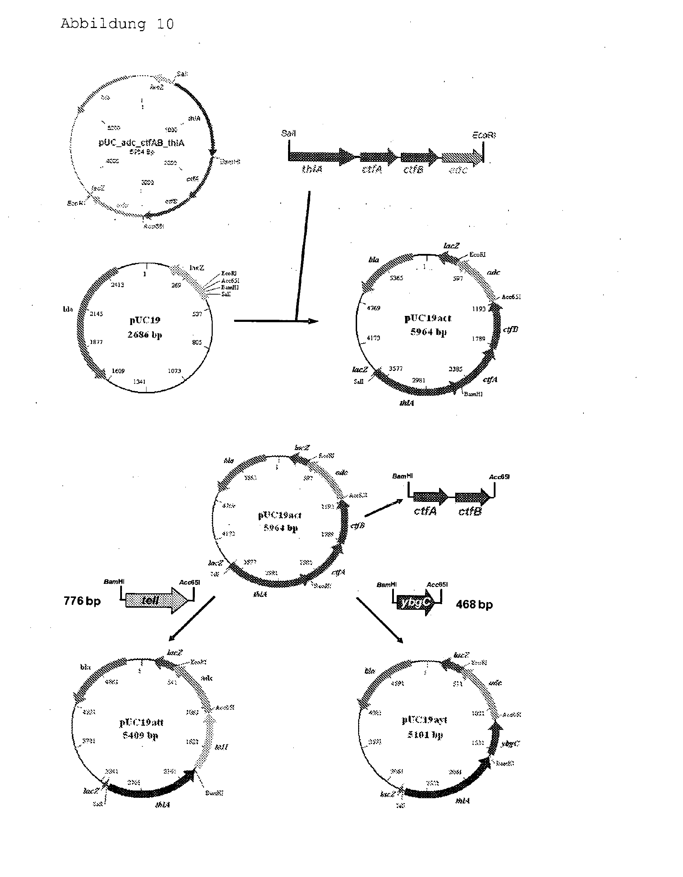

Auf Grundlage des pUC18-Konstruktes mit SEQ ID Nr. 14 (pUCadc_ctfA/B_thlA; s. Abb. 10), in welchem die clostridiellen Gene *adc, ctfA*/*B* und *thlA* bereits kloniert vorlagen, konnte einerseits eine zusätzliche Variante in dem Vektor pUC19 erzeugt werden, in dem diese Genkassette unter der Kontrolle des *lac-*Promotors exprimiert- wird. Darüber hinaus konnten anschließend die Gene *ctfA*/*B* spezifisch herausgeschnitten und, nach Einführung der passenden Schnittstellen in die Fragmente, statt dessen die Gene *teII_{srf}* bzw. *ybgC* inseriert werden (Abb. 9). Im pUC18-Konstrukt wurde außerdem das ursprügliche *thlA*-Fragment entfernt und durch ein *thlA*-Fragment ersetzt, das zusätzlich ,vorgeschaltet' die *thl*-Promotor-Sequenz enthielt.

Abb. 10 zeigt schematisch die Erzeugung von pUC19-Konstrukten auf; oben die schematische Darstellung der Vorgehensweise bei der Klonierung der Genkassette *adc*/*ctfAB*/*thlA* in das Plasmid pUC19, unten die schematische Darstellung der Vorgehensweise bei der Klonierung von *teII_{srf}* bzw. *ybgC* ausgehend von Plasmid pUC19act.

### Die Klonierungen im Detail:

### Konstruktion des Plasmids pUC19act

Grundlage hierfür war das Plasmid pUCadc_ctfA/B_thlA mit SEQ ID Nr. 14 welches die clostridiellen Gene Acetacetat-Decarboxylase (*adc*), CoA-Transferase (*ctfA*/*B*) und Thiolase (*thlA*), kloniert in den Vektor pUC18 darstellt. Die Gene sind hier in entgegen gesetzter Orientierung zum *lacZ*-Gen kloniert. Damit die Gene unter Kontrolle des lac-Promotors transkribiert werden können, wurde das adc_ctfA/B_thlA-Fragment (3311 Bp) mit den Restriktionsendonukleasen *Sal*I und *Eco*RI wieder herausgeschnitten und in den Vektor pUC19, der mit den gleichen Enzymen geschnitten worden war, ligiert (Rapid Ligation Kit nach den Herstellerangaben; Fermentas GmbH, St. Leon-Rot). Je 10 µl Ligationsansatz wurden zur Transformation von 100 µl CaCl₂-kompetenten *E. coli* XL1-B-Zellen eingesetzt. Erhaltene Klone wurden anschließend in LB-Ampicillin-Flüssigmedium angezogen und die Plasmid-DNA isoliert. Die isolierten Plasmide wurden mittels Restriktionsanalyse (*Sal*I/*EcoR*I) überprüft. Dieses Plasmid mit SEQ ID Nr. 12 wurde mit pUC19act bezeichnet und für die weitere Transformation in verschiedene *E*. *coli*-Stämme eingesetzt, welche nachfolgend auf die Bildung von Aceton untersucht wurden. Dabei diente dieses Konstrukt, welches die clostridiellen Gene zur Acetonbildung enthält, als Vergleichsmöglichkeit zu den restlichen Konstrukten.

### Konstruktion des Plasmids pUC18att

Grundlage hierfür war das Plasmid pUCadc_ctfA/B_thlA mit SEQ ID Nr. 14 welches die clostridiellen Gene für die Acetacetat-Decarboxylase (*adc*), CoA-Transferase *(ctfA*/*B)* und Thiolase (*thlA*), kloniert in den Vektor pUC18 darstellt.

Das ThioesteraseII-Gen te*II_{srf}* wurde mittels PCR vom Plasmid pTEIIsrf mit den Primern TeIIpUCBamfw (CAA TTG GGA TCC GAT AAC AAT TTC ACA CAG) und TeIIpUCAccrev (GAG ATC TGG TAC CCG GTT AAA TGA TCG GA) amplifiziert. Dabei vermittelten die Primer den Einbau einer *Bam*HI-Schnittstelle am 5'-Ende bzw. einer *Acc65*I-Schnittstelle am 3'-Ende des amplifizierten Fragmentes. Es wurde die Synergy-Polymerase (GeneCraft GmbH, Lüdinghausen) nach den Angaben des Herstellers unter folgenden Bedingungen eingesetzt:

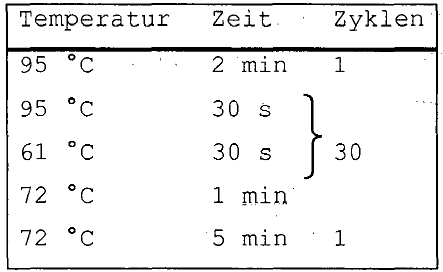

Es wurde ein Fragment der erwarteten Größe von etwa 800 Bp erhalten. Dieses wurde zunächst in den pJET-Vektor (Fermentas, St. Leon-Rot) gemäß den Herstellerangaben subkloniert (pJet_teIIpUC). Anschließend wurde das *teII_{srf}* -Fragment aus diesem Plasmid mit den Restriktionsendonukleasen *Bam*HI und *Acc*65I wieder herausgeschnitten (776 Bp) und geleluiert (Gel extraction Kit; peqlab Biotechnologie GmbH, Erlangen, nach Herstellerangaben). Der Vektor pUCadc_ctfA/B_thlA wurde ebenfalls mit den Restriktionsenzymen *Bam*HI und *Acc*65I behandelt, so dass das *ctfA*/*B*-Fragment (1331 Bp) herausfiel. Der Ansatz wurde in einem Agarosegel aufgetrennt und die Bande mit dem verbliebenen Vektor (4633 Bp) geleluiert (Gel extraction Kit; peqlab Biotechnologie GmbH, Erlangen, nach Herstellerangaben). Es schloss sich die Ligation des Vektors und des Fragments unter Verwendung des Rapid Ligation Kits nach den Herstellerangaben (Fermentas GmbH, St. Leon-Rot) an. Je 10 µl Ligationsansatz wurden zur Transformation von 100 µl CaCl₂-kompetenten *E. coli* XL1-B-Zellen eingesetzt. Erhaltene Klone wurden anschließend in LB-Ampicillin-Flüssigmedium angezogen und die Plasmid-DNA isoliert. Die isolierten Plasmide wurden mittels Restriktionsanalyse (BamHI/*Acc*65I) überprüft. Das entstandene Plasmid pUC18att_sub wurde für die Klonierung des *thlA*-Fragments mit Thiolase-Promotor weiterverwendet.

Das Thiolase-Gen *thlA* wurde inklusive des Thiolase-Promotors mittels PCR von genomischer DNA aus C. *acetobutylicum* amplifiziert. Die für die PCR eingesetzten Primer PthlApUCSalfw (CAT GAT TTG TCG ACG GTT AGC ATA TG) und PthlApUCBamrev (CAG AGT TAT TTT TAA GGA TCC TTT CTA GC) vermittelten den Einbau einer *Sal*I-Schnittstelle am 5'-Ende bzw. einer *Bam*HI-Schnittstelle am 3'-Ende des amplifizierten Fragmentes. Es wurde der Taq-Mastermix (Qiagen, Hilden) nach den Angaben des Herstellers unter Zugabe von 2,5 mM MgSO₄ und folgenden Bedingungen eingesetzt:

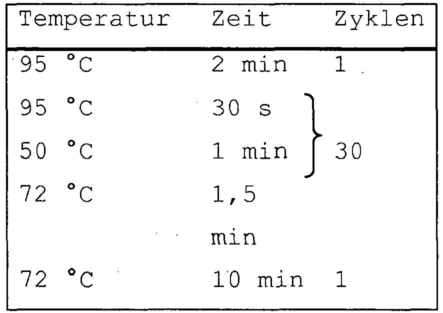

Es wurde ein Fragment der erwarteten Größe von etwa 1400 Bp erhalten. Dieses wurde zunächst in den pJET-Vektor (Fermentas, St. Leon-Rot) gemäß der Herstellerangaben subkloniert (pJet_PthlApUC). Anschließend wurde das *thlA*-Fragment aus diesem Plasmid mit den Restriktionsendonukleasen *Sal*I und *Bam*HI wieder herausgeschnitten (1397 Bp) und geleluiert (Gel extraction Kit; peqlab Biotechnologie GmbH, Erlangen, nach Herstellerangaben). Aus dem im Vorfeld konstruierten Plasmid pUC18att_sub wurde mit *Sal*I und *BamHI* das Thiolase-Fragment (ohne Promotor, 1217 Bp) herausgeschnitten, der verbleibende Vektor (4192 Bp) geleluiert und das Thiolase-Fragment inklusive Promotor (1397 Bp) einligiert (Rapid Ligation Kit nach den Herstellerangaben; Fermentas GmbH, St. Leon-Rot). Je 10 µl Ligationsansatz wurden zur Transformation von 100 µl CaCl₂-kompetenten *E. coli* XL1-B-Zellen eingesetzt. Erhaltene Klone wurden anschließend in LB-Ampicillin-Flüssigmedium angezogen und die Plasmid-DNA isoliert. Die isolierten Plasmide wurden mittels Restriktionsanalyse *(Sal*I/*Bam*HI*)* überprüft und sequenziert (Agowa GmbH, Berlin). Dieses Plasmid wurde mit pUC18att (SEQ ID Nr. 10) bezeichnet und für die weitere Transformation in verschiedene *E. coli*-Stämme eingesetzt, welche nachfolgend auf die Bildung von Aceton untersucht wurden.

### Konstruktion des Plasmids pUC19att

Grundlage hierfür war das Plasmid pUC19act, welches die clostridiellen Gene für die Acetacetat-Decarboxylase (*adc*), CoA-Transferase (*ctfA*/*B*) und Thiolase (*thlA*), kloniert in den Vektor pUC19 darstellt.

Das *teII_{srf}*-Fragment wurde mit den Restriktionsenzymen *Bam*HI und *Acc*65I aus pJet_teIIpUC herausgeschnitten (776 Bp) und geleluiert (Gel extraction Kit; peqlab Biotechnologie GmbH, Erlangen, nach Herstellerangaben). Der Vektor pUC19act wurde mit den gleichen Enzymen geschnitten, so dass das *ctfA*/*B*-Fragment herausfiel. Nach Auftrennung im Agarose wurde die verbleibende Vektorbande (4633 Bp) geleluiert (Gel extraction Kit; peqlab Biotechnologie GmbH, Erlangen, nach Herstellerangaben). Anschließend folgte die Ligation von Vektor-Fragment und *teIIpUC*-Fragment. Hierfür wurde der Quick Ligation Kit (New England Biolabs GmbH, Frankfurt a. M.) entsprechend den Herstellerangaben verwendet. Die Transformation erfolgte wie oben beschrieben. Zur Überprüfung der erhaltenen Klone wurden diese anschließend in LB-Ampicillin-Flüssigmedium angezogen und die Plasmid-DNA isoliert. Die isolierten Plasmide wurden mittels Restriktionsanalyse (*Bam*HI/*Acc*65I) überprüft. Das so erhaltene Plasmid wurde mit pUC19att (SEQ ID Nr. 9) bezeichnet und für die weitere Transformation in verschiedene *E. coli*-Stämme eingesetzt, welche nachfolgend auf die Bildung von Aceton untersucht wurden.

### Konstruktion des Plasmids pUC19ayt

### Grundlage hierfür bildete das Plasmid pUC19att.

Das Gen *ybgc* für die Acyl-CoA-Thioesterase YbgC aus *Haemophilus influenzae* wurde mittels PCR von genomischer DNA mit den Primern ybgcpUCBamfw (CTC TAG AAG GAT CCT GTT TAA CTT TAA G) und ybgcpUCAccrev (ATT GGG TAC CTC ATT GCA TAC TCC G) amplifiziert.

Dabei vermittelten die Primer den Einbau einer *BamHI-*Schnittstelle am 5'-Ende bzw. einer Acc65I-Schnittstelle am 3'- Ende des amplifizierten Fragmentes. Es wurde der Taq-Mastermix (Qiagen, Hilden) nach den Angaben des Herstellers unter folgenden Bedingungen eingesetzt:

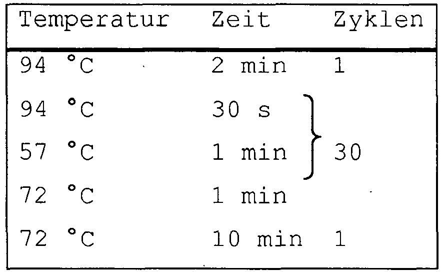

Es wurde ein Fragment der erwarteten Größe von etwa 490 Bp erhalten. Dieses wurde zunächst in den pJET-Vektor (Fermentas, St. Leön-Rot) gemäß den Herstellerangaben subkloniert (pJet-ybgcpUC). Anschließend wurde das *ybgc*-Fragment aus diesem Plasmid mit den Restriktionsendonukleasen *Bam*HI und *Acc*65I wieder herausgeschnitten (465 Bp) und geleluiert (Gel extraction Kit; peqlab Biotechnologie GmbH, Erlangen, nach Herstellerangaben).

Der Vektor pÜC19att wurde ebenfalls mit den Restriktionsenzymen *Bam*HI und *Acc*65I behandelt, so dass das *teII_{srf}*-Fragment (468 Bp) herausfiel. Der Ansatz wurde in einem Agarosegel aufgetrennt und die Bande mit dem verbliebenen Vektor (4633 Bp) geleluiert (Gel extraction Kit; peqlab Biotechnologie GmbH, Erlangen, nach Herstellerangaben). Es schloss sich die Ligation des Vektors und des Fragments unter Verwendung des Rapid Ligation Kits nach den Herstellerangaben (Fermentas GmbH, St. Leon-Rot) an. Je 10 µl Ligationsansatz wurden zur Transformation von 100 µl CaCl₂-kompetenten *E. coli* XL1-B-Zellen eingesetzt. Erhaltene Klone wurden anschließend in LB-Ampicillin-Flüssigmedium angezogen und die Plasmid-DNA isoliert. Die isolierten Plasmide wurden mittels Restriktionsanalyse (*Bam*HI/*Acc*65I) überprüft und sequenziert (Agowa GmbH, Berlin). Das entstandene Plasmid wurde mit pUC19ayt (SEQ ID Nr. 11) bezeichnet und für die weitere Transformation in verschiedene *E. coli*-Stämme eingesetzt, welche nachfolgend auf die Bildung von Aceton untersucht wurden.

Eine Übersicht über die vorliegenden Plasmidkonstrukte ist in Tabelle 1 zusammengestellt.

**Tab. 1: Plasmidkonstrukte**

| Plasmid | Größe in bp | Insert | Promotor |
|---|---|---|---|
| pSKatt | 5771 | *teII_{srf}* (*adc, thlA*) | lac |
| pKSatt | 5925 | *teII_{srf}* (*adc, thlA*) | thl |
| pUC19att | 5409 | *teII_{srf}* (*adc, thlA*) | lac |
| pUC18att | 5589 | *teII_{srf}* (*adc, thlA*) | thl |
| pUC19ayt | 5101 | *ybgC* (*adc, thlA*) | lac |
| pUC19act | 5964 | *ctfA*/*B* (*adc, thlA*) | lac |

### Beispiel 5: Acetonbildung in E. coli

Alle erhaltenen Plasmid-Varianten (siehe Tabelle 1) wurden im E. coli-Klonierstamm HB101 auf Acetonbildung hin untersucht. Die Analysen erfolgten im 5-ml-Maßstab in LB-Medium mit Ampicillin (100 µg/ml) nach Inokulation aus entsprechenden Vorkulturen und einer Inkubation bei 37 °C und 200 Upm. Die optische Dichte (600 nm) wurde photometrisch verfolgt und bei einem Wert von etwa 0,5-0,6 die Expression der klonierten Gene durch Zugabe von 1 mM IPTG (Isopropyl-β-D-thiogalactopyranosid) induziert und nach 3-4 h Glukose (20 g/l) zugegeben. Bei den Varianten unter Kontrolle des Thiolase-Promotors erfolgte keine Induktion, da davon ausgegangen wurde, dass eine konstitutive Expression erfolgt. Zu bestimmten Zeitpunkten über einen Zeitraum von ca. 48 h wurden Proben entnommen und mittels Gaschromatographie die Konzentration von Aceton und Acetat im zellfreien Medienüberstand bestimmt. Wahlweise erfolgten zusätzliche Glukose-Gaben in unterschiedlicher Menge (im Bereich von 10-40 g/l) entweder 4 h nach Induktion oder auch gleichzeitig mit der Induktion.

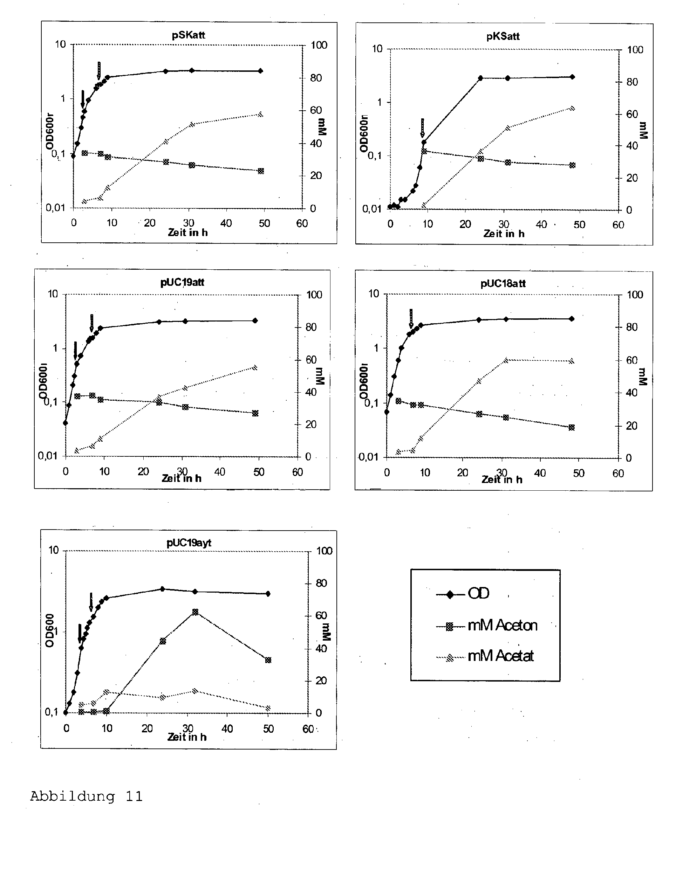

In *E. coli* HB101 ließen sich mit allen Plasmidvarianten signifikante Acetonproduktionen nachweisen. In Abbildung 11 sind beispielhaft für einzelne Varianten die Wachstumskurven mit den gebildeten Aceton- und Acetatmengen gezeigt.

Eine Zusammenfassung der Ergebnisse mit den maximalen Acetonkonzentrationen ist in Tabelle 2 gezeigt.

Abb. 11 stellt jeweils die optische Dichte (600 nm), sowie die Aceton- und Acetatkonzentrationen (mM) zu ausgewählten Zeitpunkten dar. Der schwarze Pfeil deutet jeweils den Zeitpunkt der Zugabe von IPTG (1 mM), der grüne Pfeil die Zugabe der Glukose (20 g/l)an.

**Tab. 2: Zusammenfassung der maximalen Acetonkonzentra-tionen der einzelnen Plasmidvarianten in 5-ml-Kulturen**

| Plasmid | Insert | Promotor | max. Acetonkonzentration, Stamm, Medium |
|---|---|---|---|
| pSKatt | *teII_{srf}* (*adc, thlA*) | lac | 34 mM (2,0 g/l, HB101, LB_{Amp}) |
| pKSatt | *teII_{srf}* (*adc, thlA*) | thl | 36 mM (2,1 g/l, HB101, LB_{Amp}) |
| pUC19att | *teII_{srf}* (*adc, thlA*) | lac | 30 mM (1,7 g/l, HB101, LB_{Amp}) |
| pUC18att | *teII_{srf}* (*adc,* thlA) | thl | 35 mM (2,0 g/l, HB101, LB_{Amp}) |
| pUC19ayt | *ybgC* (*adc, thlA*) | lac | 60 mM (3,5 g/l, HB101, LB_{Amp}) |
| pUC19act | *ctfA*/*B* (*adc, thlA*) | lac | 46 mM (2,7 g/l, HB101, LB_{Amp}) |

### Beispiel 6: Vergleich des Aceton-Pathways mit kloniertem ybgC-Gen aus H. influenzae mit dem Aceton-Pathway bestehend ausschließlich aus clostridiellen Genen

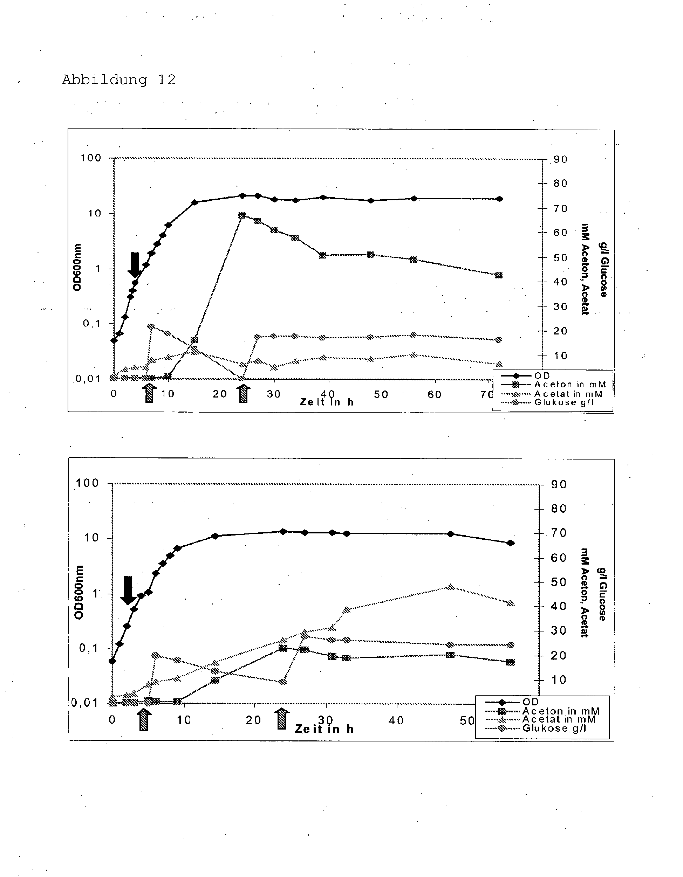

In einem anschließenden Experiment wurde nun versucht, die erhaltenen Ergebnisse auch in 100-ml-Kulturen (LB_{Amp}) mit den Varianten pUC19ayt und pUC19act als Kontrolle zu reproduzieren (37 °C, 200 upm). Die Hauptkulturen wurden mit 1 ml einer entsprechenden Vorkultur inokuliert und bei einer OD₆₀₀ₙₘ von 0,5 mit 1 mM IPTG induziert. Abweichend zu den bisherigen Versuchen wurde die Gabe von Glukose (20 g/l) 18 h nach der ersten Zugabe wiederholt. Zusätzlich erfolgte parallel zu den einzelnen Probenahmezeitpunkten die Bestimmung des Glukosegehaltes der Kultur, um den Verbrauch von Glukose ermitteln zu können. Die Bestimmung der Glukose wurde mittels eines optisch-enzymatischen Tests nach Bergmeyer (1970) vorgenommen, bei dem durch die Enzyme Hexokinase und Glukose-6-Phosphat-Dehydrogenase unter Zugabe von ATP und NADP⁺ in zwei Schritten eine Umsetzung der Glukose zu 6-Phospho-Glukonat und NADPH erfolgt (Bergmeyer, H. U. (Editor-in-Chief): Methods of Enzymatic Analysis (Methoden der enzymatischen Analyse), 2. Edition, Verlag Chemie, Weinheim - Deerfield Beach - Basel 1970). Die dabei gebildete Menge an NADPH ist proportional der vorhandenen Glukosemenge und kann bei einer Wellenlänge von 340 nm photometrisch über die Extinktionsänderung bestimmt werden. Die Ergebnisse dazu sind in den Abbildungen 12 A und 12 B dargestellt. Zu erkennen ist, dass die Glukose in Kultur A (*E*. *coli* HB101 pUC19ayt) zum Zeitpunkt der zweiten Zugabe bereits vollständig verbraucht war.

Allerdings führte die erneute Applikation von Glukose weder zu dem erhofften weiteren Anstieg der Acetonmenge noch wurde sie verbraucht, da offensichtlich keine Abnahme festzustellen war. Es ist davon auszugehen, dass zu diesem Zeitpunkt bereits andere Faktoren das Wachstum limitierten, denn es erfolgte auch keine weitere Zunahme der optischen Dichte. Es wurden maximal 67 mM (3,9 g/l) Aceton gebildet. Ähnlich stellte sich die Kultur B mit dem Kontrollplasmid pUC19act dar, die unter gleichen Bedingungen durchgeführt wurde. Die Glukose war zum Zeitpunkt der zweiten Zugabe noch nicht vollständig verbraucht, die erneute Gabe von Glukose blieb aber auch hier im Zusammenhang mit einem Anstieg der gebildeten Acetonmenge ohne Wirkung. Die maximale Acetonmenge lag bei 20 mM (1,2 g/l). Damit produzierte die Variante mit dem klonierten *ybgC-*Gen aus *H. influenzae* unter diesen Bedingungen dreimal soviel Aceton wie die Kontrollkultur mit den ausschließlich clostridiellen Genen.

Abb. 12 stellt jeweils die optische Dichte (600 nm), die Aceton- und Acetatkonzentration (mM) und der Glucosegehalt (g/l) zu ausgewählten Zeitpunkten dar. Der schwarze Pfeil deutet den Zeitpunkt der Zugabe von IPTG (1 mM), die grünen Pfeile die Zugabe von Glukose(20 g/l) an; oben: *E. coli* HB101 pUC19ayt, unten: *E. coli* HB101 pUC19act (Kontrolle)

## Patentansprüche

1. Verfahren zur Herstellung von Aceton ausgehend von Acetyl-CoenzymA umfassend die Verfahrenschritte:
A. enzymatische Umsetzung von Acetyl-CoA zu Acetoacetyl-CoA katalysiert durch eine β-Ketothiolase
B. enzymatische Umsetzung von Acetoacetyl-CoA zu Acetoacetat und CoA katalysiert durch eine Acetoacetat-CoA-Hydrolase, eine Acyl-CoA-Thioesterase, eine Acyl-CoA-Synthetase oder eine Acyl-CoA-Thiokinase
C. Decarboxylieren von Acetoacetat zu Aceton und CO₂ katalysiert durch eine Acetoacetatdecarboxylase
**dadurch gekennzeichnet,**
**dass** in Verfahrensschritt B das CoenzymA nicht auf ein Akzeptor-Molekül übertragen wird, und
die enzymatische Umsetzung in Verfahrensschritt B durch ein Polypeptid mit Acetoacetat-CoA-Hydrolase-Aktivität und mit einer Aminosäuresequenz, die zu mindestens 90% identisch mit der Aminosäuresequenz SEQ ID Nr. 1, 3 oder 5 ist, katalysiert wird.

2. Verfahren gemäß Anspruch 1,
**dadurch gekennzeichnet,**
**dass** die enzymatische Umsetzung in Verfahrensschritt A durch ein Polypeptid mit einer Ketothiolase-Aktivität und mit einer Aminosäuresequenz, die zu mindestens 90% identisch mit der Aminosäuresequenz SEQ ID Nr. 16 ist, katalysiert wird.

3. Verfahren gemäß mindestens einem der Ansprüche 1 oder 2,
**dadurch gekennzeichnet,**
**dass** die Umsetzung in Verfahrensschritt C durch ein Polypeptid mit einer Acetoacetatdecarboxylase-Aktivität und mit einer Aminosäuresequenz, die zu mindestens 90% identisch mit der Aminosäuresequenz SEQ ID Nr. 18 ist, katalysiert wird.

4. Verfahren gemäß mindestens einem der Ansprüche 1 bis 3,
**dadurch gekennzeichnet,**
**dass** die Umsetzung in Verfahrensschritt C spontan erfolgt.

5. Verfahren gemäß mindestens einem der Ansprüche 1 bis 4,
**dadurch gekennzeichnet,**
**dass** es durch einen Mikroorganismus durchgeführt wird.

6. Verfahren gemäß Anspruch 5,
**dadurch gekennzeichnet,**
**dass** der Mikroorganismus mindestens enthält:
a Nukleinsäure proteinkodierend für mindestens ein Polypeptid gemäß Anspruch 2 und
b Nukleinsäure proteinkodierend für das Polypeptid mit Acetoacetat-CoA-Hydrolase-Aktivität und mit einer Aminosäuresequenz, die zu mindestens 90% identisch mit der Aminosäuresequenz SEQ ID Nr. 1, 3 oder 5 ist und
c Nukleinsäure proteinkodierend für mindestens ein Polypeptid gemäß Anspruch 3
wobei die Nukleinsäuren a, b und c dafür verwendet werden, die Expression des Polypeptides im Mikroorganismus sicherzustellen.

7. Verfahren gemäß Anspruch 6,
**dadurch gekennzeichnet,**
**dass** die Nukleinsäure a ausgewählt wird aus:
aa DNA-Sequenz beschrieben durch Sequenz 17 oder ihren Komplementärstrang,
bb DNA-Sequenzen, die unter stringenten Bedingungen zu den unter aa beschriebenen DNA-Sequenzen hybridisieren,
cc DNA-Sequenzen, die ohne die Entartung des genetischen Codes zu den in aa und bb definierten DNA-Sequenzen hybridisieren würden.

8. Verfahren gemäß mindestens einem der Ansprüche 6 oder 7,
**dadurch gekennzeichnet,**
**dass** die Nukleinsäure c ausgewählt wird aus:
dd DNA-Sequenz beschrieben durch Sequenz 19 oder ihren Komplementärstrang
ee DNA-Sequenzen, die unter stringenten Bedingungen zu den unter dd beschriebenen DNA-Sequenzen hybridisieren
ff DNA-Sequenzen, die ohne die Entartung des genetischen Codes zu den unter dd und ee beschriebenen DNA-Sequenzen hybridisieren würden.

9. Verfahren gemäß mindestens einem der Ansprüche 6 bis 8,
**dadurch gekennzeichnet,**
**dass** die Nukleinsäure b ausgewählt wird aus:
gg DNA-Sequenz beschrieben durch Sequenz 2 oder ihren Komplementärstrang
hh DNA-Sequenzen, die unter stringenten Bedingungen zu den unter gg beschriebenen DNA-Sequenzen hybridisieren
ii DNA-Sequenzen, die ohne die Entartung des genetischen Codes zu den unter gg und hh beschriebenen DNA-Sequenzen hybridisieren würden.

10. Verfahren gemäß mindestens einem der Ansprüche 6 bis 8,
**dadurch gekennzeichnet,**
**dass** die Nukleinsäure b ausgewählt wird aus:
jj DNA-Sequenz beschrieben durch Sequenz 4 oder ihren Komplementärstrang
kk DNA-Sequenzen, die unter stringenten Bedingungen zu den unter jj beschriebenen DNA-Sequenzen hybridisieren
ll DNA-Sequenzen, die ohne die Entartung des genetischen Codes zu den unter jj und kk beschriebenen DNA-Sequenzen hybridisieren würden.

11. Verfahren gemäß mindestens einem der Ansprüche 6 bis 8,
**dadurch gekennzeichnet,**
**dass** die Nukleinsäure b ausgewählt wird aus:
mm DNA-Sequenz beschrieben durch Sequenz 6 oder ihren Komplementärstrang
nn DNA-Sequenzen, die unter stringenten Bedingungen zu den unter mm beschriebenen DNA-Sequenzen hybridisieren
oo DNA-Sequenzen, die ohne die Entartung des genetischen Codes zu den unter mm und nn beschriebenen DNA-Sequenzen hybridisieren würden.

12. Verfahren gemäß mindestens einem der Ansprüchen 6 bis 11, wobei die Nukleinsäuren a, b und c auf einem Nukleotidstrang liegen und dieser ausgewählt ist aus der Gruppe umfassend:
Plasmid pSKatt, mit der Nukleinsäuresequenz gemäß SEQ ID Nr. 7,
Plasmid pKSatt, mit der Nukleinsäuresequenz gemäß SEQ ID Nr. 8,
Plasmid pUC19att, mit der Nukleinsäuresequenz gemäß SEQ ID Nr. 9,
Plasmid pUC18att, mit der Nukleinsäuresequenz gemäß SEQ ID Nr. 10,
und
Plasmid pUC19ayt, mit der Nukleinsäuresequenz gemäß SEQ ID Nr. 11.

13. Nukleinsäure ausgewählt aus der Gruppe umfassend:
Plasmid pSKatt, mit der Nukleinsäuresequenz gemäß SEQ ID Nr. 7,
Plasmid pKSatt, mit der Nukleinsäuresequenz gemäß SEQ ID Nr. 8,
Plasmid pUC19att, mit der Nukleinsäuresequenz gemäß SEQ ID Nr. 9,
Plasmid pUC18att, mit der Nukleinsäuresequenz gemäß SEQ ID Nr. 10
und
Plasmid pUC19ayt, mit der Nukleinsäuresequenz gemäß SEQ ID Nr. 11.

## Claims

1. Process for preparing acetone starting from acetyl-coenzyme A comprising the process steps:
A. enzymatic conversion of acetyl-CoA into acetoacetyl-CoA catalysed by a β-ketothiolase
B. enzymatic conversion of acetoacetyl-CoA into acetoacetate and CoA catalysed by an acetoacetate-CoA-hydrolase, an acyl-CoA-thioesterase, an acyl-CoA-synthetase or an acyl-CoA-thiokinase
C. decarboxylation of acetoacetate to acetone and CO₂ catalysed by an acetoacetate decarboxylase
**characterized in that**
the coenzyme A is not transferred in process step B to an acceptor molecule, and
the enzymatic conversion in process step B is catalysed by a polypeptide having acetoacetate-CoA hydrolase activity and having an amino acid sequence which is at least 90% identical to the amino acid sequence of SEQ ID No. 1, 3 or 5.

2. Process according to Claim 1,
**characterized in that**
the enzymatic conversion in process step A is catalysed by a polypeptide having a ketothiolase activity and having an amino acid sequence which is at least 90% identical to the amino acid sequence of SEQ ID No. 16.

3. Process according to at least one of Claims 1 or 2, **characterized in that**
the conversion in process step C is catalysed by a polypeptide having an acetoacetate decarboxylase activity and having an amino acid sequence which is at least 90% identical to the amino acid sequence of SEQ ID No. 18.

4. Process according to at least one of Claims 1 to 3, **characterized in that**
the conversion in process step C takes place spontaneously.

5. Process according to at least one of Claims 1 to 4, **characterized in that**
it is carried out by a microorganism.

6. Process according to Claim 5,
**characterized in that**
the microorganism contains at least:
a nucleic acid protein-coding for at least one polypeptide according to Claim 2 and
b nucleic acid protein-coding for the polypeptide having acetoacetate-CoA hydrolase activity and having an amino acid sequence which is at least 90% identical to the amino acid sequence of SEQ ID No. 1,3 or 5 and
c nucleic acid protein-coding for at least one polypeptide according to Claim 3
the nucleic acids a, b and c being used to ensure expression of the polypeptide in the microorganism.

7. Process according to Claim 6, **characterized in that** the nucleic acid a is selected from:
aa DNA sequence described by sequence 17 or its complementary strand,
bb DNA sequences which hybridize under stringent conditions to the DNA sequences described under aa,
cc DNA sequences which would, without the degeneracy of the genetic code, hybridize to the DNA sequences defined in aa and bb.

8. Process according to at least one of Claims 6 or 7,
**characterized in that**
the nucleic acid c is selected from:
dd DNA sequence described by sequence 19 or its complementary strand,
ee DNA sequences which hybridize under stringent conditions to the DNA sequences described under dd,
ff DNA sequences which, without the degeneracy of the genetic code, would hybridize to the DNA sequences described under dd and ee.

9. Process according to at least one of Claims 6 to 8,
**characterized in that**
the nucleic acid b is selected from:
gg DNA sequence described by sequence 2 or its complementary strand,
hh DNA sequences which hybridize under stringent conditions to the DNA sequences described under gg,
ii DNA sequences which, without the degeneracy of the genetic code, would hybridize to the DNA sequences described under gg and hh.

10. Process according to at least one of Claims 6 to 8, **characterized in that**
the nucleic acid b is selected from:
jj DNA sequence described by sequence 4 or its complementary strand,
kk DNA sequences which hybridize under stringent conditions to the DNA sequences described under jj,
ll DNA sequences which, without the degeneracy of the genetic code, would hybridize to the DNA sequences described under jj and kk.

11. Process according to at least one of Claims 6 to 8,
**characterized in that**
the nucleic acid b is selected from:
mm DNA sequence described by sequence 6 or its complementary strand,
nn DNA sequences which hybridize under stringent conditions to the DNA sequences described under mm,
oo DNA sequences which, without the degeneracy of the genetic code, would hybridize to the DNA sequences described under mm and nn.

12. Process according to at least one of Claims 6 to 11, where the nucleic acids a, b and c are located on one nucleotide strand, and the latter is selected from the group comprising:
plasmid pSKatt having the nucleic acid sequence shown in SEQ ID No. 7,
plasmid pKSatt having the nucleic acid sequence shown in SEQ ID No. 8,
plasmid pUC19att having the nucleic acid sequence shown in SEQ ID No. 9,
plasmid pUC18att having the nucleic acid
sequence shown in SEQ ID No. 10,
and
plasmid pUC19ayt having the nucleic acid sequence shown in SEQ ID No. 11.

13. Nucleic acid selected from the group comprising:
plasmid pSKatt having the nucleic acid sequence shown in SEQ ID No. 7,
plasmid pKSatt having the nucleic acid sequence shown in SEQ ID No. 8,
plasmid pUC19att having the nucleic acid sequence shown in SEQ ID No. 9,
plasmid pUC18att having the nucleic acid
sequence shown in SEQ ID No. 10
and
plasmid pUC19ayt having the nucleic acid sequence shown in SEQ ID No. 11.

## Revendications

1. Procédé pour la préparation d'acétone partant d'acétyl-coenzyme A comprenant les étapes de procédé :
A. transformation enzymatique d'acétyl-CoA en acétoacétyl-CoA, catalysée par une β-cétothiolase
B. transformation enzymatique d'acétoacétyl-CoA en acétoacétate et CoA, catalysée par une acétoacétate-CoA-hydrolase, une acyl-CoA-thioestérase, une acyl-CoA-synthétase ou une acyl-CoA-thiokinase
C. décarboxylation d'acétoacétate en acétone et CO₂, catalysée par une acétoacétate-décarboxylase
**caractérisé en ce que**, dans l'étape de procédé B, la coenzyme A n'est pas transférée sur une molécule d'accepteur et la transformation enzymatique dans l'étape de procédé B est catalysée par un polypeptide présentant une activité d'acétoacétate-CoA-hydrolase et une séquence d'acides aminés qui est identique à raison d'au moins 90% à la séquence d'acides aminés SEQ ID NO 1, 3 ou 5.

2. Procédé selon la revendication 1, **caractérisé en ce que** la transformation enzymatique dans l'étape de procédé A est catalysée par un polypeptide présentant une activité de cétothiolase et une séquence d'acides aminés qui est identique à raison d'au moins 90% à la séquence d'acides aminés SEQ ID NO 16.

3. Procédé selon au moins l'une quelconque des revendications 1 ou 2, **caractérisé en ce que** la transformation enzymatique dans l'étape de procédé C est catalysée par un polypeptide présentant une activité d'acétoacétate-décarboxylase et une séquence d'acides aminés qui est identique à raison d'au moins 90% à la séquence d'acides aminés SEQ ID NO 18.

4. Procédé selon au moins l'une quelconque des revendications 1 à 3, **caractérisé en ce que** la transformation dans l'étape de procédé C a lieu de manière spontanée.

5. Procédé selon au moins l'une quelconque des revendications 1 à 4, **caractérisé en ce qu'**il est réalisé par un microorganisme.

6. Procédé selon la revendication 5, **caractérisé en ce que** le microorganisme contient au moins :
a un acide nucléique codant pour au moins un polypeptide selon la revendication 2 et
b un acide nucléique codant pour le polypeptide présentant une activité d'acétoacétate-CoA-hydrolase et une séquence d'acides aminés qui est identique à raison d'au moins 90% à la séquence d'acides aminés SEQ ID NO 1, 3 ou 5 et
c un acide nucléique codant pour au moins un polypeptide selon la revendication 3
les acides nucléiques a, b et c étant utilisés pour assurer l'expression du polypeptide dans le microorganisme.

7. Procédé selon la revendication 6, **caractérisé en ce que** l'acide nucléique a est choisi parmi :
aa la séquence d'ADN décrite par la séquence 17 ou son brin complémentaire,
bb les séquences d'ADN qui hybrident dans des conditions stringentes aux séquences d'ADN décrites au point aa,
cc les séquences d'ADN qui hybrideraient sans dégénérescence du code génétique aux séquences d'ADN définies aux points aa et bb.

8. Procédé selon au moins l'une quelconque des revendications 6 ou 7, **caractérisé en ce que** l'acide nucléique c est choisi parmi :
dd la séquence d'ADN décrite par la séquence 19 ou son brin complémentaire,
ee les séquences d'ADN qui hybrident dans des conditions stringentes aux séquences d'ADN décrites au point dd,
ff les séquences d'ADN qui hybrideraient sans dégénérescence du code génétique aux séquences d'ADN décrites aux points dd et ee.

9. Procédé selon au moins l'une quelconque des revendications 6 à 8, **caractérisé en ce que** l'acide nucléique b est choisi parmi :
gg la séquence d'ADN décrite par la séquence 2 ou son brin complémentaire,
hh les séquences d'ADN qui hybrident dans des conditions stringentes aux séquences d'ADN décrites au point gg,
ii les séquences d'ADN qui hybrideraient sans dégénérescence du code génétique aux séquences d'ADN décrites aux points gg et hh.

10. Procédé selon au moins l'une quelconque des revendications 6 à 8, **caractérisé en ce que** l'acide nucléique b est choisi parmi :
jj la séquence d'ADN décrite par la séquence 4 ou son brin complémentaire,
kk les séquences d'ADN qui hybrident dans des conditions stringentes aux séquences d'ADN décrites au point jj,
ll les séquences d'ADN qui hybrideraient sans dégénérescence du code génétique aux séquences d'ADN décrites aux points jj et kk.

11. Procédé selon au moins l'une quelconque des revendications 6 à 8, **caractérisé en ce que** l'acide nucléique b est choisi parmi :
mm la séquence d'ADN décrite par la séquence 6 ou son brin complémentaire,
nn les séquences d'ADN qui hybrident dans des conditions stringentes aux séquences d'ADN décrites au point mm,
oo les séquences d'ADN qui hybrideraient sans dégénérescence du code génétique aux séquences d'ADN décrites aux points mm et nn.

12. Procédé selon au moins l'une quelconque des revendications 6 à 11, les acides nucléiques a, b et c étant situés sur un brin nucléotidique et celui-ci étant choisi dans le groupe comprenant :
le plasmide pSKatt, présentant la séquence d'acide nucléique selon la SEQ ID NO 7,
le plasmide pKSatt, présentant la séquence d'acide nucléique selon la SEQ ID NO 8,
le plasmide pUC19att, présentant la séquence d'acide nucléique selon la SEQ ID NO 9,
le plasmide pUC18att, présentant la séquence d'acide nucléique selon la SEQ ID NO 10, et
le plasmide pUC19ayt, présentant la séquence d'acide nucléique selon la SEQ ID NO 11.

13. Acide nucléique, choisi dans le groupe comprenant :
le plasmide pSKatt, présentant la séquence d'acide nucléique selon la SEQ ID NO 7,
le plasmide pKSatt, présentant la séquence d'acide nucléique selon la SEQ ID NO 8,
le plasmide pUC19att, présentant la séquence d'acide nucléique selon la SEQ ID NO 9,
le plasmide pUC18att, présentant la séquence d'acide nucléique selon la SEQ ID NO 10, et
le plasmide pUC19ayt, présentant la séquence d'acide nucléique selon la SEQ ID NO 11.


### SEQUENCE LISTING

<110> Evonik Degussa GmbH
<120> Fermentative Gewinnung von Aceton aus erneuerbaren Rohstoffen mittels neuen Stoffwechselweges
<130> 200700792
<160> 21
<170> PatentIn version 3.4
<210> 1
   <211> 246
   <212> PRT
   <213> Bacillus subtilis
<400> 1
   Met Gly Gln Leu Phe Lys Ser Phe Asp Ala Ser Glu Lys Thr Gln Leu
   1               5                   10                  15
   Ile Cys Phe Pro Phe Ala Gly Gly Tyr Ser Ala Ser Phe Arg Pro Leu
               20                  25                  30
   His Ala Phe Leu Gln Gly Glu Cys Glu Met Leu Ala Ala Glu Pro Pro
           35                  40                  45
   Gly His Gly Thr Asn Gln Thr Ser Ala Ile Glu Asp Leu Glu Glu Leu
       50                  55                  60
   Thr Asp Leu Tyr Lys Gln Glu Leu Asn Leu Arg Pro Asp Arg Pro Phe
   65                  70                  75                  80
   Val Leu Phe Gly His Ser Met Gly Gly Met Ile Thr Phe Arg Leu Ala
                   85                  90                  95
   Gln Lys Leu Glu Arg Glu Gly Ile Phe Pro Gln Ala Val Ile Ile Ser
               100                 105                 110
   Ala Ile Gln Pro Pro His Ile Gln Arg Lys Lys Val Ser His Leu Pro
           115                 120                 125
   Asp Asp Gln Phe Leu Asp His Ile Ile Gln Leu Gly Gly Met Pro Ala
       130                 135                 140
   Glu Leu Val Glu Asn Lys Glu Val Met Ser Phe Phe Leu Pro Ser Phe
   145                 150                 155                 160
   Arg Ser Asp Tyr Arg Ala Leu Glu Gln Phe Glu Leu Tyr Asp Leu Ala
                   165                 170                 175
   Gln Ile Gln Ser Pro Val His Val Phe Asn Gly Leu Asp Asp Lys Lys
               180                 185                 190
   Cys Ile Arg Asp Ala Glu Gly Trp Lys Lys Trp Ala Lys Asp Ile Thr
   195 200 205
   Phe His Gln Phe Asp Gly Gly His Met Phe Leu Leu Ser Gln Thr Glu
   210 215 220
   Glu Val Ala Glu Arg Ile Phe Ala Ile Leu Asn Gln His Pro Ile Ile
   225 230 235 240
   Gln Pro Gly Ser Arg Ser
   245
<210> 2
   <211> 741
   <212> DNA
   <213> Clostridium acetobutylicum
<400> 2
   atgggccaac tcttcaaatc atttgatgcg tcggaaaaaa cacagctcat ctgttttccg     60
   tttgccggcg gctattcggc gtcgtttcgc cctctccatg cttttttgca gggggagtgc     120
   gagatgctcg ctgccgagcc gccgggacac ggcacgaatc aaacgtcagc cattgaggat    180
   ctcgaagagc tgacggattt gtacaagcaa gaactgaacc ttcgccctga tcggccgttt    240
   gtgctgttcg gacacagtat gggcggaatg atcaccttca ggctggcgca aaagcttgag    300
   cgtgaaggca tctttccgca ggcggttatc atttctgcaa tccagccgcc tcatattcag    360
   cggaagaaag tgtcccacct gcctgatgat cagtttctcg atcatattat ccaattaggc    420
   ggaatgcccg cagagcttgt tgaaaataag gaggtcatgt cctttttcct gccttctttc     480
   cgatcagatt accgggctct tgaacaattt gagctttacg atctggccca gatccagtcg    540
   cctgttcatg tctttaacgg gcttgatgat aaaaaatgca tacgagatgc ggaagggtgg    600
   aagaagtggg caaaagacat cacattccat caatttgacg gcgggcacat gttcctgctg    660
   tcacaaacgg aagaagtcgc agaacggatt tttgcgatct tgaatcagca tccgatcatt    720
   caaccgggat ccagatctta a                                               741
<210> 3
   <211> 650
   <212> PRT
   <213> Sinorhizobium meliloti
<400> 3
   Met Gln Ala Glu Arg Pro Leu Trp Val Pro Asp Arg Glu Ile Val Glu
   1               5                   10                  15
   Arg Ser Pro Met Ala Glu Phe Ile Asp Trp Cys Gly Glu Arg Phe Gly
               20                  25                  30
   Arg Ser Phe Ala Asp Tyr Asp Ala Phe His Asp Trp Ser Val Ser Glu
           35                  40                  45
   Arg Gly Ala Phe Trp Thr Ala Val Trp Glu His Cys Lys Val Ile Gly
       50                  55                  60
   Glu Ser Gly Glu Lys Ala Leu Val Asp Gly Asp Arg Met Leu Asp Ala
   65 70 75 80
   Arg Phe Phe Pro Glu Ala Arg Leu Asn Phe Ala Glu Asn Leu Leu Arg
   85 90 95
   Lys Thr Gly Ser Gly Asp Ala Leu Ile Phe Arg Gly Glu Asp Lys Val
   100 105 110
   Ser Tyr Arg Leu Thr Trp Asp Glu Leu Arg Ala Leu Val Ser Arg Leu
   115 120 125
   Gln Gln Ala Leu Arg Ala Gln Gly Ile Gly Ala Gly Asp Arg Val Ala
   130 135 140
   Ala Met Met Pro Asn Met Pro Glu Thr Ile Ala Leu Met Leu Ala Thr
   145 150 155 160
   Ala Ser Val Gly Ala Ile Trp Ser Ser Cys Ser Pro Asp Phe Gly Glu
   165 170 175
   Gln Gly Val Leu Asp Arg Phe Gly Gln Ile Ala Pro Lys Leu Phe Ile
   180 185 190
   Val Cys Asp Gly Tyr Trp Tyr Asn Gly Lys Arg Gln Asp Val Asp Ser
   195 200 205
   Lys Val Arg Ala Val Ala Lys Ser Leu Gly Ala Pro Thr Val Ile Val
   210 215 220
   Pro Tyr Ala Gly Asp Ser Ala Ala Leu Ala Pro Thr Val Glu Gly Gly
   225 230 235 240
   Val Thr Leu Ala Asp Phe Ile Ala Gly Phe Gln Ala Gly Pro Leu Val
   245 250 255
   Phe Glu Arg Leu Pro Phe Gly His Pro Leu Tyr Ile Leu Phe Ser Ser
   260 265 270
   Gly Thr Thr Gly Val Pro Lys Cys Ile Val His Ser Ala Gly Gly Thr
   275 280 285
   Leu Leu Gln His Leu Lys Glu His Arg Phe His Cys Gly Leu Arg Asp
   290 295 300
   Gly Glu Arg Leu Phe Tyr Phe Thr Thr Cys Gly Trp Met Met Trp Asn
   305 310 315 320
   Trp Leu Ala Ser Gly Leu Ala Val Gly Ala Thr Leu Cys Leu Tyr Asp
   325 330 335
   Gly Ser Pro Phe Cys Pro Asp Gly Asn Val Leu Phe Asp Tyr Ala Ala
               340                 345                 350
   Ala Glu Arg Phe Ala Val Phe Gly Thr Ser Ala Lys Tyr Ile Asp Ala
           355                 360                 365
   Val Arg Lys Gly Gly Phe Thr Pro Ala Arg Thr His Asp Leu Ser Ser
       370                 375                 380
   Leu Arg Leu Met Thr Ser Thr Gly Ser Pro Leu Ser Pro Glu Gly Phe
   385                 390                 395                 400
   Ser Phe Val Tyr Glu Gly Ile Lys Pro Asp Val Gln Leu Ala Ser Ile
                   405                 410                 415
   Ser Gly Gly Thr Asp Ile Val Ser Cys Phe Val Leu Gly Asn Pro Leu
               420                 425                 430
   Lys Pro Val Trp Arg Gly Glu Ile Gln Gly Pro Gly Leu Gly Leu Ala
           435                 440                 445
   Val Asp Val Trp Asn Asp Glu Gly Lys Pro Val Arg Gly Glu Lys Gly
       450                 455                 460
   Glu Leu Val Cys Thr Arg Ala Phe Pro Ser Met Pro Val Met Phe Trp
   465                 470                 475                 480
   Asn Asp Pro Asp Gly Ala Lys Tyr Arg Ala Ala Tyr Phe Asp Arg Phe
                   485                 490                 495
   Asp Asn Val Trp Cys His Gly Asp Phe Ala Glu Trp Thr Pro His Gly
               500                 505                 510
   Gly Ile Val Ile His Gly Arg Ser Asp Ala Thr Leu Asn Pro Gly Gly
           515                 520                 525
   Val Arg Ile Gly Thr Ala Glu Ile Tyr Asn Gln Val Glu Gln Met Asp
       530                 535                 540
   Glu Val Ala Glu Ala Leu Cys Ile Gly Gln Asp Trp Glu Asp Asp Val
   545                 550                 555                 560
   Arg Val Val Leu Phe Val Arg Leu Ala Arg Gly Val Glu Leu Thr Glu
                   565                 570                 575
   Ala Leu Thr Arg Glu Ile Lys Asn Arg Ile Arg Ser Gly Ala Ser Pro
               580                 585                 590
   Arg His Val Pro Ala Lys Ile Ile Ala Val Ala Asp Ile Pro Arg Thr
           595                 600                 605
   Lys Ser Gly Lys Ile Val Glu Leu Ala Val Arg Asp Val Val His Gly
       610                 615                 620
   Arg Pro Val Lys Asn Lys Glu Ala Leu Ala Asn Pro Glu Ala Leu Asp
   625                 630                 635                 640
   Leu Phe Ala Gly Leu Glu Glu Leu Lys Ser
                   645                 650
<210> 4
   <211> 1953
   <212> DNA
   <213> Sinorhizobium meliloti
<400> 4
   atgcaagcag aacgaccttt gtgggttccg gacagggaga tagtcgaacg cagcccgatg      60
   gctgagttca ttgactggtg cggggagcgc ttcgggcgca gcttcgccga ctatgatgcc     120
   tttcatgact ggtcggtgag cgagcgcggc gcgttctgga ccgccgtatg ggaacattgc     180
   aaggtcatcg gcgaaagcgg ggagaaggcg ctcgtcgacg gcgaccggat gctcgatgcc     240
   cgtttctttc cggaagcgag gctcaacttc gccgaaaacc tgctgcgcaa gacggggagc     300
   ggcgatgcct tgatcttccg cggcgaggac aaggtgagct accggctgac ctgggacgaa     360
   ctgcgcgccc tggtgtcgcg tctgcaacag gcgctgaggg cgcaggggat cggcgccggc     420
   gaccgcgtct ccgcgatgat gccgaacatg cccgagacga tcgcactcat gcttgcgacc     480
   gcttccgtcg gcgccatctg gtcgtcctgt tcgcccgatt tcggcgagca gggcgtcctc     540
   gaccgcttcg gccagatcgc ccccaagctc ttcatcgttt gcgacggcta ctggtacaac     600
   ggcaagcggc aggacgtgga ctcgaaggtg cgtgcggtgg ccaagtcgct cggcgcgccc     660
   accgtcatcg ttccctatgc cggagacagc gccgcgcttg cgccgaccgt cgagggcggg     720
   gtgacgcttg ccgatttcat cgccggattc caggccggac cgcttgtctt cgagcgcctg     780
   ccgttcggcc atccgctcta catactgttc tcctcgggca cgaccggcgt acccaaatgc     840
   atcgtccatt cggccggcgg aacgctgctg cagcacctca aggaacatcg cttccattgc     900
   gggctgaggg acggcgagcg gctgttctat ttcaccacct gcggctggat gatgtggaac     960
   tggctggcct cgggcctcgc cgtgggcgca actctgtgcc tctatgactg ctcacctttt    1020
   tgccccgacg gcaacgtgct cttcgattat gccgccgccg aacgcttcgc cttgttcggc    1080
   acctcggcga aatatatcga cgccgtgcgc aagggcgggt tcaccccggc aaggacgcac    1140
   gatctgtcgt ccttgcgggt catgacgtcc accggctcgc cgctttcgcc ggagggcttc    1200
   tccttcgtct atgagggcat caagcccgat gtccagctcg cctcgatttc cggcggcacc    1260
   gacatcgtct cctgcttcgt gctcggcaat cccctgaaac ccgtgtggcg cggagagatt    1320
   cagggccccg gcctcggctc cgccgtcgat gtctggaacg acgaaggcaa gccggtgcgc    1380
   ggggaaaagg gcgaactcgt ctgcaccagg gcgtttccgt cgatgcctgt catgttctgg    1440
   aacgatccgg acggcgcgaa gtatcgagcc gcctatttcg accgcttcga caatgtctgg    1500
   tgccacggcg attttgccga atggacaccg catggcggca tcgtcatcca cggccgttcc    1560
   gacgcgacat tgaaccccgg cggcgtgcgc atcggcacgg cggagatcta caatcaggtc    1620
   gaacagatgg atgaagtcgc cgaagcactg tgcatcggcc aggactggga ggacgatgtc    1680
   cgcgtcgtcc tgttcgtgcg gctggcccgc ggggtcgaac tgaccgaagc actgaccagg    1740
   gagatcaaga accggatccg gtccggcgcc tcgccgcggc acgtgccggc gaagatcatc    1800
   gccgtcgccg acatcccgcg caccaagtcc ggcaagatcg tcgaactggc ggtgcgcgac    1860
   gtggtgcacg gccgtccggt caagaacaag gaagcgctgg cgaacccgga agctctcgac    1920
   ctctttgcgg ggctcgagga actcaagagc tga                                 1953
<210> 5
   <211> 136
   <212> PRT
   <213> Haemophilus influenzae
<400> 5
   Met Leu Asp Asn Gly Phe Ser Phe Pro Val Arg Val Tyr Tyr Glu Asp
   1               5                   10                  15
   Thr Asp Ala Gly Gly Val Val Tyr His Ala Arg Tyr Leu His Phe Phe
               20                  25                  30
   Glu Arg Ala Arg Thr Glu Tyr Leu Arg Thr Leu Asn Phe Thr Gln Gln
           35                  40                  45
   Thr Leu Leu Glu Glu Gln Gln Leu Ala Phe Val Val Lys Thr Leu Ala
       50                  55                  60
   Ile Asp Tyr Cys Val Ala Ala Lys Leu Asp Asp Leu Leu Met Val Glu
   65                  70                  75                  80
   Thr Glu Val Ser Glu Val Lys Gly Ala Thr Ile Leu Phe Glu Gln Arg
                   85                  90                  95
   Leu Met Arg Asn Thr Leu Met Leu Ser Lys Ala Thr Val Lys Val Ala
               100                 105                 110
   Cys Val Asp Leu Gly Lys Met Lys Pro Val Ala Phe Pro Lys Glu Val
           115                 120                 125
   Lys Ala Ala Phe His His Leu Lys
       130                 135
<210> 6
   <211> 411
   <212> DNA
   <213> Haemophilus influenzae
<400> 6
   atgttggata atggcttttc ttttcctgtt cgtgtgtatt atgaagatac tgatgcaggt     60
   ggcgtagtgt atcacgctcg ctatttgcat ttttttgaac gagcaagaac agaatatttg    120
   cgtacattaa attttacgca acaaacctta ctagaggaac aacaactcgc atttgttgtc    180
   aaaacgctcg ccattgatta ttgcgtggca gcaaaattgg atgatttact tatggtggaa    240
   acagaggttt cagaagtaaa aggggctaca atcctttttg aacagagact gatgcgcaac    300
   accctgatgt tatcaaaggc tactgttaag gtagcctgtg ttgatctagg caagatgaaa    360
   ccagtggcgt ttcccaaaga agttaaagcg gcgtttcatc acttaaaata a             411
<210> 7
   <211> 5765
   <212> DNA
   <213> Artificial
<220>
   <223> Plasmid
<400> 7
   ctgacgcgcc ctgtagcggc gcattaagcg cggcgggtgt ggtggttacg cgcagcgtga     60
   ccgctacact tgccagcgcc ctagcgcccg ctcctttcgc tttcttccct tcctttctcg    120
   ccacgttcgc cggctttccc cgtcaagctc taaatcgggg gctcccttta gggttccgat    180
   ttagtgcttt acggcacctc gaccccaaaa aacttgatta gggtgatggt tcacgtagtg    240
   ggccatcgcc ctgatagacg gtttttcgcc ctttgacgtt ggagtccacg ttctttaata    300
   gtggactctt gttccaaact ggaacaacac tcaaccctat ctcggtctat tcttttgatt    360
   tataagggat tttgccgatt tcggcctatt ggttaaaaaa tgagctgatt taacaaaaat    420
   ttaacgcgaa ttttaacaaa atattaacgc ttacaatttc cattcgccat tcaggctgcg    480
   caactgttgg gaagggcgat cggtgcgggc ctcttcgcta ttacgccagc tggcgaaagg    540
   gggatgtgct gcaaggcgat taagttgggt aacgccaggg ttttcccagt cacgacgttg    600
   taaaacgacg gccagtgagc gcgcgtaata cgactcacta tagggcgaat tgggtaccgg    660
   gccctgaatt ctattactta agataatcat atataacttc agctctaggc aatattatat    720
   ctgcaagaat gtgagagcta gaaacaatct cttttactgg caaatcatta agtggcgcca    780
   tagcgtgatc aaataactgc agtcgagttg gtcctgtcca agcttcatgt acggtaacat    840
   ctgtgatttt cgcatttata agctcacata ttctagggct tccatcataa ttgggtatta    900
   ttttcaacat ataattaggg cgacaaattt gatcctttgc ttcattagca tctaaggctt    960
   tatgtttgta ccccattgta gctgtcgcaa ctctaagttt tccatagtct aaagttccta   1020
   ctaaagtatc tgaatccaca aaaagctttg gatacccgag ctttttagga tatgcactta   1080
   attcccttcc tactgcaatt gcaggctcat tatctaaata catcatatga agataatctc   1140
   ccttaactcc attaaagctt acgggaatag cctgtccgct ttctgtataa caaccaagtc   1200
   cactcgtatc atgcattgcc ataatttcaa acctgactaa gggctcatca atttctaaag   1260
   gctctggcac aactttacga agtgcatcca tatctgtacg atatacaatg ttaaaatact   1320
   cacgattatg aaatttatag ggtcctctag gaaatgcagg cgaagttaat ggcgtgctaa   1380
   tttgtttaat tacttcatcc tttaacataa aaggtacctt ccaatctgga tacgtaacgc   1440
   gtctcgagtc caagctcagc taattaagct tagtgatggt gatggtgatg agatcccggt   1500
   tgaatgatcg gatgctgatt caagatcgca aaaatccgtt ctgcgacttc ttccgtttgt 1560
   gacagcagga acatgtgccc gccgtcaaat tgatggaatg tgatgtcttt tgcccacttc 1620
   ttccaccctt ccgcatctcg tatgcatttt ttatcatcaa gcccgttaaa gacatgaaca 1680
   ggcgactgga tctgggccag atcgtaaagc tcaaattgtt caagagcccg gtaatctgat 1740
   cggaaagaag gcaggaaaaa ggacatgacc tccttatttt caacaagctc tgcgggcatt 1800
   ccgcctaatt ggataatatg atcgagaaac tgatcatcag gcaggtggga cactttcttc 1860
   cgctgaatat gaggcggctg gattgcagaa atgataaccg cctgcggaaa gatgccttca 1920
   cgctcaagct tttgcgccag cctgaaggtg atcattccgc ccatactgtg tccgaacagc 1980
   acaaacggcc gatcagggcg aaggttcagt tcttgcttgt acaaatccgt cagctcttcg 2040
   agatcctcaa tggctgacgt ttgattcgtg ccgtgtcccg gcggctcggc agcgagcatc 2100
   tcgcactccc cctgcaaaaa agcatggaga gggcgaaacg acgccgaata gccgccggca 2160
   aacggaaaac agatgagctg tgttttttcc gacgcatcaa atgatttgaa gagttggccc 2220
   atggttaatt tctcctcttt aatgaattct gtgtgaaatt gttatccgtc gaggtcgacg 2280
   aattcagatc agagttattt ttaaggatcc tttctagcac ttttctagca atattgctgt 2340
   tccttgtccg ccacctatac ataaagttgc taagcctttt tttgcatctc ttttttgcat 2400
   tgcgtgtaca agagtaacga gtattcttgc acctgatgct ccaattggat gaccaagggc 2460
   aatagctcct ccatttacat ttactttatt catatcaaat tttaaatctt ttgctactgc 2520
   taaactttga gctgcaaaag cttcatttga ttctattaaa tctaattcat caactgtcca 2580
   acctgctttt tcaatagctg cttttgttgc atagaaaggt ccatatccca ttattgctgg 2640
   gtcaactcct gctgaaccat aagaaactat cttagcaagt ggttttactc caagctcttt 2700
   agctttttct gcactcatga ttacaagtac tgctgcacag tcatttaatc ctgatgcatt 2760
   accagctgta actgttccat cttttttgaa ggcaggtttt aattttgcaa gtccttctat 2820
   agttgatcca aatctagggt gctcatctgt atcaactaca gtttctccct ttctgccttt 2880
   aattactaca ggaactattt catctttaaa ttgacctgat tttatagctt cttcagcttt 2940
   tttttgtgat gcaagagcaa actcatcttg ttcttctctt gaaatgttcc atctctcagc 3000
   tatgttttct gctgttattc ccatgtggta atcattaaat gcatcccaca atccgtcagt 3060
   gatcatttca tcaacaaatt tagcgtttcc cattctatat ccccatctag cgttattcgc 3120
   taagtaagga gctctagaca tattttccat accacctgct attattacgt cagcatctcc 3180
   tgcttttata atttgtgctg ctaagctaac tgttctaagt cctgaaccac aaaccttatt 3240
   aatagtcata gctggaattt caactggtaa tcctgcttta aaagatgcct gtcttgctgg 3300
   attctgtcct aaacctgctt gaagaacatt tcctaaaatg acttcattaa catcctctgg 3360
   ttttattcct gcttttttaa ctgcttcctt tatagctgta gctcctaaat ctactgctgg 3420
   tacatcctta agagactttc cataagatcc aatcgctgtt cttactgcac tagctattac 3480
   aacttctttc attctaacta acctcctaaa ttttgacccg ggggatccac tagttctaga 3540
   gcggccgcca ccgcggtgga gctccagctt ttgttccctt tagtgagggt taattgcgcg 3600
   cttggcgtaa tcatggtcat agctgtttcc tgtgtgaaat tgttatccgc tcacaattcc 3660
   acacaacata cgagccggaa gcataaagtg taaagcctgg ggtgcctaat gagtgagcta 3720
   actcacatta attgcgttgc gctcactgcc cgctttccag tcgggaaacc tgtcgtgcca 3780
   gctgcattaa tgaatcggcc aacgcgcggg gagaggcggt ttgcgtattg ggcgctcttc 3840
   cgcttcctcg ctcactgact cgctgcgctc ggtcgttcgg ctgcggcgag cggtatcagc 3900
   tcactcaaag gcggtaatac ggttatccac agaatcaggg gataacgcag gaaagaacat 3960
   gtgagcaaaa ggccagcaaa aggccaggaa ccgtaaaaag gccgcgttgc tggcgttttt 4020
   ccataggctc cgcccccctg acgagcatca caaaaatcga cgctcaagtc agaggtggcg 4080
   aaacccgaca ggactataaa gataccaggc gtttccccct ggaagctccc tcgtgcgctc 4140
   tcctgttccg accctgccgc ttaccggata cctgtccgcc tttctccctt cgggaagcgt 4200
   ggcgctttct catagctcac gctgtaggta tctcagttcg gtgtaggtcg ttcgctccaa 4260
   gctgggctgt gtgcacgaac cccccgttca gcccgaccgc tgcgccttat ccggtaacta 4320
   tcgtcttgag tccaacccgg taagacacga cttatcgcca ctggcagcag ccactggtaa 4380
   caggattagc agagcgaggt atgtaggcgg tgctacagag ttcttgaagt ggtggcctaa 4440
   ctacggctac actagaagga cagtatttgg tatctgcgct ctgctgaagc cagttacctt 4500
   cggaaaaaga gttggtagct cttgatccgg caaacaaacc accgctggta gcggtggttt 4560
   ttttgtttgc aagcagcaga ttacgcgcag aaaaaaagga tctcaagaag atcctttgat 4620
   cttttctacg gggtctgacg ctcagtggaa cgaaaactca cgttaaggga ttttggtcat 4680
   gagattatca aaaaggatct tcacctagat ccttttaaat taaaaatgaa gttttaaatc 4740
   aatctaaagt atatatgagt aaacttggtc tgacagttac caatgcttaa tcagtgaggc 4800
   acctatctca gcgatctgtc tatttcgttc atccatagtt gcctgactcc ccgtcgtgta 4860
   gataactacg atacgggagg gcttaccatc tggccccagt gctgcaatga taccgcgaga 4920
   cccacgctca ccggctccag atttatcagc aataaaccag ccagccggaa gggccgagcg 4980
   cagaagtggt cctgcaactt tatccgcctc catccagtct attaattgtt gccgggaagc 5040
   tagagtaagt agttcgccag ttaatagttt gcgcaacgtt gttgccattg ctacaggcat 5100
   cgtggtgtca cgctcgtcgt ttggtatggc ttcattcagc tccggttccc aacgatcaag 5160
   gcgagttaca tgatccccca tgttgtgcaa aaaagcggtt agctccttcg gtcctccgat 5220
   cgttgtcaga agtaagttgg ccgcagtgtt atcactcatg gttatggcag cactgcataa 5280
   ttctcttact gtcatgccat ccgtaagatg cttttctgtg actggtgagt actcaaccaa 5340
   gtcattctga gaatagtgta tgcggcgacc gagttgctct tgcccggcgt caatacggga 5400
   taataccgcg ccacatagca gaactttaaa agtgctcatc attggaaaac gttcttcggg 5460
   gcgaaaactc tcaaggatct taccgctgtt gagatccagt tcgatgtaac ccactcgtgc 5520
   acccaactga tcttcagcat cttttacttt caccagcgtt tctgggtgag caaaaacagg 5580
   aaggcaaaat gccgcaaaaa agggaataag ggcgacacgg aaatgttgaa tactcatact 5640
   cttccttttt caatattatt gaagcattta tcagggttat tgtctcatga gcggatacat 5700
   atttgaatgt atttagaaaa ataaacaaat aggggttccg cgcacatttc cccgaaaagt 5760
   gccac 5765
<210> 8
   <211> 5919
   <212> DNA
   <213> Artificial
<220>
   <223> Plasmid
<400> 8
   ctgacgcgcc ctgtagcggc gcattaagcg cggcgggtgt ggtggttacg cgcagcgtga     60
   ccgctacact tgccagcgcc ctagcgcccg ctcctttcgc tttcttccct tcctttctcg    120
   ccacgttcgc cggctttccc cgtcaagctc taaatcgggg gctcccttta gggttccgat    180
   ttagtgcttt acggcacctc gaccccaaaa aacttgatta gggtgatggt tcacgtagtg    240
   ggccatcgcc ctgatagacg gtttttcgcc ctttgacgtt ggagtccacg ttctttaata    300
   gtggactctt gttccaaact ggaacaacac tcaaccctat ctcggtctat tcttttgatt    360
   tataagggat tttgccgatt tcggcctatt ggttaaaaaa tgagctgatt taacaaaaat    420
   ttaacgcgaa ttttaacaaa atattaacgc ttacaatttc cattcgccat tcaggctgcg    480
   caactgttgg gaagggcgat cggtgcgggc ctcttcgcta ttacgccagc tggcgaaagg    540
   gggatgtgct gcaaggcgat taagttgggt aacgccaggg ttttcccagt cacgacgttg    600
   taaaacgacg gccagtgagc gcgcgtaata cgactcacta tagggcgaat tggagctcca    660
   ccgcggtggc ggccgctcta gaactagtgg atcccccggg ggttagcata tgcataagtt    720
   taattttttt gttaaaaaat attaaacttt gtgttttttt taacaaaata tattgataaa    780
   aataataata gtgggtataa ttaagttgtt agagaaaacg tataaattag ggataaacta    840
   tggaacttat gaaatagatt gaaatggttt atctgttacc ccgtatcaaa atttaggagg    900
   ttagttagaa tgaaagaagt tgtaatagct agtgcagtaa gaacagcgat tggatcttat    960
   ggaaagtctc ttaaggatgt accagcagta gatttaggag ctacagctat aaaggaagca   1020
   gttaaaaaag caggaataaa accagaggat gttaatgaag tcattttagg aaatgttctt   1080
   caagcaggtt taggacagaa tccagcaaga caggcatctt ttaaagcagg attaccagtt   1140
   gaaattccag ctatgactat taataaggtt tgtggttcag gacttagaac agttagctta   1200
   gcagcacaaa ttataaaagc aggagatgct gacgtaataa tagcaggtgg tatggaaaat   1260
   atgtctagag ctccttactt agcgaataac gctagatggg gatatagaat gggaaacgct   1320
   aaatttgttg atgaaatgat cactgacgga ttgtgggatg catttaatga ttaccacatg   1380
   ggaataacag cagaaaacat agctgagaga tggaacattt caagagaaga acaagatgag   1440
   tttgctcttg catcacaaaa aaaagctgaa gaagctataa aatcaggtca atttaaagat   1500
   gaaatagttc ctgtagtaat taaaggcaga aagggagaaa ctgtagttga tacagatgag   1560
   caccctagat ttggatcaac tatagaagga cttgcaaaat taaaacctgc cttcaaaaaa 1620
   gatggaacag ttacagctgg taatgcatca ggattaaatg actgtgcagc agtacttgta 1680
   atcatgagtg cagaaaaagc taaagagctt ggagtaaaac cacttgctaa gatagtttct 1740
   tatggttcag caggagttga cccagcaata atgggatatg gacctttcta tgcaacaaaa 1800
   gcagctattg aaaaagcagg ttggacagtt gatgaattag atttaataga atcaaatgaa 1860
   gcttttgcag ctcaaagttt agcagtagca aaagatttaa aatttgatat gaataaagta 1920
   aatgtaaatg gaggagctat tgcccttggt catccaattg gagcatcagg tgcaagaata 1980
   ctcgttactc ttgtacacgc aatgcaaaaa agagatgcaa aaaaaggctt agcaacttta 2040
   tgtataggtg gcggacaagg aacagcaata ttgctagaaa agtgctagaa agtcgacctc 2100
   gacggataac aatttcacac agaattcatt aaagaggaga aattaaccat gggccaactc 2160
   ttcaaatcat ttgatgcgtc ggaaaaaaca cagctcatct gttttccgtt tgccggcggc 2220
   tattcggcgt cgtttcgccc tctccatgct tttttgcagg gggagtgcga gatgctcgct 2280
   gccgagccgc cgggacacgg cacgaatcaa acgtcagcca ttgaggatct cgaagagctg 2340
   acggatttgt acaagcaaga actgaacctt cgccctgatc ggccgtttgt gctgttcgga 2400
   cacagtatgg gcggaatgat caccttcagg ctggcgcaaa agcttgagcg tgaaggcatc 2460
   tttccgcagg cggttatcat ttctgcaatc cagccgcctc atattcagcg gaagaaagtg 2520
   tcccacctgc ctgatgatca gtttctcgat catattatcc aattaggcgg aatgcccgca 2580
   gagcttgttg aaaataagga ggtcatgtcc tttttcctgc cttctttccg atcagattac 2640
   cgggctcttg aacaatttga gctttacgat ctggcccaga tccagtcgcc tgttcatgtc 2700
   tttaacgggc ttgatgataa aaaatgcata cgagatgcgg aagggtggaa gaagtgggca 2760
   aaagacatca cattccatca atttgacggc gggcacatgt tcctgctgtc acaaacggaa 2820
   gaagtcgcag aacggatttt tgcgatcttg aatcagcatc cgatcattca accgggatct 2880
   catcaccatc accatcacta agcttaatta gctgagcttg gactcgagac gcgttacgta 2940
   tccagattgg aaggtacctt ttatgttaaa ggatgaagta attaaacaaa ttagcacgcc 3000
   attaacttcg cctgcatttc ctagaggacc ctataaattt cataatcgtg agtattttaa 3060
   cattgtatat cgtacagata tggatgcact tcgtaaagtt gtgccagagc ctttagaaat 3120
   tgatgagccc ttagtcaggt ttgaaattat ggcaatgcat gatacgagtg gacttggttg 3180
   ttatacagaa agcggacagg ctattcccgt aagctttaat ggagttaagg gagattatct 3240
   tcatatgatg tatttagata atgagcctgc aattgcagta ggaagggaat taagtgcata 3300
   tcctaaaaag ctcgggtatc caaagctttt tgtggattca gatactttag taggaacttt 3360
   agactatgga aaacttagag ttgcgacagc tacaatgggg tacaaacata aagccttaga 3420
   tgctaatgaa gcaaaggatc aaatttgtcg ccctaattat atgttgaaaa taatacccaa 3480
   ttatgatgga agccctagaa tatgtgagct tataaatgcg aaaatcacag atgttaccgt 3540
   acatgaagct tggacaggac caactcgact gcagttattt gatcacgcta tggcgccact 3600
   taatgatttg ccagtaaaag agattgtttc tagctctcac attcttgcag atataatatt 3660
   gcctagagct gaagttatat atgattatct taagtaatag aattcagggc ccggtaccca 3720
   gcttttgttc cctttagtga gggttaattg cgcgcttggc gtaatcatgg tcatagctgt 3780
   ttcctgtgtg aaattgttat ccgctcacaa ttccacacaa catacgagcc ggaagcataa 3840
   agtgtaaagc ctggggtgcc taatgagtga gctaactcac attaattgcg ttgcgctcac 3900
   tgcccgcttt ccagtcggga aacctgtcgt gccagctgca ttaatgaatc ggccaacgcg 3960
   cggggagagg cggtttgcgt attgggcgct cttccgcttc ctcgctcact gactcgctgc 4020
   gctcggtcgt tcggctgcgg cgagcggtat cagctcactc aaaggcggta atacggttat 4080
   ccacagaatc aggggataac gcaggaaaga acatgtgagc aaaaggccag caaaaggcca 4140
   ggaaccgtaa aaaggccgcg ttgctggcgt ttttccatag gctccgcccc cctgacgagc 4200
   atcacaaaaa tcgacgctca agtcagaggt ggcgaaaccc gacaggacta taaagatacc 4260
   aggcgtttcc ccctggaagc tccctcgtgc gctctcctgt tccgaccctg ccgcttaccg 4320
   gatacctgtc cgcctttctc ccttcgggaa gcgtggcgct ttctcatagc tcacgctgta 4380
   ggtatctcag ttcggtgtag gtcgttcgct ccaagctggg ctgtgtgcac gaaccccccg 4440
   ttcagcccga ccgctgcgcc ttatccggta actatcgtct tgagtccaac ccggtaagac 4500
   acgacttatc gccactggca gcagccactg gtaacaggat tagcagagcg aggtatgtag 4560
   gcggtgctac agagttcttg aagtggtggc ctaactacgg ctacactaga aggacagtat 4620
   ttggtatctg cgctctgctg aagccagtta ccttcggaaa aagagttggt agctcttgat 4680
   ccggcaaaca aaccaccgct ggtagcggtg gtttttttgt ttgcaagcag cagattacgc 4740
   gcagaaaaaa aggatctcaa gaagatcctt tgatcttttc tacggggtct gacgctcagt 4800
   ggaacgaaaa ctcacgttaa gggattttgg tcatgagatt atcaaaaagg atcttcacct 4860
   agatcctttt aaattaaaaa tgaagtttta aatcaatcta aagtatatat gagtaaactt 4920
   ggtctgacag ttaccaatgc ttaatcagtg aggcacctat ctcagcgatc tgtctatttc 4980
   gttcatccat agttgcctga ctccccgtcg tgtagataac tacgatacgg gagggcttac 5040
   catctggccc cagtgctgca atgataccgc gagacccacg ctcaccggct ccagatttat 5100
   cagcaataaa ccagccagcc ggaagggccg agcgcagaag tggtcctgca actttatccg 5160
   cctccatcca gtctattaat tgttgccggg aagctagagt aagtagttcg ccagttaata 5220
   gtttgcgcaa cgttgttgcc attgctacag gcatcgtggt gtcacgctcg tcgtttggta 5280
   tggcttcatt cagctccggt tcccaacgat caaggcgagt tacatgatcc cccatgttgt 5340
   gcaaaaaagc ggttagctcc ttcggtcctc cgatcgttgt cagaagtaag ttggccgcag 5400
   tgttatcact catggttatg gcagcactgc ataattctct tactgtcatg ccatccgtaa 5460
   gatgcttttc tgtgactggt gagtactcaa ccaagtcatt ctgagaatag tgtatgcggc 5520
   gaccgagttg ctcttgcccg gcgtcaatac gggataatac cgcgccacat agcagaactt 5580
   taaaagtgct catcattgga aaacgttctt cggggcgaaa actctcaagg atcttaccgc 5640
   tgttgagatc cagttcgatg taacccactc gtgcacccaa ctgatcttca gcatctttta 5700
   ctttcaccag cgtttctggg tgagcaaaaa caggaaggca aaatgccgca aaaaagggaa 5760
   taagggcgac acggaaatgt tgaatactca tactcttcct ttttcaatat tattgaagca 5820
   tttatcaggg ttattgtctc atgagcggat acatatttga atgtatttag aaaaataaac 5880
   aaataggggt tccgcgcaca tttccccgaa aagtgccac 5919
<210> 9
   <211> 5409
   <212> DNA
   <213> Artificial
<220>
   <223> Plasmid
<400> 9
   tcgcgcgttt cggtgatgac ggtgaaaacc tctgacacat gcagctcccg gagacggtca 60
   cagcttgtct gtaagcggat gccgggagca gacaagcccg tcagggcgcg tcagcgggtg 120
   ttggcgggtg tcggggctgg cttaactatg cggcatcaga gcagattgta ctgagagtgc 180
   accatatgcg gtgtgaaata ccgcacagat gcgtaaggag aaaataccgc atcaggcgcc 240
   attcgccatt caggctgcgc aactgttggg aagggcgatc ggtgcgggcc tcttcgctat 300
   tacgccagct ggcgaaaggg ggatgtgctg caaggcgatt aagttgggta acgccagggt 360
   tttcccagtc acgacgttgt aaaacgacgg ccagtgaatt caggtaactc ttatttttat 420
   tacttaagat aatcatatat aacttcagct ctaggcaata ttatatctgc aagaatgtga 480
   gagctagaaa caatctcttt tactggcaaa tcattaagtg gcgccatagc gtgatcaaat 540
   aactgcagtc gagttggtcc tgtccaagct tcatgtacgg taacatctgt gattttcgca 600
   tttataagct cacatattct agggcttcca tcataattgg gtattatttt caacatataa 660
   ttagggcgac aaatttgatc ctttgcttca ttagcatcta aggctttatg tttgtacccc 720
   attgtagctg tcgcaactct aagttttcca tagtctaaag ttcctactaa agtatctgaa 780
   tccacaaaaa gctttggata cccgagcttt ttaggatatg cacttaattc ccttcctact 840
   gcaattgcag gctcattatc taaatacatc atatgaagat aatctccctt aactccatta 900
   aagcttacgg gaatagcctg tccgctttct gtataacaac caagtccact cgtatcatgc 960
   attgccataa tttcaaacct gactaagggc tcatcaattt ctaaaggctc tggcacaact 1020
   ttacgaagtg catccatatc tgtacgatat acaatgttaa aatactcacg attatgaaat 1080
   ttatagggtc ctctaggaaa tgcaggcgaa gttaatggcg tgctaatttg tttaattact 1140
   tcatccttta acataaaagg tacccggtta aatgatcgga tgctgattca agatcgcaaa 1200
   aatccgttct gcgacttctt ccgtttgtga cagcaggaac atgtgcccgc cgtcaaattg 1260
   atggaatgtg atgtcttttg cccacttctt ccacccttcc gcatctcgta tgcatttttt 1320
   atcatcaagc ccgttaaaga catgaacagg cgactggatc tgggccagat cgtaaagctc 1380
   aaattgttca agagcccggt aatctgatcg gaaagaaggc aggaaaaagg acatgacctc 1440
   cttattttca acaagctctg cgggcattcc gcctaattgg ataatatgat cgagaaactg 1500
   atcatcaggc aggtgggaca ctttcctccg ctgaatatga ggcggctgga ttgcagaaat 1560
   gataaccgcc tgcggaaaga tgccttcacg ctcaagcttt tgcgccagcc tgaaggtgat 1620
   cattccgccc atactgtgtc cgaacagcac aaacggccga tcagggcgaa ggttcagttc 1680
   ttgcttgtac aaatccgtca gctcttcgag atcctcaatg gctgacgttt gattcgtgcc 1740
   gtgtcccggc ggctcggcag cgagcatctc gcactccccc tgcaaaaaag catggagagg 1800
   gcgaaacgac gccgaatagc cgccggcaaa cggaaaacag atgagcggtg ttttttccga 1860
   cgcatcaaat gatttgaaga gttggcccat ggttaatttc tcctctttaa tgaattctgt 1920
   gtgaaattgt tatcggatcc tttctagcac ttttctagca atattgctgt tccttgtccg 1980
   ccacctatac ataaagttgc taagcctttt tttgcatctc ttttttgcat tgcgtgtaca 2040
   agagtaacga gtattcttgc acctgatgct ccaattggat gaccaagggc aatagctcct 2100
   ccatttacat ttactttatt catatcaaat tttaaatctt ttgctactgc taaactttga 2160
   gctgcaaaag cttcatttga ttctattaaa tctaattcat caactgtcca acctgctttt 2220
   tcaatagctg cttttgttgc atagaaaggt ccatatccca ttattgctgg gtcaactcct 2280
   gctgaaccat aagaaactat cttagcaagt ggttttactc caagctcttt agctttttct 2340
   gcactcatga ttacaagtac tgctgcacag tcatttaatc ctgatgcatt accagctgta 2400
   actgttccat cttttttgaa ggcaggtttt aattttgcaa gtccttctat agttgatcca 2460
   aatctagggt gctcatctgt atcaactaca gtttctccct ttctgccttt aattactaca 2520
   ggaactattt catctttaaa ttgacctgat tttatagctt cttcagcttt tttttgtgat 2580
   gcaagagcaa actcatcttg ttcttctctt gaaatgttcc atctctcagc tatgttttct 2640
   gctgttattc ccatgtggta atcattaaat gcatcccaca atccgtcagt gatcatttca 2700
   tcaacaaatt tagcgtttcc cattctatat ccccatctag cgttattcgc taagtaagga 2760
   gctctagaca tattttccat accacctgct attattacgt cagcatctcc tgcttttata 2820
   atttgtgctg ctaagctaac tgttctaagt cctgaaccac aaaccttatt aatagtcata 2880
   gctggaattt caactggtaa tcctgcttta aaagatgcct gtcttgctgg attctgtcct 2940
   aaacctgctt gaagaacatt tcctaaaatg acttcattaa catcctctgg ttttattcct 3000
   gcttttttaa ctgcttcctt tatagctgta gctcctaaat ctactgctgg tacatcctta 3060
   agagactttc cataagatcc aatcgctgtt cttactgcac tagctattac aacttctttc 3120
   attctaacta acctcctaaa ttttgatacg ggtcgacctg caggcatgca agcttggcgt 3180
   aatcatggtc atagctgttt cctgtgtgaa attgttatcc gctcacaatt ccacacaaca 3240
   tacgagccgg aagcataaag tgtaaagcct ggggtgccta atgagtgagc taactcacat 3300
   taattgcgtt gcgctcactg cccgctttcc agtcgggaaa cctgtcgtgc cagctgcatt 3360
   aatgaatcgg ccaacgcgcg gggagaggcg gtttgcgtat tgggcgctct tccgcttcct 3420
   cgctcactga ctcgctgcgc tcggtcgttc ggctgcggcg agcggtatca gctcactcaa 3480
   aggcggtaat acggttatcc acagaatcag gggataacgc aggaaagaac atgtgagcaa 3540
   aaggccagca aaaggccagg aaccgtaaaa aggccgcgtt gctggcgttt ttccataggc 3600
   tccgcccccc tgacgagcat cacaaaaatc gacgctcaag tcagaggtgg cgaaacccga    3660
   caggactata aagataccag gcgtttcccc ctggaagctc cctcgtgcgc tctcctgttc    3720
   cgaccctgcc gcttaccgga tacctgtccg cctttctccc ttcgggaagc gtggcgcttt    3780
   ctcatagctc acgctgtagg tatctcagtt cggtgtaggt cgttcgctcc aagctgggct    3840
   gtgtgcacga accccccgtt cagcccgacc gctgcgcctt atccggtaac tatcgtcttg    3900
   agtccaaccc ggtaagacac gacttatcgc cactggcagc agccactggt aacaggatta    3960
   gcagagcgag gtatgtaggc ggtgctacag agttcttgaa gtggtggcct aactacggct    4020
   acactagaag gacagtattt ggtatctgcg ctctgctgaa gccagttacc ttcggaaaaa    4080
   gagttggtag ctcttgatcc ggcaaacaaa ccaccgctgg tagcggtggt ttttttgttt    4140
   gcaagcagca gattacgcgc agaaaaaaag gatctcaaga agatcctttg atcttttcta    4200
   cggggtctga cgctcagtgg aacgaaaact cacgttaagg gattttggtc atgagattat    4260
   caaaaaggat cttcacctag atccttttaa attaaaaatg aagttttaaa tcaatctaaa    4320
   gtatatatga gtaaacttgg tctgacagtt accaatgctt aatcagtgag gcacctatct    4380
   cagcgatctg tctatttcgt tcatccatag ttgcctgact ccccgtcgtg tagataacta    4440
   cgatacggga gggcttacca tctggcccca gtgctgcaat gataccgcga gacccacgct    4500
   caccggctcc agatttatca gcaataaacc agccagccgg aagggccgag cgcagaagtg    4560
   gtcctgcaac tttatccgcc tccatccagt ctattaattg ttgccgggaa gctagagtaa    4620
   gtagttcgcc agttaatagt ttgcgcaacg ttgttgccat tgctacaggc atcgtggtgt    4680
   cacgctcgtc gtttggtatg gcttcattca gctccggttc ccaacgatca aggcgagtta    4740
   catgatcccc catgttgtgc aaaaaagcgg ttagctcctt cggtcctccg atcgttgtca    4800
   gaagtaagtt ggccgcagtg ttatcactca tggttatggc agcactgcat aattctctta    4860
   ctgtcatgcc atccgtaaga tgcttttctg tgactggtga gtactcaacc aagtcattct    4920
   gagaatagtg tatgcggcga ccgagttgct cttgcccggc gtcaatacgg gataataccg    4980
   cgccacatag cagaacttta aaagtgctca tcattggaaa acgttcttcg gggcgaaaac    5040
   tctcaaggat cttaccgctg ttgagatcca gttcgatgta acccactcgt gcacccaact    5100
   gatcttcagc atcttttact ttcaccagcg tttctgggtg agcaaaaaca ggaaggcaaa    5160
   atgccgcaaa aaagggaata agggcgacac ggaaatgttg aatactcata ctcttccttt    5220
   ttcaatatta ttgaagcatt tatcagggtt attgtctcat gagcggatac atatttgaat    5280
   gtatttagaa aaataaacaa ataggggttc cgcgcacatt tccccgaaaa gtgccacctg    5340
   acgtctaaga aaccattatt atcatgacat taacctataa aaataggcgt atcacgaggc    5400
   cctttcgtc                                                            5409
<210> 10
   <211> 5589
   <212> DNA
   <213> Artificial
<220>
   <223> Plasmid
<400> 10
   tcgcgcgttt cggtgatgac ggtgaaaacc tctgacacat gcagctcccg gagacggtca 60
   cagcttgtct gtaagcggat gccgggagca gacaagcccg tcagggcgcg tcagcgggtg 120
   ttggcgggtg tcggggctgg cttaactatg cggcatcaga gcagattgta ctgagagtgc 180
   accatatgcg gtgtgaaata ccgcacagat gcgtaaggag aaaataccgc atcaggcgcc 240
   attcgccatt caggctgcgc aactgttggg aagggcgatc ggtgcgggcc tcttcgctat 300
   tacgccagct ggcgaaaggg ggatgtgctg caaggcgatt aagttgggta acgccagggt 360
   tttcccagtc acgacgttgt aaaacgacgg ccagtgccaa gcttgcatgc ctgcaggtcg 420
   acggttagca tatgcataag tttaattttt ttgttaaaaa atattaaact ttgtgttttt 480
   tttaacaaaa tatattgata aaaataataa tagtgggtat aattaagttg ttagagaaaa 540
   cgtataaatt agggataaac tatggaactt atgaaataga ttgaaatggt ttatctgtta 600
   ccccgtatca aaatttagga ggttagttag aatgaaagaa gttgtaatag ctagtgcagt 660
   aagaacagcg attggatctt atggaaagtc tcttaaggat gtaccagcag tagatttagg 720
   agctacagct ataaaggaag cagttaaaaa agcaggaata aaaccagagg atgttaatga 780
   agtcatttta ggaaatgttc ttcaagcagg tttaggacag aatccagcaa gacaggcatc 840
   ttttaaagca ggattaccag ttgaaattcc agctatgact attaataagg tttgtggttc 900
   aggacttaga acagttagct tagcagcaca aattataaaa gcaggagatg ctgacgtaat 960
   aatagcaggt ggtatggaaa atatgtctag agctccttac ttagcgaata acgctagatg 1020
   gggatataga atgggaaacg ctaaatttgt tgatgaaatg atcactgacg gattgtggga 1080
   tgcatttaat gattaccaca tgggaataac agcagaaaac atagctgaga gatggaacat 1140
   ttcaagagaa gaacaagatg agtttgctct tgcatcacaa aaaaaagctg aagaagctat 1200
   aaaatcaggt caatttaaag atgaaatagt tcctgtagta attaaaggca gaaagggaga 1260
   aactgtagtt gatacagatg agcaccctag atttggatca actatagaag gacttgcaaa 1320
   attaaaacct gccttcaaaa aagatggaac agttacagct ggtaatgcat caggattaaa 1380
   tgactgtgca gcagtacttg taatcatgag tgcagaaaaa gctaaagagc ttggagtaaa 1440
   accacttgct aagatagttt cttatggttc agcaggagtt gacccagcaa taatgggata 1500
   tggacctttc tatgcaacaa aagcagctat tgaaaaagca ggttggacag ttgatgaatt 1560
   agatttaata gaatcaaatg aagcttttgc agctcaaagt ttagcagtag caaaagattt 1620
   aaaatttgat atgaataaag taaatgtaaa tggaggagct attgcccttg gtcatccaat 1680
   tggagcatca ggtgcaagaa tactcgttac tcttgtacac gcaatgcaaa aaagagatgc 1740
   aaaaaaaggc ttagcaactt tatgtatagg tggcggacaa ggaacagcaa tattgctaga 1800
   aaagtgctag aaaggatccg ataacaattt cacacagaat tcattaaaga ggagaaatta 1860
   accatgggcc aactcttcaa atcatttgat gcgtcggaaa aaacacagct catctgtttt 1920
   ccgtttgccg gcggctattc ggcgtcgttt cgccctctcc atgctttttt gcagggggag 1980
   tgcgagatgc tcgctgccga gccgccggga cacggcacga atcaaacgtc agccattgag    2040
   gatctcgaag agctgacgga tttgtacaag caagaactga accttcgccc tgatcggccg    2100
   tttgtgctgt tcggacacag tatgggcgga atgatcacct tcaggctggc gcaaaagctt    2160
   gagcgtgaag gcatctttcc gcaggcggtt atcatttctg caatccagcc gcctcatatt    2220
   cagcggaaga aagtgtccca cctgcctgat gatcagtttc tcgatcatat tatccaatta    2280
   ggcggaatgc ccgcagagct tgttgaaaat aaggaggtca tgtccttttt cctgccttct    2340
   ttccgatcag attaccgggc tcttgaacaa tttgagcttt acgatctggc ccagatccag    2400
   tcgcctgttc atgtctttaa cgggcttgat gataaaaaat gcatacgaga tgcggaaggg    2460
   tggaagaagt gggcaaaaga catcacattc catcaatttg acggcgggca catgttcctg    2520
   ctgtcacaaa cggaagaagt cgcagaacgg atttttgcga tcttgaatca gcatccgatc    2580
   atttaaccgg gtacctttta tgttaaagga tgaagtaatt aaacaaatta gcacgccatt    2640
   aacttcgcct gcatttccta gaggacccta taaatttcat aatcgtgagt attttaacat    2700
   tgtatatcgt acagatatgg atgcacttcg taaagttgtg ccagagcctt tagaaattga    2760
   tgagccctta gtcaggtttg aaattatggc aatgcatgat acgagtggac ttggttgtta    2820
   tacagaaagc ggacaggcta ttcccgtaag ctttaatgga gttaagggag attatcttca    2880
   tatgatgtat ttagataatg agcctgcaat tgcagtagga agggaattaa gtgcatatcc    2940
   taaaaagctc gggtatccaa agctttttgt ggattcagat actttagtag gaactttaga    3000
   ctatggaaaa cttagagttg cgacagctac aatggggtac aaacataaag ccttagatgc    3060
   taatgaagca aaggatcaaa tttgtcgccc taattatatg ttgaaaataa tacccaatta    3120
   tgatggaagc cctagaatat gtgagcttat aaatgcgaaa atcacagatg ttaccgtaca    3180
   tgaagcttgg acaggaccaa ctcgactgca gttatttgat cacgctatgg cgccacttaa    3240
   tgatttgcca gtaaaagaga ttgtttctag ctctcacatt cttgcagata taatattgcc    3300
   tagagctgaa gttatatatg attatcttaa gtaataaaaa taagagttac ctgaattcgt    3360
   aatcatggtc atagctgttt cctgtgtgaa attgttatcc gctcacaatt ccacacaaca    3420
   tacgagccgg aagcataaag tgtaaagcct ggggtgccta atgagtgagc taactcacat    3480
   taattgcgtt gcgctcactg cccgctttcc agtcgggaaa cctgtcgtgc cagctgcatt    3540
   aatgaatcgg ccaacgcgcg gggagaggcg gtttgcgtat tgggcgctct tccgcttcct    3600
   cgctcactga ctcgctgcgc tcggtcgttc ggctgcggcg agcggtatca gctcactcaa    3660
   aggcggtaat acggttatcc acagaatcag gggataacgc aggaaagaac atgtgagcaa    3720
   aaggccagca aaaggccagg aaccgtaaaa aggccgcgtt gctggcgttt ttccataggc    3780
   tccgcccccc tgacgagcat cacaaaaatc gacgctcaag tcagaggtgg cgaaacccga    3840
   caggactata aagataccag gcgtttcccc ctggaagctc cctcgtgcgc tctcctgttc    3900
   cgaccctgcc gcttaccgga tacctgtccg cctttctccc ttcgggaagc gtggcgcttt    3960
   ctcatagctc acgctgtagg tatctcagtt cggtgtaggt cgttcgctcc aagctgggct    4020
   gtgtgcacga accccccgtt cagcccgacc gctgcgcctt atccggtaac tatcgtcttg    4080
   agtccaaccc ggtaagacac gacttatcgc cactggcagc agccactggt aacaggatta 4140
   gcagagcgag gtatgtaggc ggtgctacag agttcttgaa gtggtggcct aactacggct 4200
   acactagaag gacagtattt ggtatctgcg ctctgctgaa gccagttacc ttcggaaaaa 4260
   gagttggtag ctcttgatcc ggcaaacaaa ccaccgctgg tagcggtggt ttttttgttt 4320
   gcaagcagca gattacgcgc agaaaaaaag gatctcaaga agatcctttg atcttttcta 4380
   cggggtctga cgctcagtgg aacgaaaact cacgttaagg gattttggtc atgagattat 4440
   caaaaaggat cttcacctag atccttttaa attaaaaatg aagttttaaa tcaatctaaa 4500
   gtatatatga gtaaacttgg tctgacagtt accaatgctt aatcagtgag gcacctatct 4560
   cagcgatctg tctatttcgt tcatccatag ttgcctgact ccccgtcgtg tagataacta 4620
   cgatacggga gggcttacca tctggcccca gtgctgcaat gataccgcga gacccacgct 4680
   caccggctcc agatttatca gcaataaacc agccagccgg aagggccgag cgcagaagtg 4740
   gtcctgcaac tttatccgcc tccatccagt ctattaattg ttgccgggaa gctagagtaa 4800
   gtagttcgcc agttaatagt ttgcgcaacg ttgttgccat tgctacaggc atcgtggtgt 4860
   cacgctcgtc gtttggtatg gcttcattca gctccggttc ccaacgatca aggcgagtta 4920
   catgatcccc catgttgtgc aaaaaagcgg ttagctcctt cggtcctccg atcgttgtca 4980
   gaagtaagtt ggccgcagtg ttatcactca tggttatggc agcactgcat aattctctta 5040
   ctgtcatgcc atccgtaaga tgcttttctg tgactggtga gtactcaacc aagtcattct 5100
   gagaatagtg tatgcggcga ccgagttgct cttgcccggc gtcaatacgg gataataccg 5160
   cgccacatag cagaacttta aaagtgctca tcattggaaa acgttcttcg gggcgaaaac 5220
   tctcaaggat cttaccgctg ttgagatcca gttcgatgta acccactcgt gcacccaact 5280
   gatcttcagc atcttttact ttcaccagcg tttctgggtg agcaaaaaca ggaaggcaaa 5340
   atgccgcaaa aaagggaata agggcgacac ggaaatgttg aatactcata ctcttccttt 5400
   ttcaatatta ttgaagcatt tatcagggtt attgtctcat gagcggatac atatttgaat 5460
   gtatttagaa aaataaacaa ataggggttc cgcgcacatt tccccgaaaa gtgccacctg 5520
   acgtctaaga aaccattatt atcatgacat taacctataa aaataggcgt atcacgaggc 5580
   cctttcgtc 5589
<210> 11
   <211> 5098
   <212> DNA
   <213> Artificial
<220>
   <223> Plasmid
<400> 11
   tcgcgcgttt cggtgatgac ggtgaaaacc tctgacacat gcagctcccg gagacggtca 60
   cagcttgtct gtaagcggat gccgggagca gacaagcccg tcagggcgcg tcagcgggtg 120
   ttggcgggtg tcggggctgg cttaactatg cggcatcaga gcagattgta ctgagagtgc 180
   accatatgcg gtgtgaaata ccgcacagat gcgtaaggag aaaataccgc atcaggcgcc 240
   attcgccatt caggctgcgc aactgttggg aagggcgatc ggtgcgggcc tcttcgctat 300
   tacgccagct ggcgaaaggg ggatgtgctg caaggcgatt aagttgggta acgccagggt 360
   tttcccagtc acgacgttgt aaaacgacgg ccagtgaatt caggtaactc ttatttttat 420
   tacttaagat aatcatatat aacttcagct ctaggcaata ttatatctgc aagaatgtga 480
   gagctagaaa caatctcttt tactggcaaa tcattaagtg gcgccatagc gtgatcaaat 540
   aactgcagtc gagttggtcc tgtccaagct tcatgtacgg taacatctgt gattttcgca 600
   tttataagct cacatattct agggcttcca tcataattgg gtattatttt caacatataa 660
   ttagggcgac aaatttgatc ctttgcttca ttagcatcta aggctttatg tttgtacccc 720
   attgtagctg tcgcaactct aagttttcca tagtctaaag ttcctactaa agtatctgaa 780
   tccacaaaaa gctttggata cccgagcttt ttaggatatg cacttaattc ccttcctact 840
   gcaattgcag gctcattatc taaatacatc atatgaagat aatctccctt aactccatta 900
   aagcttacgg gaatagcctg tccgctttct gtataacaac caagtccact cgtatcatgc 960
   attgccataa tttcaaacct gactaagggc tcatcaattt ctaaaggctc tggcacaact 1020
   ttacgaagtg catccatatc tgtacgatat acaatgttaa aatactcacg attatgaaat 1080
   ttatagggtc ctctaggaaa tgcaggcgaa gttaatggcg tgctaatttg tttaattact 1140
   tcatccttta acataaaagg tacctcattg catactccga aaaattattt taagtgatga 1200
   aacgccgctt taacttcttt gggaaacgcc actggtttca tcttgcctag atcaacacag 1260
   gctaccttaa cagtagcctt tgataacatc agggtgttgc gcatcagtct ctgttcaaaa 1320
   aggattgtag ccccttttac ttctgaaacc tctgtttcca ccataagtaa atcatccaat 1380
   tttgctgcca cgcaataatc aatggcgagc gttttgacaa caaatgcgag ttgttgttcc 1440
   tctagtaagg tttgttgcgt aaaatttaat gtacgcaaat attctgttct tgctcgttca 1500
   aaaaaatgca aatagcgagc gtgatacact acgccacctg catcagtatc ttcataatac 1560
   acacgaacag gaaaagaaaa gccattatcc aacatatgta tatctccttc ttaaagttaa 1620
   acaggatcct ttctagcact tttctagcaa tattgctgtt ccttgtccgc cacctataca 1680
   taaagttgct aagccttttt ttgcatctct tttttgcatt gcgtgtacaa gagtaacgag 1740
   tattcttgca cctgatgctc caattggatg accaagggca atagctcctc catttacatt 1800
   tactttattc atatcaaatt ttaaatcttt tgctactgct aaactttgag ctgcaaaagc 1860
   ttcatttgat tctattaaat ctaattcatc aactgtccaa cctgcttttt caatagctgc 1920
   ttttgttgca tagaaaggtc catatcccat tattgctggg tcaactcctg ctgaaccata 1980
   agaaactatc ttagcaagtg gttttactcc aagctcttta gctttttctg cactcatgat 2040
   tacaagtact gctgcacagt catttaatcc tgatgcatta ccagctgtaa ctgttccatc 2100
   tttttgaagg gcaggtttta attttgcaag tccttctata gttgatccaa atctagggtg 2160
   ctcatctgta tcaactacag tttctccctt tctgccttta attactacag gaactatttc 2220
   atctttaaat tgacctgatt ttatagcttc ttcagctttt ttttgtgatg caagagcaaa 2280
   ctcatcttgt tcttctcttg aaatgttcca tctctcagct atgttttctg ctgttattcc 2340
   catgtggtaa tcattaaatg catcccacaa tccgtcagtg atcatttcat caacaaattt 2400
   agcgtttccc attctatatc cccatctagc gttattcgct aagtaaggag ctctagacat 2460
   attttccata ccacctgcta ttattacgtc agcatctcct gcttttataa tttgtgctgc 2520
   taagctaact gttctaagtc ctgaaccaca aaccttatta atagtcatag ctggaatttc 2580
   aactggtaat cctgctttaa aagatgcctg tcttgctgga ttctgtccta aacctgcttg 2640
   aagaacattt cctaaaatga cttcattaac atcctctggt tttattcctg cttttttaac 2700
   tgcttccttt atagctgtag ctcctaaatc tactgctggt acatccttaa gagactttcc 2760
   ataagatcca atcgctgttc ttactgcact agctattaca acttctttca ttctaactaa 2820
   cctcctaaat tttgatacgg gtcgacctgc aggcatgcaa gcttggcgta atcatggtca 2880
   tagctgtttc ctgtgtgaaa ttgttatccg ctcacaattc cacacaacat acgagccgga 2940
   agcataaagt gtaaagcctg gggtgcctaa tgagtgagct aactcacatt aattgcgttg 3000
   cgctcactgc ccgctttcca gtcgggaaac ctgtcgtgcc agctgcatta atgaatcggc 3060
   caacgcgcgg ggagaggcgg tttgcgtatt gggcgctctt ccgcttcctc gctcactgac 3120
   tcgctgcgct cggtcgttcg gctgcggcga gcggtatcag ctcactcaaa ggcggtaata 3180
   cggttatcca cagaatcagg ggataacgca ggaaagaaca tgtgagcaaa aggccagcaa 3240
   aaggccagga accgtaaaaa ggccgcgttg ctggcgtttt tccataggct ccgcccccct 3300
   gacgagcatc acaaaaatcg acgctcaagt cagaggtggc gaaacccgac aggactataa 3360
   agataccagg cgtttccccc tggaagctcc ctcgtgcgct ctcctgttcc gaccctgccg 3420
   cttaccggat acctgtccgc ctttctccct tcgggaagcg tggcgctttc tcatagctca 3480
   cgctgtaggt atctcagttc ggtgtaggtc gttcgctcca agctgggctg tgtgcacgaa 3540
   ccccccgttc agcccgaccg ctgcgcctta tccggtaact atcgtcttga gtccaacccg 3600
   gtaagacacg acttatcgcc actggcagca gccactggta acaggattag cagagcgagg 3660
   tatgtaggcg gtgctacaga gttcttgaag tggtggccta actacggcta cactagaagg 3720
   acagtatttg gtatctgcgc tctgctgaag ccagttacct tcggaaaaag agttggtagc 3780
   tcttgatccg gcaaacaaac caccgctggt agcggtggtt tttttgtttg caagcagcag 3840
   attacgcgca gaaaaaaagg atctcaagaa gatcctttga tcttttctac ggggtctgac 3900
   gctcagtgga acgaaaactc acgttaaggg attttggtca tgagattatc aaaaaggatc 3960
   ttcacctaga tccttttaaa ttaaaaatga agttttaaat caatctaaag tatatatgag 4020
   taaacttggt ctgacagtta ccaatgctta atcagtgagg cacctatctc agcgatctgt 4080
   ctatttcgtt catccatagt tgcctgactc cccgtcgtgt agataactac gatacgggag 4140
   ggcttaccat ctggccccag tgctgcaatg ataccgcgag acccacgctc accggctcca 4200
   gatttatcag caataaacca gccagccgga agggccgagc gcagaagtgg tcctgcaact 4260
   ttatccgcct ccatccagtc tattaattgt tgccgggaag ctagagtaag tagttcgcca 4320
   gttaatagtt tgcgcaacgt tgttgccatt gctacaggca tcgtggtgtc acgctcgtcg 4380
   tttggtatgg cttcattcag ctccggttcc caacgatcaa ggcgagttac atgatccccc 4440
   atgttgtgca aaaaagcggt tagctccttc ggtcctccga tcgttgtcag aagtaagttg 4500
   gccgcagtgt tatcactcat ggttatggca gcactgcata attctcttac tgtcatgcca 4560
   tccgtaagat gcttttctgt gactggtgag tactcaacca agtcattctg agaatagtgt 4620
   atgcggcgac cgagttgctc ttgcccggcg tcaatacggg ataataccgc gccacatagc 4680
   agaactttaa aagtgctcat cattggaaaa cgttcttcgg ggcgaaaact ctcaaggatc 4740
   ttaccgctgt tgagatccag ttcgatgtaa cccactcgtg cacccaactg atcttcagca 4800
   tcttttactt tcaccagcgt ttctgggtga gcaaaaacag gaaggcaaaa tgccgcaaaa 4860
   aagggaataa gggcgacacg gaaatgttga atactcatac tcttcctttt tcaatattat 4920
   tgaagcattt atcagggtta ttgtctcatg agcggataca tatttgaatg tatttagaaa 4980
   aataaacaaa taggggttcc gcgcacattt ccccgaaaag tgccacctga cgtctaagaa 5040
   accattatta tcatgacatt aacctataaa aataggcgta tcacgaggcc ctttcgtc 5098
<210> 12
   <211> 5964
   <212> DNA
   <213> Artificial
<220>
   <223> Plasmid
<400> 12
   tcgcgcgttt cggtgatgac ggtgaaaacc tctgacacat gcagctcccg gagacggtca      60
   cagcttgtct gtaagcggat gccgggagca gacaagcccg tcagggcgcg tcagcgggtg     120
   ttggcgggtg tcggggctgg cttaactatg cggcatcaga gcagattgta ctgagagtgc     180
   accatatgcg gtgtgaaata ccgcacagat gcgtaaggag aaaataccgc atcaggcgcc     240
   attcgccatt caggctgcgc aactgttggg aagggcgatc ggtgcgggcc tcttcgctat     300
   tacgccagct ggcgaaaggg ggatgtgctg caaggcgatt aagttgggta acgccagggt     360
   tttcccagtc acgacgttgt aaaacgacgg ccagtgaatt caggtaactc ttatttttat     420
   tacttaagat aatcatatat aacttcagct ctaggcaata ttatatctgc aagaatgtga     480
   gagctagaaa caatctcttt tactggcaaa tcattaagtg gcgccatagc gtgatcaaat     540
   aactgcagtc gagttggtcc tgtccaagct tcatgtacgg taacatctgt gattttcgca     600
   tttataagct cacatattct agggcttcca tcataattgg gtattatttt caacatataa     660
   ttagggcgac aaatttgatc ctttgcttca ttagcatcta aggctttatg tttgtacccc     720
   attgtagctg tcgcaactct aagttttcca tagtctaaag ttcctactaa agtatctgaa     780
   tccacaaaaa gctttggata cccgagcttt ttaggatatg cacttaattc ccttcctact     840
   gcaattgcag gctcattatc taaatacatc atatgaagat aatctccctt aactccatta     900
   aagcttacgg gaatagcctg tccgctttct gtataacaac caagtccact cgtatcatgc     960
   attgccataa tttcaaacct gactaagggc tcatcaattt ctaaaggctc tggcacaact    1020
   ttacgaagtg catccatatc tgtacgatat acaatgttaa aatactcacg attatgaaat 1080
   ttatagggtc ctctaggaaa tgcaggcgaa gttaatggcg tgctaatttg tttaattact 1140
   tcatccttta acataaaagg tacctaaaca gccatgggtc taagttcatt ggatatgagt 1200
   aaatctgcag cagttaaaga ccttatttca tcaatggttg tgtttttatt aatttcagtg 1260
   agaagtaaac catcattaat aacctcaatt actccaagtt ctgttacaat tagatttgct 1320
   tgagactttg ccgtgagggg aagtgtacat ttttttaaaa ttttaggttg acctttattt 1380
   gtatgtctca ttgcaattat tactttctta gctccattta ctaaatccat agctccaccc 1440
   ataccagaga gcatttttcc aggaacaatc caattggcta tattaccctt ttcatctacc 1500
   tggagagccc ctaaaacagt aacatctacg tgaccaccac ggattagtga aaacgaaact 1560
   gagctatcga aaaatgtgcc gtcaggaagt actgttgtat agtctcctcc tgcatttact 1620
   acatctttat ctgcctcatt tattttagga ctagcgccca ttccaactat tccgttttct 1680
   gattggaaag taattttgaa attttttggt atataatctg caaccatggt aggaagacct 1740
   acacctaagt ttacaagttg accatttttt aattctcttg caactctttt ggctattatt 1800
   tctttcgcta ggtttttatc attaatcatt ttatgcaggc tcctttacta tataatttat 1860
   aagaactccg ggggtcattg ctttttcctt ttctagtttt tcacagctaa ctaaattttc 1920
   agcttcaact attacggttt tagctgccat tgccatatag ggattaaagt ttttagtagt 1980
   acctttatag aaggtgtttc cggcctcatc tacaatacta cctttaatta atgctacatc 2040
   ggctgtaaga ggtagctcta acaaatattc cgttccattt atagatattt ttttctttcc 2100
   tttttcaatc aaagttccta aacctgtttt agttagtaca ccacctaagc cagatccgcc 2160
   tgcacgtatt ctttccacta gagttccttg gggagagagc tctacttcaa gttcattatt 2220
   aaaaagtttt ttgccagtat ctgggttgct gcctatatat gaagcaataa gcttttttac 2280
   ttgattattt gatattaact taccaatacc tgtattagga taacatgtat cattacttat 2340
   aatcgttaaa ttctttatat ttaaattaac taaaaaatca attaatttgg ttggagtgcc 2400
   acagtttaaa aaacctccaa tcataattgt catcccatct ttaaagaatg accttaaatt 2460
   ttcaaatcta attattttag agttcatttg gatcctttct agcacttttc tagcaatatt 2520
   gctgttcctt gtccgccacc tatacataaa gttgctaagc ctttttttgc atctcttttt 2580
   tgcattgcgt gtacaagagt aacgagtatt cttgcacctg atgctccaat tggatgacca 2640
   agggcaatag ctcctccatt tacatttact ttattcatat caaattttaa atcttttgct 2700
   actgctaaac tttgagctgc aaaagcttca tttgattcta ttaaatctaa ttcatcaact 2760
   gtccaacctg ctttttcaat agctgctttt gttgcataga aaggtccata tcccattatt 2820
   gctgggtcaa ctcctgctga accataagaa actatcttag caagtggttt tactccaagc 2880
   tctttagctt tttctgcact catgattaca agtactgctg cacagtcatt taatcctgat 2940
   gcattaccag ctgtaactgt tccatctttt ttgaaggcag gttttaattt tgcaagtcct 3000
   tctatagttg atccaaatct agggtgctca tctgtatcaa ctacagtttc tccctttctg 3060
   cctttaatta ctacaggaac tatttcatct ttaaattgac ctgattttat agcttcttca 3120
   gctttttttt gtgatgcaag agcaaactca tcttgttctt ctcttgaaat gttccatctc 3180
   tcagctatgt tttctgctgt tattcccatg tggtaatcat taaatgcatc ccacaatccg 3240
   tcagtgatca tttcatcaac aaatttagcg tttcccattc tatatcccca tctagcgtta 3300
   ttcgctaagt aaggagctct agacatattt tccataccac ctgctattat tacgtcagca 3360
   tctcctgctt ttataatttg tgctgctaag ctaactgttc taagtcctga accacaaacc 3420
   ttattaatag tcatagctgg aatttcaact ggtaatcctg ctttaaaaga tgcctgtctt 3480
   gctggattct gtcctaaacc tgcttgaaga acatttccta aaatgacttc attaacatcc 3540
   tctggtttta ttcctgcttt tttaactgct tcctttatag ctgtagctcc taaatctact 3600
   gctggtacat ccttaagaga ctttccataa gatccaatcg ctgttcttac tgcactagct 3660
   attacaactt ctttcattct aactaacctc ctaaattttg atacgggtcg acctgcaggc 3720
   atgcaagctt ggcgtaatca tggtcatagc tgtttcctgt gtgaaattgt tatccgctca 3780
   caattccaca caacatacga gccggaagca taaagtgtaa agcctggggt gcctaatgag 3840
   tgagctaact cacattaatt gcgttgcgct cactgcccgc tttccagtcg ggaaacctgt 3900
   cgtgccagct gcattaatga atcggccaac gcgcggggag aggcggtttg cgtattgggc 3960
   gctcttccgc ttcctcgctc actgactcgc tgcgctcggt cgttcggctg cggcgagcgg 4020
   tatcagctca ctcaaaggcg gtaatacggt tatccacaga atcaggggat aacgcaggaa 4080
   agaacatgtg agcaaaaggc cagcaaaagg ccaggaaccg taaaaaggcc gcgttgctgg 4140
   cgtttttcca taggctccgc ccccctgacg agcatcacaa aaatcgacgc tcaagtcaga 4200
   ggtggcgaaa cccgacagga ctataaagat accaggcgtt tccccctgga agctccctcg 4260
   tgcgctctcc tgttccgacc ctgccgctta ccggatacct gtccgccttt ctcccttcgg 4320
   gaagcgtggc gctttctcat agctcacgct gtaggtatct cagttcggtg taggtcgttc 4380
   gctccaagct gggctgtgtg cacgaacccc ccgttcagcc cgaccgctgc gccttatccg 4440
   gtaactatcg tcttgagtcc aacccggtaa gacacgactt atcgccactg gcagcagcca 4500
   ctggtaacag gattagcaga gcgaggtatg taggcggtgc tacagagttc ttgaagtggt 4560
   ggcctaacta cggctacact agaaggacag tatttggtat ctgcgctctg ctgaagccag 4620
   ttaccttcgg aaaaagagtt ggtagctctt gatccggcaa acaaaccacc gctggtagcg 4680
   gtggtttttt tgtttgcaag cagcagatta cgcgcagaaa aaaaggatct caagaagatc 4740
   ctttgatctt ttctacgggg tctgacgctc agtggaacga aaactcacgt taagggattt 4800
   tggtcatgag attatcaaaa aggatcttca cctagatcct tttaaattaa aaatgaagtt 4860
   ttaaatcaat ctaaagtata tatgagtaaa cttggtctga cagttaccaa tgcttaatca 4920
   gtgaggcacc tatctcagcg atctgtctat ttcgttcatc catagttgcc tgactccccg 4980
   tcgtgtagat aactacgata cgggagggct taccatctgg ccccagtgct gcaatgatac 5040
   cgcgagaccc acgctcaccg gctccagatt tatcagcaat aaaccagcca gccggaaggg 5100
   ccgagcgcag aagtggtcct gcaactttat ccgcctccat ccagtctatt aattgttgcc 5160
   gggaagctag agtaagtagt tcgccagtta atagtttgcg caacgttgtt gccattgcta 5220
   caggcatcgt ggtgtcacgc tcgtcgtttg gtatggcttc attcagctcc ggttcccaac 5280
   gatcaaggcg agttacatga tcccccatgt tgtgcaaaaa agcggttagc tccttcggtc 5340
   ctccgatcgt tgtcagaagt aagttggccg cagtgttatc actcatggtt atggcagcac 5400
   tgcataattc tcttactgtc atgccatccg taagatgctt ttctgtgact ggtgagtact 5460
   caaccaagtc attctgagaa tagtgtatgc ggcgaccgag ttgctcttgc ccggcgtcaa 5520
   tacgggataa taccgcgcca catagcagaa ctttaaaagt gctcatcatt ggaaaacgtt 5580
   cttcggggcg aaaactctca aggatcttac cgctgttgag atccagttcg atgtaaccca 5640
   ctcgtgcacc caactgatct tcagcatctt ttactttcac cagcgtttct gggtgagcaa 5700
   aaacaggaag gcaaaatgcc gcaaaaaagg gaataagggc gacacggaaa tgttgaatac 5760
   tcatactctt cctttttcaa tattattgaa gcatttatca gggttattgt ctcatgagcg 5820
   gatacatatt tgaatgtatt tagaaaaata aacaaatagg ggttccgcgc acatttcccc 5880
   gaaaagtgcc acctgacgtc taagaaacca ttattatcat gacattaacc tataaaaata 5940
   ggcgtatcac gaggcccttt cgtc 5964
<210> 13
   <211> 4151
   <212> DNA
   <213> Artificial
<220>
   <223> Plasmid
<400> 13
   gatccagatc tcatcaccat caccatcact aagcttaatt agctgagctt ggactcctgt 60
   tgatagatcc agtaatgacc tcagaactcc atctggattt gttcagaacg ctcggttgcc 120
   gccgggcgtt ttttattggt gagaatccaa gctagcttgg cgagattttc aggagctaag 180
   gaagctaaaa tggagaaaaa aatcactgga tataccaccg ttgatatatc ccaatggcat 240
   cgtaaagaac attttgaggc atttcagtca gttgctcaat gtacctataa ccagaccgtt 300
   cagctggata ttacggcctt tttaaagacc gtaaagaaaa ataagcacaa gttttatccg 360
   gcctttattc acattcttgc ccgcctgatg aatgctcatc cggaatttcg tatggcaatg 420
   aaagacggtg agctggtgat atgggatagt gttcaccctt gttacaccgt tttccatgag 480
   caaactgaaa cgttttcatc gctctggagt gaataccacg acgatttccg gcagtttcta 540
   cacatatatt cgcaagatgt ggcgtgttac ggtgaaaacc tggcctattt ccctaaaggg 600
   tttattgaga atatgttttt cgtctcagcc aatccctggg tgagtttcac cagttttgat 660
   ttaaacgtgg ccaatatgga caacttcttc gcccccgttt tcaccatgca tgggcaaata 720
   ttatacgcaa ggcgacaagg tgctgatgcc gctggcgatt caggttcatc atgccgtctg 780
   tgatggcttc catgtcggca gaatgcttaa tgaattacaa cagtactgcg atgagtggca 840
   gggcggggcg taattttttt aaggcagtta ttggtgccct taaacgcctg gggtaatgac 900
   tctctagctt gaggcatcaa ataaaacgaa aggctcagtc gaaagactgg gcctttcgtt 960
   ttatctgttg tttgtcggtg aacgctctcc tgagtaggac aaatccgccg ctctagagct 1020
   gcctcgcgcg tttcggtgat gacggtgaaa acctctgaca catgcagctc ccggagacgg 1080
   tcacagcttg tctgtaagcg gatgccggga gcagacaagc ccgtcagggc gcgtcagcgg 1140
   gtgttggcgg gtgtcggggc gcagccatga cccagtcacg tagcgatagc ggagtgtata 1200
   ctggcttaac tatgcggcat cagagcagat tgtactgaga gtgcaccata tgcggtgtga 1260
   aataccgcac agatgcgtaa ggagaaaata ccgcatcagg cgctcttccg cttcctcgct 1320
   cactgactcg ctgcgctcgg tctgtcggct gcggcgagcg gtatcagctc actcaaaggc 1380
   ggtaatacgg ttatccacag aatcagggga taacgcagga aagaacatgt gagcaaaagg 1440
   ccagcaaaag gccaggaacc gtaaaaaggc cgcgttgctg gcgtttttcc ataggctccg 1500
   cccccctgac gagcatcaca aaaatcgacg ctcaagtcag aggtggcgaa acccgacagg 1560
   actataaaga taccaggcgt ttccccctgg aagctccctc gtgcgctctc ctgttccgac 1620
   cctgccgctt accggatacc tgtccgcctt tctcccttcg ggaagcgtgg cgctttctca 1680
   atgctcacgc tgtaggtatc tcagttcggt gtaggtcgtt cgctccaagc tgggctgtgt 1740
   gcacgaaccc cccgttcagc ccgaccgctg cgccttatcc ggtaactatc gtcttgagtc 1800
   caacccggta agacacgact tatcgccact ggcagcagcc actggtaaca ggattagcag 1860
   agcgaggtat gtaggcggtg ctacagagtt cttgaagtgg tggcctaact acggctacac 1920
   tagaaggaca gtatttggta tctgcgctct gctgaagcca gttaccttcg gaaaaagagt 1980
   tggtagctct tgatccggca aacaaaccac cgctggtagc ggtggttttt ttgtttgcaa 2040
   gcagcagatt acgcgcagaa aaaaaggatc tcaagaagat cctttgatct tttctacggg 2100
   gtctgacgct cagtggaacg aaaactcacg ttaagggatt ttggtcatga gattatcaaa 2160
   aaggatcttc acctagatcc ttttaaatta aaaatgaagt tttaaatcaa tctaaagtat 2220
   atatgagtaa acttggtctg acagttacca atgcttaatc agtgaggcac ctatctcagc 2280
   gatctgtcta tttcgttcat ccatagctgc ctgactcccc gtcgtgtaga taactacgat 2340
   acgggagggc ttaccatctg gccccagtgc tgcaatgata ccgcgagacc cacgctcacc 2400
   ggctccagat ttatcagcaa taaaccagcc agccggaagg gccgagcgca gaagtggtcc 2460
   tgcaacttta tccgcctcca tccagtctat taattgttgc cgggaagcta gagtaagtag 2520
   ttcgccagtt aatagtttgc gcaacgttgt tgccattgct acaggcatcg tggtgtcacg 2580
   ctcgtcgttt ggtatggctt cattcagctc cggttcccaa cgatcaaggc gagttacatg 2640
   atcccccatg ttgtgcaaaa aagcggttag ctccttcggt cctccgatcg ttgtcagaag 2700
   taagttggcc gcagtgttat cactcatggt tatggcagca ctgcataatt ctcttactgt 2760
   catgccatcc gtaagatgct tttctgtgac tggtgagtac tcaaccaagt cattctgaga 2820
   atagtgtatg cggcgaccga gttgctcttg cccggcgtca atacgggata ataccgcgcc 2880
   acatagcaga actttaaaag tgctcatcat tggaaaacgt tcttcggggc gaaaactctc 2940
   aaggatctta ccgctgttga gatccagttc gatgtaaccc actcgtgcac ccaactgatc 3000
   ttcagcatct tttactttca ccagcgtttc tgggtgagca aaaacaggaa ggcaaaatgc 3060
   cgcaaaaaag ggaataaggg cgacacggaa atgttgaata ctcatactct tcctttttca 3120
   atattattga agcatttatc agggttattg tctcatgagc ggatacatat ttgaatgtat 3180
   ttagaaaaat aaacaaatag gggttccgcg cacatttccc cgaaaagtgc cacctgacgt 3240
   ctaagaaacc attattatca tgacattaac ctataaaaat aggcgtatca cgaggccctt 3300
   tcgtcttcac ctcgagaaat cataaaaaat ttatttgctt tgtgagcgga taacaattat 3360
   aatagattca attgtgagcg gataacaatt tcacacagaa ttcattaaag aggagaaatt 3420
   aaccatgggc caactcttca aatcatttga tgcgtcggaa aaaacacagc tcatctgttt 3480
   tccgtttgcc ggcggctatt cggcgtcgtt tcgccctctc catgcttttt tgcaggggga 3540
   gtgcgagatg ctcgctgccg agccgccggg acacggcacg aatcaaacgt cagccattga 3600
   ggatctcgaa gagctgacgg atttgtacaa gcaagaactg aaccttcgcc ctgatcggcc 3660
   gtttgtgctg ttcggacaca gtatgggcgg aatgatcacc ttcaggctgg cgcaaaagct 3720
   tgagcgtgaa ggcatctttc cgcaggcggt tatcatttct gcaatccagc cgcctcatat 3780
   tcagcggaag aaagtgtccc acctgcctga tgatcagttt ctcgatcata ttatccaatt 3840
   aggcggaatg cccgcagagc ttgttgaaaa taaggaggtc atgtcctttt tcctgccttc 3900
   tttccgatca gattaccggg ctcttgaaca atttgagctt tacgatctgg cccagatcca 3960
   gtcgcctgtt catgtcttta acgggcttga tgataaaaaa tgcatacgag atgcggaagg 4020
   gtggaagaag tgggcaaaag acatcacatt ccatcaattt gacggcgggc acatgttcct 4080
   gctgtcacaa acggaagaag tcgcagaacg gatttttgcg atcttgaatc agcatccgat 4140
   cattcaaccg g 4151
<210> 14
   <211> 5964
   <212> DNA
   <213> Artificial
<220>
   <223> Plasmid
<400> 14
   tcgcgcgttt cggtgatgac ggtgaaaacc tctgacacat gcagctcccg gagacggtca     60
   cagcttgtct gtaagcggat gccgggagca gacaagcccg tcagggcgcg tcagcgggtg    120
   ttggcgggtg tcggggctgg cttaactatg cggcatcaga gcagattgta ctgagagtgc    180
   accatatgcg gtgtgaaata ccgcacagat gcgtaaggag aaaataccgc atcaggcgcc    240
   attcgccatt caggctgcgc aactgttggg aagggcgatc ggtgcgggcc tcttcgctat    300
   tacgccagct ggcgaaaggg ggatgtgctg caaggcgatt aagttgggta acgccagggt    360
   tttcccagtc acgacgttgt aaaacgacgg ccagtgccaa gcttgcatgc ctgcaggtcg    420
   acccgtatca aaatttagga ggttagttag aatgaaagaa gttgtaatag ctagtgcagt    480
   aagaacagcg attggatctt atggaaagtc tcttaaggat gtaccagcag tagatttagg    540
   agctacagct ataaaggaag cagttaaaaa agcaggaata aaaccagagg atgttaatga 600
   agtcatttta ggaaatgttc ttcaagcagg tttaggacag aatccagcaa gacaggcatc 660
   ttttaaagca ggattaccag ttgaaattcc agctatgact attaataagg tttgtggttc 720
   aggacttaga acagttagct tagcagcaca aattataaaa gcaggagatg ctgacgtaat 780
   aatagcaggt ggtatggaaa atatgtctag agctccttac ttagcgaata acgctagatg 840
   gggatataga atgggaaacg ctaaatttgt tgatgaaatg atcactgacg gattgtggga 900
   tgcatttaat gattaccaca tgggaataac agcagaaaac atagctgaga gatggaacat 960
   ttcaagagaa gaacaagatg agtttgctct tgcatcacaa aaaaaagctg aagaagctat 1020
   aaaatcaggt caatttaaag atgaaatagt tcctgtagta attaaaggca gaaagggaga 1080
   aactgtagtt gatacagatg agcaccctag atttggatca actatagaag gacttgcaaa 1140
   attaaaacct gccttcaaaa aagatggaac agttacagct ggtaatgcat caggattaaa 1200
   tgactgtgca gcagtacttg taatcatgag tgcagaaaaa gctaaagagc ttggagtaaa 1260
   accacttgct aagatagttt cttatggttc agcaggagtt gacccagcaa taatgggata 1320
   tggacctttc tatgcaacaa aagcagctat tgaaaaagca ggttggacag ttgatgaatt 1380
   agatttaata gaatcaaatg aagcttttgc agctcaaagt ttagcagtag caaaagattt 1440
   aaaatttgat atgaataaag taaatgtaaa tggaggagct attgcccttg gtcatccaat 1500
   tggagcatca ggtgcaagaa tactcgttac tcttgtacac gcaatgcaaa aaagagatgc 1560
   aaaaaaaggc ttagcaactt tatgtatagg tggcggacaa ggaacagcaa tattgctaga 1620
   aaagtgctag aaaggatcca aatgaactct aaaataatta gatttgaaaa tttaaggtca 1680
   ttctttaaag atgggatgac aattatgatt ggaggttttt taaactgtgg cactccaacc 1740
   aaattaattg attttttagt taatttaaat ataaagaatt taacgattat aagtaatgat 1800
   acatgttatc ctaatacagg tattggtaag ttaatatcaa ataatcaagt aaaaaagctt 1860
   attgcttcat atataggcag caacccagat actggcaaaa aactttttaa taatgaactt 1920
   gaagtagagc tctctcccca aggaactcta gtggaaagaa tacgtgcagg cggatctggc 1980
   ttaggtggtg tactaactaa aacaggttta ggaactttga ttgaaaaagg aaagaaaaaa 2040
   atatctataa atggaacgga atatttgtta gagctacctc ttacagccga tgtagcatta 2100
   attaaaggta gtattgtaga tgaggccgga aacaccttct ataaaggtac tactaaaaac 2160
   tttaatccct atatggcaat ggcagctaaa accgtaatag ttgaagctga aaatttagtt 2220
   agctgtgaaa aactagaaaa ggaaaaagca atgacccccg gagttcttat aaattatata 2280
   gtaaaggagc ctgcataaaa tgattaatga taaaaaccta gcgaaagaaa taatagccaa 2340
   aagagttgca agagaattaa aaaatggtca acttgtaaac ttaggtgtag gtcttcctac 2400
   catggttgca gattatatac caaaaaattt caaaattact ttccaatcag aaaacggaat 2460
   agttggaatg ggcgctagtc ctaaaataaa tgaggcagat aaagatgtag taaatgcagg 2520
   aggagactat acaacagtac ttcctgacgg cacatttttc gatagctcag tttcgttttc 2580
   actaatccgt ggtggtcacg tagatgttac tgttttaggg gctctccagg tagatgaaaa 2640
   gggtaatata gccaattgga ttgttcctgg aaaaatgctc tctggtatgg gtggagctat   2700
   ggatttagta aatggagcta agaaagtaat aattgcaatg agacatacaa ataaaggtca   2760
   acctaaaatt ttaaaaaaat gtacacttcc cctcacggca aagtctcaag caaatctaat   2820
   tgtaacagaa cttggagtaa ttgaggttat taatgatggt ttacttctca ctgaaattaa   2880
   taaaaacaca accattgatg aaataaggtc tttaactgct gcagatttac tcatatccaa   2940
   tgaacttaga cccatggctg tttaggtacc ttttatgtta aaggatgaag taattaaaca   3000
   aattagcacg ccattaactt cgcctgcatt tcctagagga ccctataaat ttcataatcg   3060
   tgagtatttt aacattgtat atcgtacaga tatggatgca cttcgtaaag ttgtgccaga   3120
   gcctttagaa attgatgagc ccttagtcag gtttgaaatt atggcaatgc atgatacgag   3180
   tggacttggt tgttatacag aaagcggaca ggctattccc gtaagcttta atggagttaa   3240
   gggagattat cttcatatga tgtatttaga taatgagcct gcaattgcag taggaaggga   3300
   attaagtgca tatcctaaaa agctcgggta tccaaagctt tttgtggatt cagatacttt   3360
   agtaggaact ttagactatg gaaaacttag agttgcgaca gctacaatgg ggtacaaaca   3420
   taaagcctta gatgctaatg aagcaaagga tcaaatttgt cgccctaatt atatgttgaa   3480
   aataataccc aattatgatg gaagccctag aatatgtgag cttataaatg cgaaaatcac   3540
   agatgttacc gtacatgaag cttggacagg accaactcga ctgcagttat ttgatcacgc   3600
   tatggcgcca cttaatgatt tgccagtaaa agagattgtt tctagctctc acattcttgc   3660
   agatataata ttgcctagag ctgaagttat atatgattat cttaagtaat aaaaataaga   3720
   gttacctgaa ttcgtaatca tggtcatagc tgtttcctgt gtgaaattgt tatccgctca   3780
   caattccaca caacatacga gccggaagca taaagtgtaa agcctggggt gcctaatgag   3840
   tgagctaact cacattaatt gcgttgcgct cactgcccgc tttccagtcg ggaaacctgt   3900
   cgtgccagct gcattaatga atcggccaac gcgcggggag aggcggtttg cgtattgggc   3960
   gctcttccgc ttcctcgctc actgactcgc tgcgctcggt cgttcggctg cggcgagcgg   4020
   tatcagctca ctcaaaggcg gtaatacggt tatccacaga atcaggggat aacgcaggaa   4080
   agaacatgtg agcaaaaggc cagcaaaagg ccaggaaccg taaaaaggcc gcgttgctgg   4140
   cgtttttcca taggctccgc ccccctgacg agcatcacaa aaatcgacgc tcaagtcaga   4200
   ggtggcgaaa cccgacagga ctataaagat accaggcgtt tccccctgga agctccctcg   4260
   tgcgctctcc tgttccgacc ctgccgctta ccggatacct gtccgccttt ctcccttcgg   4320
   gaagcgtggc gctttctcat agctcacgct gtaggtatct cagttcggtg taggtcgttc   4380
   gctccaagct gggctgtgtg cacgaacccc ccgttcagcc cgaccgctgc gccttatccg   4440
   gtaactatcg tcttgagtcc aacccggtaa gacacgactt atcgccactg gcagcagcca   4500
   ctggtaacag gattagcaga gcgaggtatg taggcggtgc tacagagttc ttgaagtggt   4560
   ggcctaacta cggctacact agaaggacag tatttggtat ctgcgctctg ctgaagccag   4620
   ttaccttcgg aaaaagagtt ggtagctctt gatccggcaa acaaaccacc gctggtagcg   4680
   gtggtttttt tgtttgcaag cagcagatta cgcgcagaaa aaaaggatct caagaagatc 4740
   ctttgatctt ttctacgggg tctgacgctc agtggaacga aaactcacgt taagggattt 4800
   tggtcatgag attatcaaaa aggatcttca cctagatcct tttaaattaa aaatgaagtt 4860
   ttaaatcaat ctaaagtata tatgagtaaa cttggtctga cagttaccaa tgcttaatca 4920
   gtgaggcacc tatctcagcg atctgtctat ttcgttcatc catagttgcc tgactccccg 4980
   tcgtgtagat aactacgata cgggagggct taccatctgg ccccagtgct gcaatgatac 5040
   cgcgagaccc acgctcaccg gctccagatt tatcagcaat aaaccagcca gccggaaggg 5100
   ccgagcgcag aagtggtcct gcaactttat ccgcctccat ccagtctatt aattgttgcc 5160
   gggaagctag agtaagtagt tcgccagtta atagtttgcg caacgttgtt gccattgcta 5220
   caggcatcgt ggtgtcacgc tcgtcgtttg gtatggcttc attcagctcc ggttcccaac 5280
   gatcaaggcg agttacatga tcccccatgt tgtgcaaaaa agcggttagc tccttcggtc 5340
   ctccgatcgt tgtcagaagt aagttggccg cagtgttatc actcatggtt atggcagcac 5400
   tgcataattc tcttactgtc atgccatccg taagatgctt ttctgtgact ggtgagtact 5460
   caaccaagtc attctgagaa tagtgtatgc ggcgaccgag ttgctcttgc ccggcgtcaa 5520
   tacgggataa taccgcgcca catagcagaa ctttaaaagt gctcatcatt ggaaaacgtt 5580
   cttcggggcg aaaactctca aggatcttac cgctgttgag atccagttcg atgtaaccca 5640
   ctcgtgcacc caactgatct tcagcatctt ttactttcac cagcgtttct gggtgagcaa 5700
   aaacaggaag gcaaaatgcc gcaaaaaagg gaataagggc gacacggaaa tgttgaatac 5760
   tcatactctt cctttttcaa tattattgaa gcatttatca gggttattgt ctcatgagcg 5820
   gatacatatt tgaatgtatt tagaaaaata aacaaatagg ggttccgcgc acatttcccc 5880
   gaaaagtgcc acctgacgtc taagaaacca ttattatcat gacattaacc tataaaaata 5940
   ggcgtatcac gaggcccttt cgtc 5964
<210> 15
   <211> 7849
   <212> DNA
   <213> Artificial
<220>
   <223> Plasmid
<400> 15
   aactacgtca ggtggcactt ttcggggaaa tgtgcgcgga acccctattt gtttattttt 60
   ctaaatacat tcaaatatgt atccgctcat gagacaataa ccctgataaa tgcttcaata 120
   atattgaaaa aggaagagta tgagtattca acatttccgt gtcgccctta ttcccttttt 180
   tgcggcattt tgccttcctg tttttgctca cccagaaacg ctggtgaaag taaaagatgc 240
   tgaagatcag ttgggtgcac gagtgggtta catcgaactg gatctcaaca gcggtaagat 300
   ccttgagagt tttcgccccg aagaacgttc tccaatgatg agcactttta aagttctgct 360
   atgtggcgcg gtattatccc gtgttgacgc cgggcaagag caactcggtc gccgcataca 420
   ctattctcag aatgacttgg ttgagtactc accagtcaca gaaaagcatc ttacggatgg 480
   catgacagta agagaattat gcagtgctgc cataaccatg agtgataaca ctgcggccaa 540
   cttacttctg acaacgatcg gaggaccgaa ggagctaacc gcttttttgc acaacatggg 600
   ggatcatgta actcgccttg atcgttggga accggagctg aatgaagcca taccaaacga 660
   cgagcgtgac accacgatgc ctgtagcaat ggcaacaacg ttgcgcaaac tattaactgg 720
   cgaactactt actctagctt cccggcaaca attaatagac tggatggagg cggataaagt 780
   tgcaggacca cttctgcgct cggcccttcc ggctggctgg tttattgctg ataaatctgg 840
   agccggtgag cgtgggtctc gcggtatcat tgcagcactg gggccagatg gtaagccctc 900
   ccgtatcgta gttatctaca cgacggggag tcaggcaact atggatgaac gaaatagaca 960
   gatcgctgag ataggtgcct cactgattaa gcattggtaa ctgtcagacc aagtttactc 1020
   atatatactt tagattgatt taccccggtt gataatcaga aaagccccaa aaacaggaag 1080
   attgtataag caaatattta aattgtaaac gttaatattt tgttaaaatt cgcgttaaat 1140
   ttttgttaaa tcagctcatt ttttaaccaa taggccgaaa tcggcaaaat cccttataaa 1200
   tcaaaagaat agcccgagat agggttgagt gttgttccag tttggaacaa gagtccacta 1260
   ttaaagaacg tggactccaa cgtcaaaggg cgaaaaaccg tctatcaggg cgatggccca 1320
   ctacgtgaac catcacccaa atcaagtttt ttggggtcga ggtgccgtaa agcactaaat 1380
   cggaacccta aagggagccc ccgatttaga gcttgacggg gaaagccggc gaacgtggcg 1440
   agaaaggaag ggaagaaagc gaaaggagcg ggcgctaggg cgctggcaag tgtagcggtc 1500
   acgctgcgcg taaccaccac acccgccgcg cttaatgcgc cgctacaggg cgcgtaaaag 1560
   gatctaggtg aagatccttt ttgataatct catgaccaaa atcccttaac gtgagttttc 1620
   gttccactga gcgtcagacc ccgtagaaaa gatcaaagga tcttcttgag atcctttttt 1680
   tctgcgcgta atctgctgct tgcaaacaaa aaaaccaccg ctaccagcgg tggtttgttt 1740
   gccggatcaa gagctaccaa ctctttttcc gaaggtaact ggcttcagca gagcgcagat 1800
   accaaatact gtccttctag tgtagccgta gttaggccac cacttcaaga actctgtagc 1860
   accgcctaca tacctcgctc tgctaatcct gttaccagtg gctgctgcca gtggcgataa 1920
   gtcgtgtctt accgggttgg actcaagacg atagttaccg gataaggcgc agcggtcggg 1980
   ctgaacgggg ggttcgtgca cacagcccag cttggagcga acgacctaca ccgaactgag 2040
   atacctacag cgtgagctat gagaaagcgc cacgcttccc gaagggagaa aggcggacag 2100
   gtatccggta agcggcaggg tcggaacagg agagcgcacg agggagcttc cagggggaaa 2160
   cgcctggtat ctttatagtc ctgtcgggtt tcgccacctc tgacttgagc gtcgattttt 2220
   gtgatgctcg tcaggggggc ggagcctatg gaaaaacgcc agcaacgcgg cctttttacg 2280
   gttcctggcc ttttgctggc cttttgctca catgttcttt cctgcgttat cccctgattc 2340
   tgtggataac cgtattaccg cctttgagtg agctgatacc gctcgccgca gccgaacgac 2400
   cgagcgcagc gagtcagtga gcgaggaagc tatggtgcac tctcagtaca atctgctctg 2460
   atgccgcata gttaagccag tatacactcc gctatcgcta cgtgactggg tcatggctgc 2520
   gccccgacac ccgccaacac ccgctgacgc gccctgacgg gcttgtctgc tcccggcatc 2580
   cgcttacaga caagctgtga ccgtctccgg gagctgcatg tgtcagaggt tttcaccgtc 2640
   atcaccgaaa cgcgcgaggc agctgcggta aagctcatca gcgtggtcgt gcagcgattc 2700
   acagatgtct gcctgttcat ccgcgtccag ctcgttgagt ttctccagaa gcgttaatgt 2760
   ctggcttctg ataaagcggg ccatgttaag ggcggttttt tcctgtttgg tcactgatgc 2820
   ctccgtgtaa gggggatttc tgttcatggg ggtaatgata ccgatgaaac gagagaggat 2880
   gctcacgata cgggttactg atgatgaaca tgcccggtta ctggaacgtt gtgagggtaa 2940
   acaactggcg gtatggatgc ggcgggacca gagaaaaatc actcagggtc aatgccagcc 3000
   gaacgccagc aagacgtagc ccagcgcgtc ggccgccatg ccggcgataa tggcctgctt 3060
   ctcgccgaaa cgtttggtgg cgggaccagt gacgaaggct tgagcgaggg cgtgcaagat 3120
   tccgaatacc gcaagcgaca ggccgatcat cgtcgcgctc cagcgaaagc ggtcctcgcc 3180
   gaaaatgacc cagagcgctg ccggcacctg tcctacgagt tgcatgataa agaagacagt 3240
   cataagtgcg gcgacgatag tcatgccccg cgcccaccgg aaggagctga ctgggttgaa 3300
   ggctctcaag ggcatcggtc gagatcccgg tgcctaatga gtgagctaac ttacattaat 3360
   tgcgttgcgc tcactgcccg ctttccagtc gggaaacctg tcgtgccagc tgcattaatg 3420
   aatcggccaa cgcgcgggga gaggcggttt gcgtattggg cgccagggtg gtttttcttt 3480
   tcaccagtga gacgggcaac agctgattgc ccttcaccgc ctggccctga gagagttgca 3540
   gcaagcggtc cacgctggtt tgccccagca ggcgaaaatc ctgtttgatg gtggttaacg 3600
   gcgggatata acatgagctg tcttcggtat cgtcgtatcc cactaccgag atatccgcac 3660
   caacgcgcag cccggactcg gtaatggcgc gcattgcgcc cagcgccatc tgatcgttgg 3720
   caaccagcat cgcagtggga acgatgccct cattcagcat ttgcatggtt tgttgaaaac 3780
   cggacatggc actccagtcg ccttcccgtt ccgctatcgg ctgaatttga ttgcgagtga 3840
   gatatttatg ccagccagcc agacgcagac gcgccgagac agaacttaat gggcccgcta 3900
   acagcgcgat ttgctggtga cccaatgcga ccagatgctc cacgcccagt cgcgtaccgt 3960
   cttcatggga gaaaataata ctgttgatgg gtgtctggtc agagacatca agaaataacg 4020
   ccggaacatt agtgcaggca gcttccacag caatggcatc ctggtcatcc agcggatagt 4080
   taatgatcag cccactgacg cgttgcgcga gaagattgtg caccgccgct ttacaggctt 4140
   cgacgccgct tcgttctacc atcgacacca ccacgctggc acccagttga tcggcgcgag 4200
   atttaatcgc cgcgacaatt tgcgacggcg cgtgcagggc cagactggag gtggcaacgc 4260
   caatcagcaa cgactgtttg cccgccagtt gttgtgccac gcggttggga atgtaattca 4320
   gctccgccat cgccgcttcc acttttcccc gcgttttcgc agaaacgtgg ctggcctggt 4380
   tcaccacgcg ggaaacggtc tgataagaga caccggcata ctctgcgaca tcgtataacg 4440
   ttactggttt cacattcacc accctgaatt gactctcttc cgggcgctat catgccatac 4500
   cgcgaaaggt tttgcgccat tcgatggtgt ccgggatctc gacgctctcc cttatgcgac 4560
   tcctgcatta ggaagcagcc cagtagtagg ttgaggccgt tgagcaccgc cgccgcaagg 4620
   aatggtgcat gccggcatgc cgccctttcg tcttcaagaa ttaattccca attccccagg 4680
   catcaaataa aacgaaaggc tcagtcgaaa gactgggcct ttcgttttat ctgttgtttg 4740
   tcggtgaacg ctctcctgag taggacaaat ccgccgggag cggatttgaa cgttgcgaag 4800
   caacggcccg gagggtggcg ggcaggacgc ccgccataaa ctgccaggaa ttaattcccc 4860
   aggcatcaaa taaaacgaaa ggctcagtcg aaagactggg cctttcgttt tatctgttgt 4920
   ttgtcggtga acgctctcct gagtaggaca aatccgccgg gagcggattt gaacgttgcg 4980
   aagcaacggc ccggagggtg gcgggcagga cgcccgccat aaactgccag gaattaattc 5040
   cccaggcatc aaataaaacg aaaggctcag tcgaaagact gggcctttcg ttttatctgt 5100
   tgtttgtcgg tgaacgctct cctgagtagg acaaatccgc cgggagcgga tttgaacgtt 5160
   gcgaagcaac ggcccggagg gtggcgggca ggacgcccgc cataaactgc caggaattaa 5220
   ttccccaggc atcaaataaa acgaaaggct cagtcgaaag actgggcctt tcgttttatc 5280
   tgttgtttgt cggtgaacgc tctcctgagt aggacaaatc cgccgggagc ggatttgaac 5340
   gttgcgaagc aacggcccgg agggtggcgg gcaggacgcc cgccataaac tgccaggaat 5400
   taattcccca ggcatcaaat aaaacgaaag gctcagtcga aagactgggc ctttcgtttt 5460
   atctgttgtt tgtcggtgaa cgctctcctg agtaggacaa atccgccggg agcggatttg 5520
   aacgttgcga agcaacggcc cggagggtgg cgggcaggac gcccgccata aactgccagg 5580
   aattggggat cggaattaat tcccggttta aaccggggat ctcgatcccg cgaaattaat 5640
   acgactcact ataggggaat tgtgagcgga taacaattcc cctctagaaa taattttgtt 5700
   taactttaag aaggagatat acatatgttg gataatggct tttcttttcc tgttcgtgtg 5760
   tattatgaag atactgatgc aggtggcgta gtgtatcacg ctcgctattt gcattttttt 5820
   gaacgagcaa gaacagaata tttgcgtaca ttaaatttta cgcaacaaac cttactagag 5880
   gaacaacaac tcgcatttgt tgtcaaaacg ctcgccattg attattgcgt ggcagcaaaa 5940
   ttggatgatt tacttatggt ggaaacagag gtttcagaag taaaaggggc tacaatcctt 6000
   tttgaacaga gactgatgcg caacaccctg atgttatcaa aggctactgt taaggtagcc 6060
   tgtgttgatc taggcaagat gaaaccagtg gcgtttccca aagaagttaa agcggcgttt 6120
   catcacttaa aactcgaggg ctcttcctgc tttgccaagg gtaccaatgt tttaatggcg 6180
   gatgggtcta ttgaatgtat tgaaaacatt gaggttggta ataaggtcat gggtaaagat 6240
   ggcagacctc gtgaggtaat taaattgccc agaggaagag aaactatgta cagcgtcgtg 6300
   cagaaaagtc agcacagagc ccacaaaagt gactcaagtc gtgaagtgcc agaattactc 6360
   aagtttacgt gtaatgcgac ccatgagttg gttgttagaa cacctcgtag tgtccgccgt 6420
   ttgtctcgta ccattaaggg tgtcgaatat tttgaagtta ttacttttga gatgggccaa 6480
   aagaaagccc ccgacggtag aattgttgag cttgtcaagg aagtttcaaa gagctaccca 6540
   atatctgagg ggcctgagag agccaacgaa ttagtagaat cctatagaaa ggcttcaaat 6600
   aaagcttatt ttgagtggac tattgaggcc agagatcttt ctctgttggg ttcccatgtt 6660
   cgtaaagcta cctaccagac ttacgctcca attctttatg agaatgacca ctttttcgac 6720
   tacatgcaaa aaagtaagtt tcatctcacc attgaaggtc caaaagtact tgcttattta 6780
   cttggtttat ggattggtga tggattgtct gacagggcaa ctttttcggt tgattccaga 6840
   gatacttctt tgatggaacg tgttactgaa tatgctgaaa agttgaattt gtgcgccgag 6900
   tataaggaca gaaaagaacc acaagttgcc aaaactgtta atttgtactc taaagttgtc 6960
   agaggtaatg gtattcgcaa taatcttaat actgagaatc cattatggga cgctattgtt 7020
   ggcttaggat tcttgaagga cggtgtcaaa aatattcctt ctttcttgtc tacggacaat 7080
   atcggtactc gtgaaacatt tcttgctggt ctaattgatt ctgatggcta tgttactgat 7140
   gagcatggta ttaaagcaac aataaagaca attcatactt ctgtcagaga tggtttggtt 7200
   tcccttgctc gttctttagg cttagtagtc tcggttaacg cagaacctgc taaggttgac 7260
   atgaatgtca ccaaacataa aattagttat gctatttata tgtctggtgg agatgttttg 7320
   cttaacgttc tttcgaagtg tgccggctct aaaaaattca ggcctgctcc cgccgctgct 7380
   tttgcacgtg agtgccgcgg attttatttc gagttacaag aattgaagga agacgattat 7440
   tatgggatta ctttatctga tgattctgat catcagtttt tgcttggatc ccaggttgtc 7500
   gtccatgcat gcggtggcct gaccggtctg aactcaggcc tcacgacaaa tcctggtgta 7560
   tccgcttggc aggtcaacac agcttatact gcgggacaat tggtcacata taacggcaag 7620
   acgtataaat gtttgcagcc ccacacctcc ttggcaggat gggaaccatc caacgttcct 7680
   gccttgtggc agcttcaatg actgcaggaa ggggatccgg ctgctaacaa agcccgaaag 7740
   gaagctgagt tggctgctgc caccgctgag caataactag cataacccct tggggcctct 7800
   aaacgggtct tgaggggttt tttgctgaaa ggaggaacta tatccggat 7849
<210> 16
   <211> 392
   <212> PRT
   <213> Clostridium acetobutylicum
<400> 16
   Met Lys Glu Val Val Ile Ala Ser Ala Val Arg Thr Ala Ile Gly Ser
   1               5                   10                  15
   Tyr Gly Lys Ser Leu Lys Asp Val Pro Ala Val Asp Leu Gly Ala Thr
               20                  25                  30
   Ala Ile Lys Glu Ala Val Lys Lys Ala Gly Ile Lys Pro Glu Asp Val
           35                  40                  45
   Asn Glu Val Ile Leu Gly Asn Val Leu Gln Ala Gly Leu Gly Gln Asn
       50                  55                  60
   Pro Ala Arg Gln Ala Ser Phe Lys Ala Gly Leu Pro Val Glu Ile Pro
   65                  70                  75                  80
   Ala Met Thr Ile Asn Lys Val Cys Gly Ser Gly Leu Arg Thr Val Ser
   85 90 95
   Leu Ala Ala Gln Ile Ile Lys Ala Gly Asp Ala Asp Val Ile Ile Ala
   100 105 110
   Gly Gly Met Glu Asn Met Ser Arg Ala Pro Tyr Leu Ala Asn Asn Ala
   115 120 125
   Arg Trp Gly Tyr Arg Met Gly Asn Ala Lys Phe Val Asp Glu Met Ile
   130 135 140
   Thr Asp Gly Leu Trp Asp Ala Phe Asn Asp Tyr His Met Gly Ile Thr
   145 150 155 160
   Ala Glu Asn Ile Ala Glu Arg Trp Asn Ile Ser Arg Glu Glu Gln Asp
   165 170 175
   Glu Phe Ala Leu Ala Ser Gln Lys Lys Ala Glu Glu Ala Ile Lys Ser
   180 185 190
   Gly Gln Phe Lys Asp Glu Ile Val Pro Val Val Ile Lys Gly Arg Lys
   195 200 205
   Gly Glu Thr Val Val Asp Thr Asp Glu His Pro Arg Phe Gly Ser Thr
   210 215 220
   Ile Glu Gly Leu Ala Lys Leu Lys Pro Ala Phe Lys Lys Asp Gly Thr
   225 230 235 240
   Val Thr Ala Gly Asn Ala Ser Gly Leu Asn Asp Cys Ala Ala Val Leu
   245 250 255
   Val Ile Met Ser Ala Glu Lys Ala Lys Glu Leu Gly Val Lys Pro Leu
   260 265 270
   Ala Lys Ile Val Ser Tyr Gly Ser Ala Gly Val Asp Pro Ala Ile Met
   275 280 285
   Gly Tyr Gly Pro Phe Tyr Ala Thr Lys Ala Ala Ile Glu Lys Ala Gly
   290 295 300
   Trp Thr Val Asp Glu Leu Asp Leu Ile Glu Ser Asn Glu Ala Phe Ala
   305 310 315 320
   Ala Gln Ser Leu Ala Val Ala Lys Asp Leu Lys Phe Asp Met Asn Lys
   325 330 335
   Val Asn Val Asn Gly Gly Ala Ile Ala Leu Gly His Pro Ile Gly Ala
   340 345 350
   Ser Gly Ala Arg Ile Leu Val Thr Leu Val His Ala Met Gln Lys Arg
   355 360 365
   Asp Ala Lys Lys Gly Leu Ala Thr Leu Cys Ile Gly Gly Gly Gln Gly
       370                 375                 380
   Thr Ala Ile Leu Leu Glu Lys Cys
   385                 390
<210> 17
   <211> 1179
   <212> DNA
   <213> Clostridium acetobutylicum
<400> 17
   atgaaagaag ttgtaatagc tagtgcagta agaacagcga ttggatctta tggaaagtct      60
   cttaaggatg taccagcagt agatttagga gctacagcta taaaggaagc agttaaaaaa     120
   gcaggaataa aaccagagga tgttaatgaa gtcattttag gaaatgttct tcaagcaggt     180
   ttaggacaga atccagcaag acaggcatct tttaaagcag gattaccagt tgaaattcca     240
   gctatgacta ttaataaggt ttgtggttca ggacttagaa cagttagctt agcagcacaa     300
   attataaaag caggagatgc tgacgtaata atagcaggtg gtatggaaaa tatgtctaga     360
   gctccttact tagcgaataa cgctagatgg ggatatagaa tgggaaacgc taaatttgtt     420
   gatgaaatga tcactgacgg attgtgggat gcatttaatg attaccacat gggaataaca     480
   gcagaaaaca tagctgagag atggaacatt tcaagagaag aacaagatga gtttgctctt     540
   gcatcacaaa aaaaagctga agaagctata aaatcaggtc aatttaaaga tgaaatagtt     600
   cctgtagtaa ttaaaggcag aaagggagaa actgtagttg atacagatga gcaccctaga     660
   tttggatcaa ctatagaagg acttgcaaaa ttaaaacctg ccttcaaaaa agatggaaca     720
   gttacagctg gtaatgcatc aggattaaat gactgtgcag cagtacttgt aatcatgagt     780
   gcagaaaaag ctaaagagct tggagtaaaa ccacttgcta agatagtttc ttatggttca     840
   gcaggagttg acccagcaat aatgggatat ggacctttct atgcaacaaa agcagctatt     900
   gaaaaagcag gttggacagt tgatgaatta gatttaatag aatcaaatga agcttttgca     960
   gctcaaagtt tagcagtagc aaaagattta aaatttgata tgaataaagt aaatgtaaat    1020
   ggaggagcta ttgcccttgg tcatccaatt ggagcatcag gtgcaagaat actcgttact    1080
   cttgtacacg caatgcaaaa aagagatgca aaaaaaggct tagcaacttt atgtataggt    1140
   ggcggacaag gaacagcaat attgctagaa aagtgctag                            1179
<210> 18
   <211> 244
   <212> PRT
   <213> Clostridium acetobutylicum
<400> 18
   Met Leu Lys Asp Glu Val Ile Lys Gln Ile Ser Thr Pro Leu Thr Ser
   1 5 10 15
   Pro Ala Phe Pro Arg Gly Pro Tyr Lys Phe His Asn Arg Glu Tyr Phe
   20 25 30
   Asn Ile Val Tyr Arg Thr Asp Met Asp Ala Leu Arg Lys Val Val Pro
   35 40 45
   Glu Pro Leu Glu Ile Asp Glu Pro Leu Val Arg Phe Glu Ile Met Ala
   50 55 60
   Met His Asp Thr Ser Gly Leu Gly Cys Tyr Thr Glu Ser Gly Gln Ala
   65 70 75 80
   Ile Pro Val Ser Cys Asn Gly Val Lys Gly Asp Tyr Leu His Met Met
   85 90 95
   Tyr Leu Asp Asn Glu Pro Ala Ile Ala Val Gly Arg Glu Leu Ser Ala
   100 105 110
   Tyr Pro Lys Lys Leu Gly Tyr Pro Lys Leu Phe Val Asp Ser Asp Thr
   115 120 125
   Leu Val Gly Thr Leu Asp Tyr Gly Lys Leu Arg Val Ala Thr Ala Thr
   130 135 140
   Met Gly Tyr Lys His Lys Ala Leu Asp Ala Asn Glu Ala Lys Asp Gln
   145 150 155 160
   Ile Cys Arg Pro Asn Tyr Met Leu Lys Ile Ile Pro Asn Tyr Asp Gly
   165 170 175
   Ser Pro Arg Ile Cys Glu Leu Ile Asn Ala Lys Ile Thr Asp Val Thr
   180 185 190
   Val His Glu Ala Trp Thr Gly Pro Thr Arg Leu Gln Leu Phe Asp His
   195 200 205
   Ala Met Ala Pro Leu Asn Asp Leu Pro Val Lys Glu Ile Val Ser Ser
   210 215 220
   Ser His Ile Leu Ala Asp Ile Ile Leu Pro Arg Ala Glu Val Ile Tyr
   225 230 235 240
   Asp Tyr Leu Lys
<210> 19
   <211> 735
   <212> DNA
   <213> Clostridium acetobutylicum
<400> 19
   atgttaaagg atgaagtaat taaacaaatt agcacgccat taacttcgcc tgcatttcct     60
   agaggaccct ataaatttca taatcgtgag tattttaaca ttgtatatcg tacagatatg    120
   gatgcacttc gtaaagttgt gccagagcct ttagaaattg atgagccctt agtcaggttt    180
   gaaattatgg caatgcatga tacgagtgga cttggttgtt atacagaaag cggacaggct    240
   attcccgtaa gctttaatgg agttaaggga gattatcttc atatgatgta tttagataat    300
   gagcctgcaa ttgcagtagg aagggaatta agtgcatatc ctaaaaagct cgggtatcca    360
   aagctttttg tggattcaga tactttagta ggaactttag actatggaaa acttagagtt    420
   gcgacagcta caatggggta caaacataaa gccttagatg ctaatgaagc aaaggatcaa    480
   atttgtcgcc ctaattatat gttgaaaata atacccaatt atgatggaag ccctagaata    540
   tgtgagctta taaatgcgaa aatcacagat gttaccgtac atgaagcttg gacaggacca    600
   actcgactgc agttatttga tcacgctatg gcgccactta atgatttgcc agtaaaagag    660
   attgtttcta gctctcacat tcttgcagat ataatattgc ctagagctga agttatatat    720
   gattatctta agtaa                                                     735
<210> 20
   <211> 4616
   <212> DNA
   <213> Artificial
<220>
   <223> Plasmid
<400> 20
   gcgcccaata cgcaaaccgc ctctccccgc gcgttggccg attcattaat gcagctggca      60
   cgacaggttt cccgactgga aagcgggcag tgagcgcaac gcaattaatg tgagttagct     120
   cactcattag gcaccccagg ctttacactt tatgcttccg gctcgtatgt tgtgtggaat     180
   tgtgagcgga taacaatttc acacaggaaa cagctatgac catgattacg ccaagctcta     240
   atacgactca ctatagggaa agctcggtac cacgcatgct gcagacgcgt tacgtatcgg     300
   atccagaatt cgtgatggaa ggtacctttt atgttaaagg atgaagtaat taaacaaatt     360
   agcacgccat taacttcgcc tgcatttcct agaggaccct ataaatttca taatcgtgag     420
   tattttaaca ttgtatatcg tacagatatg gatgcacttc gtaaagttgt gccagagcct     480
   ttagaaattg atgagccctt agtcaggttt gaaattatgg caatgcatga tacgagtgga     540
   cttggttgtt atacagaaag cggacaggct attcccgtaa gctttaatgg agttaaggga     600
   gattatcttc atatgatgta tttagataat gagcctgcaa ttgcagtagg aagggaatta     660
   agtgcatatc ctaaaaagct cgggtatcca aagctttttg tggattcaga tactttagta     720
   ggaactttag actatggaaa acttagagtt gcgacagcta caatggggta caaacataaa     780
   gccttagatg ctaatgaagc aaaggatcaa atttgtcgcc ctaattatat gttgaaaata     840
   atacccaatt atgatggaag ccctagaata tgtgagctta taaatgcgaa aatcacagat     900
   gttaccgtac atgaagcttg gacaggacca actcgactgc agttatttga tcacgctatg     960
   gcgccactta atgatttgcc agtaaaagag attgtttcta gctctcacat tcttgcagat    1020
   ataatattgc ctagagctga agttatatat gattatctta agtaatagaa ttcagagtta    1080
   catctgaatt cgtcgacaag cttctcgagc ctaggctagc tctagaccac acgtgtgggg    1140
   gcccgagctc gcggccgctg tattctatag tgtcacctaa atggccgcac aattcactgg   1200
   ccgtcgtttt acaacgtcgt gactgggaaa accctggcgt tacccaactt aatcgccttg   1260
   cagcacatcc ccctttcgcc agctggcgta atagcgaaga ggcccgcacc gatcgccctt   1320
   cccaacagtt gcgcagcctg aatggcgaat ggaaattgta agcgttaata ttttgttaaa   1380
   attcgcgtta aatttttgtt aaatcagctc attttttaac caataggccg aaatcggcaa   1440
   aatcccttat aaatcaaaag aatagaccga gatagggttg agtgttgttc cagtttggaa   1500
   caagagtcca ctattaaaga acgtggactc caacgtcaaa gggcgaaaaa ccgtctatca   1560
   gggcgatggc ccactacgtg aaccatcacc ctaatcaagt tttttggggt cgaggtgccg   1620
   taaagcacta aatcggaacc ctaaagggag cccccgattt agagcttgac ggggaaagcc   1680
   ggcgaacgtg gcgagaaagg aagggaagaa agcgaaagga gcgggcgcta gggcgctggc   1740
   aagtgtagcg gtcacgctgc gcgtaaccac cacacccgcc gcgcttaatg cgccgctaca   1800
   gggcgcgtca ggtggcactt ttcggggaaa tgtgcgcgga acccctattt gtttattttt   1860
   ctaaatacat tcaaatatgt atccgctcat gagacaataa ccctgataaa tgcttcaata   1920
   atattgaaaa aggaagagta tgagtattca acatttccgt gtcgccctta ttcccttttt   1980
   tgcggcattt tgccttcctg tttttgctca cccagaaacg ctggtgaaag taaaagatgc   2040
   tgaagatcag ttgggtgcac gagtgggtta catcgaactg gatctcaaca gcggtaagat   2100
   ccttgagagt tttcgccccg aagaacgttt tccaatgatg agcactttta aagttctgct   2160
   atgtggcgcg gtattatccc gtattgacgc cgggcaagag caactcggtc gccgcataca   2220
   ctattctcag aatgacttgg ttgagtactc accagtcaca gaaaagcatc ttacggatgg   2280
   catgacagta agagaattat gcagtgctgc cataaccatg agtgataaca ctgcggccaa   2340
   cttacttctg acaacgatcg gaggaccgaa ggagctaacc gcttttttgc acaacatggg   2400
   ggatcatgta actcgccttg atcgttggga accggagctg aatgaagcca taccaaacga   2460
   cgagcgtgac accacgatgc ctgtagcaat ggcaacaacg ttgcgcaaac tattaactgg   2520
   cgaactactt actctagctt cccggcaaca attaatagac tggatggagg cggataaagt   2580
   tgcaggacca cttctgcgct cggcccttcc ggctggctgg tttattgctg ataaatctgg   2640
   agccggtgag cgtgggtctc gcggtatcat tgcagcactg gggccagatg gtaagccctc   2700
   ccgtatcgta gttatctaca cgacggggag tcaggcaact atggatgaac gaaatagaca   2760
   gatcgctgag ataggtgcct cactgattaa gcattggtaa ctgtcagacc aagtttactc   2820
   atatatactt tagattgatt taaaacttca tttttaattt aaaaggatct aggtgaagat   2880
   cctttttgat aatctcatga acaataaaac tgtctgctta cataaacagt aatacaaggg   2940
   gtgttatgag ccatattcaa cgggaaacgt cttgctctag gccgcgatta aattccaaca   3000
   tggatgctga tttatatggg tataaatggg ctcgcgataa tgtcgggcaa tcaggtgcga   3060
   caatctatcg attgtatggg aagcccgatg cgccagagtt gtttctgaaa catggcaaag   3120
   gtagcgttgc caatgatgtt acagatgaga tggtcagact aaactggctg acggaattta   3180
   tgcctcttcc gaccatcaag cattttatcc gtactcctga tgatgcatgg ttactcacca    3240
   ctgcgatccc cgggaaaaca gcattccagg tattagaaga atatcctgat tcaggtgaaa    3300
   atattgttga tgcgctggca gtgttcctgc gccggttgca ttcgattcct gtttgtaatt    3360
   gtccttttaa cagcgatcgc gtatttcgtc tcgctcaggc gcaatcacga atgaataacg    3420
   gtttggttga tgcgagtgat tttgatgacg agcgtaatgg ctggcctgtt gaacaagtct    3480
   ggaaagaaat gcataaactt ttgccattct caccggattc agtcgtcact catggtgatt    3540
   tctcacttga taaccttatt tttgacgagg ggaaattaat aggttgtatt gatgttggac    3600
   gagtcggaat cgcagaccga taccaggatc ttgccatcct atggaactgc ctcggtgagt    3660
   tttctccttc attacagaaa cggctttttc aaaaatatgg tattgataat cctgatatga    3720
   ataaattgca gtttcatttg atgctcgatg agtttttcta agaattaatt catgaccaaa    3780
   atcccttaac gtgagttttc gttccactga gcgtcagacc ccgtagaaaa gatcaaagga    3840
   tcttcttgag atcctttttt tctgcgcgta atctgctgct tgcaaacaaa aaaaccaccg    3900
   ctaccagcgg tggtttgttt gccggatcaa gagctaccaa ctctttttcc gaaggtaact    3960
   ggcttcagca gagcgcagat accaaatact gtccttctag tgtagccgta gttaggccac    4020
   cacttcaaga actctgtagc accgcctaca tacctcgctc tgctaatcct gttaccagtg    4080
   gctgctgcca gtggcgataa gtcgtgtctt accgggttgg actcaagacg atagttaccg    4140
   gataaggcgc agcggtcggg ctgaacgggg ggttcgtgca cacagcccag cttggagcga    4200
   acgacctaca ccgaactgag atacctacag cgtgagctat gagaaagcgc cacgcttccc    4260
   gaagggagaa aggcggacag gtatccggta agcggcaggg tcggaacagg agagcgcacg    4320
   agggagcttc cagggggaaa cgcctggtat ctttatagtc ctgtcgggtt tcgccacctc    4380
   tgacttgagc gtcgattttt gtgatgctcg tcaggggggc ggagcctatg gaaaaacgcc    4440
   agcaacgcgg cctttttacg gttcctggcc ttttgctggc cttttgctca catgttcttt    4500
   cctgcgttat cccctgattc tgtggataac cgtattaccg cctttgagtg agctgatacc    4560
   gctcgccgca gccgaacgac cgagcgcagc gagtcagtga gcgaggaagc ggaaga        4616
<210> 21
   <211> 5098
   <212> DNA
   <213> Artificial
<220>
   <223> Plasmid
<400> 21
   gcgcccaata cgcaaaccgc ctctccccgc gcgttggccg attcattaat gcagctggca 60
   cgacaggttt cccgactgga aagcgggcag tgagcgcaac gcaattaatg tgagttagct 120
   cactcattag gcaccccagg ctttacactt tatgcttccg gctcgtatgt tgtgtggaat 180
   tgtgagcgga taacaatttc acacaggaaa cagctatgac catgattacg ccaagctcta 240
   atacgactca ctatagggaa agctcggtac cacgcatgct gcagacgcgt tacgtatcgg 300
   atccagaatt cgtgatggtt tatctgtcga cccgtatcaa aatttaggag gttagttaga 360
   atgaaagaag ttgtaatagc tagtgcagta agaacagcga ttggatctta tggaaagtct 420
   cttaaggatg taccagcagt agatttagga gctacagcta taaaggaagc agttaaaaaa 480
   gcaggaataa aaccagagga tgttaatgaa gtcattttag gaaatgttct tcaagcaggt 540
   ttaggacaga atccagcaag acaggcatct tttaaagcag gattaccagt tgaaattcca 600
   gctatgacta ttaataaggt ttgtggttca ggacttagaa cagttagctt agcagcacaa 660
   attataaaag caggagatgc tgacgtaata atagcaggtg gtatggaaaa tatgtctaga 720
   gctccttact tagcgaataa cgctagatgg ggatatagaa tgggaaacgc taaatttgtt 780
   gatgaaatga tcactgacgg attgtgggat gcatttaatg attaccacat gggaataaca 840
   gcagaaaaca tagctgagag atggaacatt tcaagagaag aacaagatga gtttgctctt 900
   gcatcacaaa aaaaagctga agaagctata aaatcaggtc aatttaaaga tgaaatagtt 960
   cctgtagtaa ttaaaggcag aaagggagaa actgtagttg atacagatga gcaccctaga 1020
   tttggatcaa ctatagaagg acttgcaaaa ttaaaacctg ccttcaaaaa agatggaaca 1080
   gttacagctg gtaatgcatc aggattaaat gactgtgcag cagtacttgt aatcatgagt 1140
   gcagaaaaag ctaaagagct tggagtaaaa ccacttgcta agatagtttc ttatggttca 1200
   gcaggagttg acccagcaat aatgggatat ggacctttct atgcaacaaa agcagctatt 1260
   gaaaaagcag gttggacagt tgatgaatta gatttaatag aatcaaatga agcttttgca 1320
   gctcaaagtt tagcagtagc aaaagattta aaatttgata tgaataaagt aaatgtaaat 1380
   ggaggagcta ttgcccttgg tcatccaatt ggagcatcag gtgcaagaat actcgttact 1440
   cttgtacacg caatgcaaaa aagagatgca aaaaaaggct tagcaacttt atgtataggt 1500
   ggcggacaag gaacagcaat attgctagaa aagtgctaga aaggatcctt aaaaataact 1560
   ctgatctgaa ttcgtcgaca agcttctcga gcctaggcta gctctagacc acacgtgtgg 1620
   gggcccgagc tcgcggccgc tgtattctat agtgtcacct aaatggccgc acaattcact 1680
   ggccgtcgtt ttacaacgtc gtgactggga aaaccctggc gttacccaac ttaatcgcct 1740
   tgcagcacat ccccctttcg ccagctggcg taatagcgaa gaggcccgca ccgatcgccc 1800
   ttcccaacag ttgcgcagcc tgaatggcga atggaaattg taagcgttaa tattttgtta 1860
   aaattcgcgt taaatttttg ttaaatcagc tcatttttta accaataggc cgaaatcggc 1920
   aaaatccctt ataaatcaaa agaatagacc gagatagggt tgagtgttgt tccagtttgg 1980
   aacaagagtc cactattaaa gaacgtggac tccaacgtca aagggcgaaa aaccgtctat 2040
   cagggcgatg gcccactacg tgaaccatca ccctaatcaa gttttttggg gtcgaggtgc 2100
   cgtaaagcac taaatcggaa ccctaaaggg agcccccgat ttagagcttg acggggaaag 2160
   ccggcgaacg tggcgagaaa ggaagggaag aaagcgaaag gagcgggcgc tagggcgctg 2220
   gcaagtgtag cggtcacgct gcgcgtaacc accacacccg ccgcgcttaa tgcgccgcta 2280
   cagggcgcgt caggtggcac ttttcgggga aatgtgcgcg gaacccctat ttgtttattt 2340
   ttctaaatac attcaaatat gtatccgctc atgagacaat aaccctgata aatgcttcaa 2400
   taatattgaa aaaggaagag tatgagtatt caacatttcc gtgtcgccct tattcccttt 2460
   tttgcggcat tttgccttcc tgtttttgct cacccagaaa cgctggtgaa agtaaaagat 2520
   gctgaagatc agttgggtgc acgagtgggt tacatcgaac tggatctcaa cagcggtaag 2580
   atccttgaga gttttcgccc cgaagaacgt tttccaatga tgagcacttt taaagttctg 2640
   ctatgtggcg cggtattatc ccgtattgac gccgggcaag agcaactcgg tcgccgcata 2700
   cactattctc agaatgactt ggttgagtac tcaccagtca cagaaaagca tcttacggat 2760
   ggcatgacag taagagaatt atgcagtgct gccataacca tgagtgataa cactgcggcc 2820
   aacttacttc tgacaacgat cggaggaccg aaggagctaa ccgctttttt gcacaacatg 2880
   ggggatcatg taactcgcct tgatcgttgg gaaccggagc tgaatgaagc cataccaaac 2940
   gacgagcgtg acaccacgat gcctgtagca atggcaacaa cgttgcgcaa actattaact 3000
   ggcgaactac ttactctagc ttcccggcaa caattaatag actggatgga ggcggataaa 3060
   gttgcaggac cacttctgcg ctcggccctt ccggctggct ggtttattgc tgataaatct 3120
   ggagccggtg agcgtgggtc tcgcggtatc attgcagcac tggggccaga tggtaagccc 3180
   tcccgtatcg tagttatcta cacgacgggg agtcaggcaa ctatggatga acgaaataga 3240
   cagatcgctg agataggtgc ctcactgatt aagcattggt aactgtcaga ccaagtttac 3300
   tcatatatac tttagattga tttaaaactt catttttaat ttaaaaggat ctaggtgaag 3360
   atcctttttg ataatctcat gaacaataaa actgtctgct tacataaaca gtaatacaag 3420
   gggtgttatg agccatattc aacgggaaac gtcttgctct aggccgcgat taaattccaa 3480
   catggatgct gatttatatg ggtataaatg ggctcgcgat aatgtcgggc aatcaggtgc 3540
   gacaatctat cgattgtatg ggaagcccga tgcgccagag ttgtttctga aacatggcaa 3600
   aggtagcgtt gccaatgatg ttacagatga gatggtcaga ctaaactggc tgacggaatt 3660
   tatgcctctt ccgaccatca agcattttat ccgtactcct gatgatgcat ggttactcac 3720
   cactgcgatc cccgggaaaa cagcattcca ggtattagaa gaatatcctg attcaggtga 3780
   aaatattgtt gatgcgctgg cagtgttcct gcgccggttg cattcgattc ctgtttgtaa 3840
   ttgtcctttt aacagcgatc gcgtatttcg tctcgctcag gcgcaatcac gaatgaataa 3900
   cggtttggtt gatgcgagtg attttgatga cgagcgtaat ggctggcctg ttgaacaagt 3960
   ctggaaagaa atgcataaac ttttgccatt ctcaccggat tcagtcgtca ctcatggtga 4020
   tttctcactt gataacctta tttttgacga ggggaaatta ataggttgta ttgatgttgg 4080
   acgagtcgga atcgcagacc gataccagga tcttgccatc ctatggaact gcctcggtga 4140
   gttttctcct tcattacaga aacggctttt tcaaaaatat ggtattgata atcctgatat 4200
   gaataaattg cagtttcatt tgatgctcga tgagtttttc taagaattaa ttcatgacca 4260
   aaatccctta acgtgagttt tcgttccact gagcgtcaga ccccgtagaa aagatcaaag 4320
   gatcttcttg agatcctttt tttctgcgcg taatctgctg cttgcaaaca aaaaaaccac 4380
   cgctaccagc ggtggtttgt ttgccggatc aagagctacc aactcttttt ccgaaggtaa 4440
   ctggcttcag cagagcgcag ataccaaata ctgtccttct agtgtagccg tagttaggcc 4500
   accacttcaa gaactctgta gcaccgccta catacctcgc tctgctaatc ctgttaccag   4560
   tggctgctgc cagtggcgat aagtcgtgtc ttaccgggtt ggactcaaga cgatagttac   4620
   cggataaggc gcagcggtcg ggctgaacgg ggggttcgtg cacacagccc agcttggagc   4680
   gaacgaccta caccgaactg agatacctac agcgtgagct atgagaaagc gccacgcttc   4740
   ccgaagggag aaaggcggac aggtatccgg taagcggcag ggtcggaaca ggagagcgca   4800
   cgagggagct tccaggggga aacgcctggt atctttatag tcctgtcggg tttcgccacc   4860
   tctgacttga gcgtcgattt ttgtgatgct cgtcaggggg gcggagccta tggaaaaacg   4920
   ccagcaacgc ggccttttta cggttcctgg ccttttgctg gccttttgct cacatgttct   4980
   ttcctgcgtt atcccctgat tctgtggata accgtattac cgcctttgag tgagctgata   5040
   ccgctcgccg cagccgaacg accgagcgca gcgagtcagt gagcgaggaa gcggaaga     5098